(19) Europäisches Patentamt
European Patent Office
Office européen des brevets

(11) Veröffentlichungsnummer: **0 128 120**
**A2**

(12) # EUROPÄISCHE PATENTANMELDUNG

(21) Anmeldenummer: **84810263.8**

(22) Anmeldetag: **28.05.84**

(51) Int. Cl.³: **C 07 D 498/04**
C 07 D 498/10, A 61 K 31/42
A 61 K 31/435, C 07 D 207/08
C 07 D 211/22, C 07 D 401/06
//C07D207/50, C07D211/98,
C07D221/20, (C07D498/04,
263/00, 209/00), (C07D498/04,
263/00, 221/00), (C07D498/10,
263/00, 221/00)

(30) Priorität: **02.06.83 CH 3013/83**

(43) Veröffentlichungstag der Anmeldung:
**12.12.84 Patentblatt 84/50**

(84) Benannte Vertragsstaaten:
**AT BE CH DE FR GB IT LI LU NL SE**

(71) Anmelder: **CIBA-GEIGY AG**
**Postfach**
**CH-4002 Basel(CH)**

(72) Erfinder: **Paioni, Romeo, Dr.**
**Wettsteinstrasse 6**
**CH-4125 Riehen(CH)**

(54) **Trisubstituierte Oxazolidinone.**

(57) Trisubstituierte Oxazolidinone der Formel

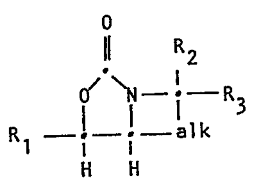

(I),

worin $R_1$ einen Aryl- oder Heteroarylrest bedeutet, $R_2$ und $R_3$ unabhängig voneinander Wasserstoff oder Niederalkyl oder gemeinsam Niederalkylen darstellen und alk für Niederalkylen steht, dessen Valenzen von benachbarten oder zueinander 1,3-ständigen Kohlenstoffatomen ausgehen, mit der Massgabe, dass in Verbindungen mit (–)-trans-Anordnung der Wasserstoffatome in 4- und 5-Stellung des Oxazolidinringes $R_1$ von unsubstituiertem Phenyl verschieden ist, wenn $R_2$ und $R_3$ für Wasserstoff stehen und alk 1,3-Propylen bedeutet, werden als antidepressive Arzneimittelwirkstoffe vorgeschlagen.

Sie werden hergestellt, indem man z.B. eine Verbindung der Formel

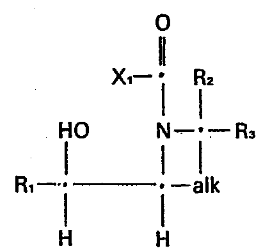

(III),

worin $X_1$ eine nukleofuge Abgangsgruppe darstellt, oder ein Salz davon intramolekular cyclisiert.

EP 0 128 120 A2

Croydon Printing Company Ltd.

CIBA-GEIGY AG
Basel (Schweiz)

4-14453/+

## Trisubstituierte Oxazolidinone

Die Erfindung betrifft trisubstituierte Oxazolidinone der Formel

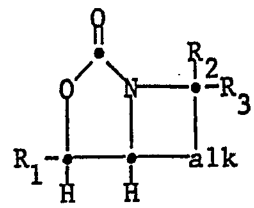

(I),

worin $R_1$ einen Aryl- oder Heteroarylrest bedeutet, $R_2$ und $R_3$ unabhängig voneinander Wasserstoff oder Niederalkyl oder gemeinsam Niederalkylen darstellen und alk für Niederalkylen steht, dessen Valenzen von benachbarten oder zueinander 1,3-ständigen Kohlenstoffatomen ausgehen, und ihre pharmazeutisch verwendbaren Salze zur Anwendung in einem Verfahren zur Behandlung des menschlichen oder tierischen Körpers, ihre Verwendung als Arzneimittelwirkstoffe, sie enthaltende pharmazeutische Präparate, sowie Verbindungen der Formel I selbst, mit der Massgabe, dass in Verbindungen mit (-)-trans-Anordnung der Wasserstoffatome in 4- und 5-Stellung des Oxazolidinringes $R_1$ von unsubstituiertem Phenyl verschieden ist, wenn $R_2$ und $R_3$ für Wasserstoff stehen und alk 1,3-Propylen bedeutet, d.h. unter Ausschluss des (-)-trans-1-Phenyl-3-oxo-perhydro-3H-oxazolo[3,4-a]pyridins, und ihre Salze sowie Verfahren zur Herstellung der erfindungsgemässen Verbindungen.

Arylreste sind beispielsweise 6-gliedrige monocyclische oder mindestens einen 6-Ring aufweisende bicyclische Arylreste, wie Phenyl oder Naphthyl, z.B. 1- oder 2-Naphthyl. Heteroarylreste sind beispielsweise monocyclische, als Heteroatom(e) Stickstoff, Sauerstoff, Schwefel, Sauerstoff sowie Stickstoff oder Schwefel sowie Stickstoff aufweisende

5-gliedrige oder als Heteroatom(e) 1 oder 2 Stickstoffatome aufweisende 6-gliedrige Heteroarylreste, wie Furyl, z.B. 2-Furyl, Thienyl, z.B. 2-Thienyl, Pyrrolyl, z.B. 1-Pyrrolyl, Thiazolyl, z.B. 2-Thiazolyl, Oxazolyl, z.B. 2-Oxazolyl, Pyridyl, z.B. 3- oder 4-Pyridyl, Pyrimidinyl, z.B. 2- oder 4-Pyrimidinyl, oder Pyrazinyl, z.B. 2-Pyrazinyl.

Als Substituenten von Aryl- bzw. Heteroarylresten $R_1$ kommen beispielsweise Niederalkyl, Niederalkoxy, Halogen, Cyano und/oder Trifluormethyl in Betracht, wobei einer oder mehr als einer, z.B. ein, zwei oder drei gleiche oder verschiedene, dieser Substituenten vorhanden sein können.

Als "niedere" organische Reste und Verbindungen sind vor- und nachstehend beispielsweise solche zu verstehen, die bis und mit 7, vorzugsweise bis und mit 4, Kohlenstoffatome (C-Atome) aufweisen.

Niederalkyl ist beispielsweise Methyl, Aethyl, Propyl, Isopropyl, Butyl, Isobutyl, Sekundärbutyl oder Tertiärbutyl, ferner beliebig gebundenes Pentyl, Hexyl oder Heptyl. In Verbindungen I, worin $R_2$ und $R_3$ für Niederalkylreste stehen, handelt es sich bei diesen vorzugsweise um gleiche oder verschiedene, insbesondere jedoch gleiche, primäre Niederalkylreste, wie Methyl, Aethyl, Propyl, Butyl, Pentyl, Hexyl oder Heptyl.

Niederalkylen ist beispielsweise Aethylen oder 1,3-Propylen, im Falle von durch $R_2$ und $R_3$ gemeinsam dargestellte Niederalkylenresten, deren freie Valenzen z.B. von benachbarten oder zueinander 1,3-, 1,4- oder 1,5-ständigen C-Atomen ausgehen, ferner 1,4-Butylen, 1,5-Pentylen, 1,4-(2,2-Dimethyl)-butylen oder 1,5-(3,3-Dimethyl)-pentylen und im Falle von Niederalkylenresten alk ferner 1,2-(2-Methyl)-propylen, 1,3-(3-Methyl)-butylen oder 1,3-(2,2-Dimethyl)-propylen.

Niederalkoxy ist beispielsweise Methoxy, Aethoxy, Propyloxy, Isopropyloxy, Butyloxy, Isobutyloxy, Sekundärbutyloxy oder Tertiärbutyloxy, kann aber auch eine Pentyloxy-, Hexyloxy- oder Heptyloxygruppe sein.

Halogen ist beispielsweise Halogen der Atomnummer bis und mit 35, wie Chlor, ferner Fluor oder Brom.

Die Verbindungen der Formel I weisen mindestens 2 asymmetrische C-Atome (die Ringatome 4 und 5 des Oxazolidinringes) auf. Sie können dementsprechend als cis/trans-Isomere bezüglich der gegenseitigen Orientierung der Wasserstoffatome in 4- und 5-Stellung des Oxazolidinringes auftreten, wobei das trans-Isomere threo-, das cis-Isomere erythro-Konfiguration besitzt. Die neuen Verbindungen können dementsprechend in Form zweier zueinander diastereomerer Enantiomerenpaare auftreten. Sind zusätzlich die Variablen $R_2$ und $R_3$ verschieden oder weisen die durch $R_1$, $R_2$, $R_3$ und/oder alk dargestellten Teilstrukturen mindestens ein weiteres Asymmetriezentrum auf, sind noch weitere stereoisomere Formen möglich. Diese entsprechen sämtlich der Formel I und sind dementsprechend ebenfalls Gegenstand der Erfindung. Bevorzugt sind jedoch jeweils die threo-Diastereomeren, d.h. diejenigen Verbindungen I, in denen die Wasserstoffatome in 4- und 5-Stellung des Oxazolidinringes zueinander trans-ständig sein, und gegebenenfalls davon abgeleitete Diastereomere bzw. Diastereomerengemische mit mindestens einem weiteren Asymmetriezentrum.

Salze von Verbindungen der Formel I sind beispielsweise Säureadditionssalze, insbesondere pharmazeutisch verwendbaren Säureadditionssalze von Verbindungen der Formel I, worin $R_1$ einen basischen, d.h. mindestens ein Stickstoffatom aufweisenden Heteroarylrest bedeutet, insbesondere mit Mineralsäuren oder starken organischen Säuren, wie Halogenwasserstoffsäuren, Sulfonsäuren, Sulfaminsäuren oder gegebenenfalls hydroxylierten aliphatischen Mono- oder Dicarbonsäuren, z.B. Hydrochloride, Hydrobromide, Methansulfonate, p-Toluolsulfonate, N-Cyclohexylsulfaminate, Fumarate, Maleinate, Maleate oder Tartrate.

Die Verbindungen der Formel I und ihre pharmazeutisch verwendbaren Salze besitzen vorteilhafte pharmakologische Eigenschaften. So bewirken sie beispielsweise an der Maus im Schwimm-Test nach Porsolt et al., Arch. Int. Pharmacodyn. 229, 327-336 (1977) im Dosisbereich ab etwa 25 mg/kg p.o. eine deutliche Steigerung der Motilität sowie an der Ratte im Lochbrett-Test nach Noble und Delini-Stula, Psychopharmacology 49, 17-22 (1976) im Dosisbereich von etwa 25 bis etwa 50 mg/kg p.o. eine deutliche Erhöhung der Explorationsrate. Neurobiochemisch zeichnen sie sich in der Versuchsanordnung nach Waldmeier, de Herdt und Maitre, Chir. Chem. 20, 81 (1974) im Dosisbereich ab etwa 100 mg/kg i.p. durch eine deutliche Dopamin- sowie Noradrenalin-Aufnahmehemmung im Rattenhirn aus, wobei die Dopaminaufnahmehemmung überwiegt. Die Verbindungen der Formel I sind ferner wenig toxisch, die $LD_{50}$ an der Maus liegt vielfach deutlich über 1000 mg/kg p.o..

Die neuen Verbindungen und ihre pharmazeutisch verwendbaren Salze sind dementsprechend geeignet als Antidepressiva, insbesondere mit nicht Amphetamin-ähnlicher, leicht stimulierender Wirkungskomponente.

Die Erfindung betrifft in erster Linie Verbindungen der Formel I, worin $R_1$ einen als Substituenten gegebenenfalls Niederalkyl, Niederalkoxy, Halogen, Cyano und/oder Trifluormethyl aufweisenden 6-gliedrigen monocyclischen oder mindestens einen 6-gliedrigen Ring aufweisenden bicyclischen Arylrest oder monocyclischen als Heteroatom(e) Stickstoff, Sauerstoff, Schwefel, Sauerstoff sowie Stickstoff bzw. Schwefel sowie Stickstoff aufweisenden 5-gliedrigen bzw. als Heteroatom(e) 1 oder 2 Stickstoffatome aufweisenden 6-gliedrigen Heteroarylrest bedeutet, $R_2$ und $R_3$ unabhängig voneinander Wasserstoff, Niederalkyl oder gemeinsam Niederalkylen darstellen und alk Niederalkylen bedeutet, dessen Valenzen von benachbarten oder zueinander 1,3-ständigen C-Atomen ausgehen, vor allem solche, in denen die Wasserstoffatome in 4- und 5-Stellung des Oxazolidinringes zueinander trans-ständig sind, und ihre pharmazeutisch verwendbaren Salze zur Anwendung in einem Verfahren zur Behandlung des menschlichen oder tierischen Körpers, ihre

Verwendung als Arzneimittelwirkstoffe und sie enthaltende pharmazeutische Präparate sowie die vorstehend definierten Verbindungen selbst, mit der Massgabe, dass in Verbindungen mit (-)-trans-Anordnung der Wasserstoffatome in 4- und 5-Stellung des Oxazolidinringes $R_1$ von unsubstituiertem Phenyl verschieden ist, wenn $R_2$ und $R_3$ für Wasserstoff stehen und alk 1,3-Propylen bedeutet, d.h, unter Ausschluss des (-)-trans-1-Phenyl-3-oxo-perhydro-3H-oxazolo[3,4-a]pyridins, und ihre Salze sowie Verfahren zu ihrer Herstellung.

Die Erfindung betrifft vor allem Verbindungen der Formel I, worin $R_1$ einen unsubstituierten oder durch Niederalkyl mit bis und mit 4 C-Atomen, wie Methyl, Niederalkoxy mit bis und mit 4 C-Atomen, wie Methoxy, Halogen der Atomnummer bis und mit 35, wie Chlor, und/oder Trifluormethyl substituierten Phenyl-, Naphthyl-, wie 1- oder 2-Naphthyl-, Furyl-, wie 2-Furyl-, Thienyl-, wie 2-Thienyl-, Pyrrolyl-, wie 1-Pyrrolyl, Thiazolyl-, wie 2-Thiazolyl-, Oxazolyl-, wie 2-Oxazolyl-, Pyridyl-, wie 3- oder 4-Pyridyl-, Pyrimidinyl-, wie 2- oder 4-Pyrimidinyl-, oder Pyrazinyl-, wie 2-Pyrazinylrest bedeutet, $R_2$ und $R_3$ unabhängig voneinander Wasserstoff oder Niederalkyl mit bis und mit 4 C-Atomen, wie Methyl, oder gemeinsam Niederalkylen mit bis und mit 7 C-Atomen, dessen freie Valenzen von benachbarten oder zueinander 1,3-, 1,4- oder 1,5-ständigen C-Atomen ausgehen, wie Aethylen, 1,3-Propylen, 1,4-Butylen, 1,5-Pentylen oder 1,5-(3,3-Dimethyl)-pentylen, darstellen und alk Niederalkylen mit bis und mit 7 C-Atomen, dessen freie Valenzen von benachbarten oder zueinander 1,3-ständigen C-Atomen ausgehen, wie Aethylen, 1,3-Propylen, 1,2-(2-Methyl)-propylen, 1,3-(3-Methyl)-butylen oder 1,3-(2,2-Dimethyl)-propylen, bedeutet, vor allem solche, in denen die Wasserstoffatome in 4- und 5-Stellung des Oxazolidinringes zueinander trans-ständig sind, und ihre pharmazeutisch verwendbaren Salze zur Anwendung in einem Verfahren zur therapeutischen Behandlung des menschlichen oder tierischen Körpers, sie enthaltende pharmazeutische Präparate und ihre Verwendung als Arzneimittelwirkstoffe sowie die vorstehend definierten Verbindungen selbst, worin $R_1$ die angegebene Bedeutung hat und worin entweder $R_2$ und $R_3$ unabhängig voneinander Wasserstoff oder Niederalkyl mit bis und mit 4 C-Atomen,

wie Methyl, oder gemeinsam Niederalkylen mit bis und mit 7 C-Atomen, dessen freie Valenzen von benachbarten oder zueinander 1,3-, 1,4- oder 1,5-ständigen C-Atomen ausgehen, wie Aethylen, 1,3-Propylen, 1,5-Pentylen, 1,4-Butylen, 1,5-(3,3-Dimethyl)-pentylen, darstellen und alk Niederalkylen mit bis und mit 7 C-Atomen, dessen Valenzen von benachbarten C-Atomen ausgehen, wie Aethylen oder 1,2-(2-Methyl)-propylen, bedeutet oder $R_2$ und $R_3$ unabhängig voneinander Niederalkyl mit bis und mit 4 C-Atomen, wie Methyl, oder gemeinsam Niederalkylen mit bis und mit 7 C-Atomen, dessen freie Valenzen von benachbarten oder zueinander 1,3-, 1,4- oder 1,5-ständigen C-Atomen ausgehen, wie Aethylen, 1,3-Propylen, 1,4-Butylen, 1,5-Pentylen oder 1,5-(3,3-Dimethyl)-pentylen, darstellen und alk Niederalkylen mit bis und mit 7 C-Atomen, dessen Valenzen von zueinander 1,3-ständigen C-Atomen ausgehen, wie 1,3-Propylen, 1,3-(3-Methyl)-butylen oder 1,3-(2,2-Dimethyl)-propylen, bedeutet, selbst und ihre Salze, insbesondere ihre pharmazeutisch verwendbaren Salze, sowie Verfahren zu ihrer Herstellung.

Die Erfindung betrifft insbesondere Verbindungen der Formel I, worin $R_1$ unsubstituiertes oder durch Niederalkyl mit bis und mit 4 C-Atomen, wie Methyl, Niederalkoxy mit bis und mit 4 C-Atomen, wie Methoxy, Halogen der Atomnummer bis und mit 35, wie Chlor, und/oder Trifluormethyl substituiertes Phenyl oder unsubstituiertes Furyl, z.B. 2-Furyl, Thienyl, z.B. 2-Thienyl, oder Pyridyl, z.B. 3- oder 4-Pyridyl, bedeutet, $R_2$ und $R_3$ jeweils, vorzugsweise gleiche, Niederalkylreste mit bis und mit 4 C-Atomen, z.B. Methyl, oder gemeinsam Niederalkylen mit bis und mit 7 C-Atomen, dessen freie Valenzen von benachbarten oder zueinander 1,4- oder 1,5-ständigen C-Atomen ausgehen, wie Aethylen, 1,4-Butylen oder 1,5-Pentylen, darstellen und alk für Aethylen oder 1,3-Propylen steht, vor allem diejenigen, in denen die beiden Wasserstoffatome in 4- und 5-Stellung des Oxazolidinringes zueinander trans-ständig sind, und ihre, insbesondere pharmazeutisch verwendbaren Salze, sowie Verfahren zur ihrer Herstellung.

Die Erfindung betrifft in allererster Linie Verbindungen der Formel I, worin $R_1$, vorzugsweise in 3- und/oder 4-Stellung, durch Halogen der Atomnummer bis und mit 35, wie Chlor, substituiertes Phenyl bedeutet, $R_2$ und $R_3$ gleiche Niederalkylreste mit bis und mit 4 C-Atomen, wie Methyl, darstellen und alk für Aethylen steht, vor allem diejenigen, in denen die beiden Wasserstoffatome in 4- und 5-Stellung des Oxazolidinringes zueinander trans-ständig sind, und ihre Salze, insbesondere pharmazeutisch verwendbaren Salze, Verfahren zu ihrer Herstellung, sie enthaltende pharmazeutische Präparate und ihre Verwendung als Arzneimittelwirkstoffe.

Die Erfindung betrifft namentlich die in den Beispielen genannten Verbindungen der Formel I und ihre Salze, insbesondere pharmazeutisch verwendbaren Salze, Verfahren zu ihrer Herstellung, sie enthaltende pharmazeutische Präparate und ihre Verwendung als Arzneimittelwirkstoffe.

Die Verbindungen der Formel I können nach an sich bekannten Methoden hergestellt werden, beispielsweise indem man

a) in einer Verbindung der Formel

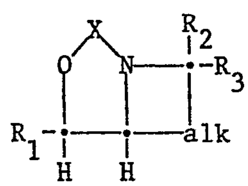

(II) ,

worin X einen in die Carbonylgruppe überführbaren Rest bedeutet, oder in einem Salz davon X in die Carbonylgruppe überführt oder

b) eine Verbindung der Formel

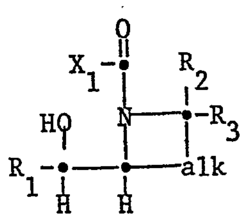

(III),

worin $X_1$ eine nukleofuge Abgangsgruppe darstellt, oder ein Salz davon intramolekular cyclisiert oder

c) in einer Verbindung der Formel

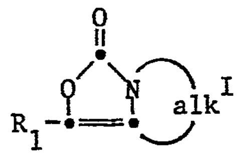

(IV),

worin $alk^{I}$ eine Gruppe der Formel $-alk-C(R_2)(R_3)-$, $-alk^{II}-C(R_2)(R_3)-$ oder $-alk^{III}=C(R_2)-$ bedeutet, die mit dem Stickstoffatom der Oxazolinringes über die $C(R_2)(R_3)-$ bzw. $=C(R_2)$-Gruppe verbunden ist, wobei $alk^{II}$ einen Niederalkenylenrest und $alk^{III}$ einen Niederalkanylyliden- bzw. Niederalkenylylidenrest, dessen freie Valenzen von benachbarten oder zueinander 1,3-ständigen Kohlenstoffatomen ausgehen, darstellt, die Doppelbindung(en) des Oxazolinringes und gegebenenfalls des Restes $alk^{I}$ absättigt und gewünschtenfalls ein verfahrensgemäss erhältliches Isomerengemisch in die Komponenten auftrennt bzw. aus einem solchen das oder die gewünschte(n) Isomere(n) isoliert, eine verfahrensgemäss erhältliche Verbindung in eine andere Verbindung der Formel I umwandelt und/oder eine verfahrensgemäss erhältliche freie Verbindung in ein Salz oder ein verfahrensgemäss erhältliches Salz in die freie Verbindung oder in ein anderes Salz überführt.

In die Carbonylgruppe überführbare Reste X sind beispielsweise funktionell abgewandelte Carbonylgruppen der Formel $-C(X_2)(X_3)-$, worin $X_2$ und $X_3$ unabhängig voneinander verestertes oder mit einem einwertigen Alkohol oder Mercaptan veräthertes Hydroxy oder gemeinsam mit einem zweiwertigen Alkohol oder Mercaptan ketalisiertes Oxo bzw. Thioxo oder gemeinsam gegebenenfalls substituiertes Imino darstellen. Verestertes Hydroxy ist dabei beispielsweise Halogen, mit einem einwertigen Alkohol bzw. Mercaptan veräthertes Hydroxy beispielsweise Niederalkoxy bzw. Niederalkylthio oder gegebenenfalls substituiertes, z.B. Nitro und/oder Halogen aufweisendes, Phenoxy bzw. Phenylthio und mit einem zweiwertigen Alkohol bzw. Mercaptan ketalisiertes Oxo beispielsweise Niederalkylendioxy bzw. Niederalkylendithio

oder gegebenenfalls substituiertes 1,2-Phenylendioxy bzw. 1,2-Phenylendithio. Durch $X_2$ und $X_3$ gemeinsam dargestelltes, gegebenenfalls substituiertes Imino ist beispielsweise Imino, Niederalkylimino oder gegebenenfalls substituiertes, wie Nitro und/oder Halogen aufweisendes, Anilo.

Nukleofuge Abgangsgruppen $X_1$ sind beispielsweise reaktionsfähige veresterte Hydroxygruppen, verätherte Hydroxygruppen, aktivierte Aminogruppen oder Ammoniogruppen.. Reaktionsfähiges verestertes Hydroxy ist beispielsweise mit einer Mineralsäure verestert und bedeutet insbesondere ein Halogenatom, wie Chlor. Veräthertes Hydroxy ist beispielsweise Niederalkoxy, z.B. Methoxy, Aethoxy, Isopropyloxy oder Tertiärbutyloxy, oder gegebenenfalls, insbesondere durch elektronenanziehende Substituenten, wie Nitro und/oder Halogen, substituiertes Phenoxy, wie Phenoxy, p-Nitro- oder 2,4-Dinitrophenoxy oder 3,5-Dichlorphenoxy. Aktiviertes Amino ist beispielsweise sekundäres Amino als Teil eines heteroaromatischen Systems, wie 1-Imidazolyl, 1-Pyrazolyl bzw. 1-(3,5-Dimethyl)-pyrazolyl. Ammonio ist beispielsweise Triniederalkyl-, z.B. Trimethylammonio, oder tertiäres Ammonio als Teil eines heteroaromatischen Systems, wie Pyridinio.

In ungesättigten Resten $alk^{I}$ bedeutet $alk^{II}$ beispielsweise Vinylen oder 1,3-Prop-2-enylen und $alk^{III}$ beispielsweise Aethanylyliden oder 1-Prop-1-enyl-3-yliden.

Als Salze von Zwischenprodukten II, III und IV kommen beispielsweise deren Säureadditionssalze, z.B. Hydrohalogenide, als Salze von Zwischenprodukten III ferner deren Metallsalze, in denen das Proton der Hydroxygruppe durch ein Metallkation ersetzt ist, wie entsprechende Natrium- oder Kaliumsalze, in Betracht.

Die Durchführung der verfahrensgemässen Reaktionen sowie die Herstellung neuer Ausgangsstoffe bzw. Zwischenprodukte erfolgt in Analogie zur Reaktions- und Bildungsweise bekannter Ausgangsstoffe bzw. Zwischenprodukte. Dabei werden, auch wenn nachstehend nicht ausdrücklich erwähnt, die jeweils üblichen Hilfsmittel, wie Katalysatoren, Kondensations- sowie Solvolysemittel und/oder Lösungs- bzw. Verdünnungsmittel, und Reaktions-, wie Temperatur- und Druckbedingungen, sowie gegebenenfalls Schutzgase verwendet.

Die Ueberführung von X in die Carbonylgruppe gemäss der Verfahrensvariante a) erfolgt beispielsweise durch Hydrolyse, vorzugsweise unter neutralen oder mild-sauren Bedingungen, z.B. in Gegenwart eines geeigneten sauren Mittels, wie einer Mineralsäure, z.B. von Chlor- oder Bromwasserstoffsäure, oder einer organischen Sulfon- oder Carbonsäure, wie einer Alkan- oder gegebenenfalls substituierten Benzolsulfonsäure oder einer Niederalkansäure, z.B. von p-Toluolsulfonsäure oder Essigsäure, oder indem man das Zwischenprodukt II als Salz, z.B. als Hydrochlorid oder Hydrobromid, einsetzt. Erforderlichenfalls arbeitet man in Anwesenheit weiterer Hilfsmittel, beispielsweise von Puffersystemen, ausgehend von Thioketalen II von Schwermetallsalzen oder oxidierenden Mitteln bzw. ausgehend von N-unsubstituierten Iminen II von salpetriger Säure. Geeignete Schwermetallsalze sind dabei beispielsweise Silber-, Quecksilber- oder Kupfersalze, geeignete Oxidationsmittel sind beispielsweise oxidierende Schwermetallverbindungen, wie Kupfer-II-, Eisen-III-, Eisen-VI-, Chrom-VI-, Mangan-IV- und Mangan-VII-Verbindungen, insbesondere Oxide oder Chloride, Halogene, wie Chlor oder Jod, bzw. Halogensauerstoffsäuren, z.B. Jodsäure, oder deren Salze. Bei der Durchführung der Hydrolyse können intermediär instabile Zwischenstufen gebildet werden. So können Thioketale II beispielsweise mit Halogenen leicht hydrolysierbare Sulfoniumhalogenide bilden und N-unsubstituierte Imine II mit salpetriger Säure zu leicht hydrolysierbaren Zwischenprodukten reagieren. Zumindest unter den erwähnten schonenden Bedingungen bleibt die Einwirkung der

Reagentien auf die Gruppe X beschränkt. Die Stereotaxie der Ringatome 4 und 5 des Oxazolidinringes sowie gegebenenfalls weiterer Asymmetriezentren wird dementsprechend nicht beeinflusst.

In einer bevorzugten Ausführungsform der Verfahrensvariante a) geht man beispielsweise von einem Säureadditionssalz, z.B. dem Hydrochlorid, eines N-unsubstituierten Imins II (X = Iminomethylen) aus und hydrolysiert dieses unter Erwärmen auf etwa 40-110°C, z.B. in der Siedehitze, im pH-Bereich von etwa 3,5 bis etwa 9, beispielsweise in Gegenwart eines Phosphatpuffers (pH etwa 6,5).

In einer anderen bevorzugten Ausführungsform behandelt man ein N-unsubstituiertes Imin II (X = Iminomethylen) in Gegenwart von Salzsäure unter Erwärmen, z.B. in der Siedehitze, mit einem Alkalimetallnitrit.

Die Zwischenprodukte II können beispielsweise hergestellt werden, indem man eine Verbindung der Formel

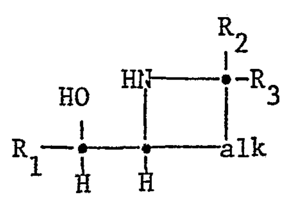

(V)

in üblicher Weise mit einer Verbindung der Formel $X_1-C(X_2)(X_3)-Y_1$ (VI) umsetzt, worin $X_1$, $X_2$ und $X_3$ gemeinsam die Nitrilogruppe darstellen und $Y_1$ Hydroxy oder Halogen bedeutet oder $X_1$ und $Y_1$ unabhängig voneinander veräthertes Hydroxy, z.B. Niederalkoxy oder gegebenenfalls substituiertes Phenoxy, und $X_2$ und $X_3$ jeweils mit einem einwertigen Alkohol oder Mercaptan veräthertes Hydroxy oder mit einem zweiwertigen Alkohol oder Mercaptan ketalisiertes Oxo bedeuten oder $X_1$ und $Y_1$ reaktionsfähiges verestertes Hydroxy, wie Halogen, und $X_2$ und $X_3$ gemeinsam Thioxo darstellen, oder $X_1$ und $Y_1$ gemeinsam Oxo und $X_2$ und $X_3$ jeweils mit einem einwertigen Mercaptan veräthertes Hydroxy oder gemeinsam mit einem zweiwertigen Mercaptan ketalisiertes Oxo darstellen oder $X_1$ und $Y_1$ gemeinsam Oxo oder Thioxo und $X_2$ und $X_3$ gemeinsam gegebenenfalls substituiertes Imino darstellen.

So kann man beispielsweise eine Verbindung V durch Umsetzung mit Brom- oder Chlorcyan in Gegenwart eines säurebindenden Mittels, z.B. von Natriumhydrogencarbonat, N-cyanieren und das gebildete N-Cyan-Zwischenprodukt, z.B. mit niederalkanolischer Salzsäure, zum entsprechenden Imin (II, $X_2$ + $X_3$ = Imino) cyclisieren. Die Umsetzung verläuft unter weitgehender Konfigurationserhaltung: Aus der threo-Verbindung V entsteht die entsprechende trans-Verbindung II und umgekehrt.
Man kann das Ausgangsmaterial V aber auch mit Cyansäure bzw. einem Alkalimetallcyanat unter sauren Bedingungen in das N-Carbamylderivat überführen und dieses mit Thionylchlorid oder Phosgen zum Imin (II, $X_2$ + $X_3$ = Imino) cyclisieren, wobei Konfigurationsumkehr erfolgt, d.h. aus der threo-Verbindung V die entsprechende cis-Verbindung II entsteht. Analog kann man eine Verbindung V auch mit einem organischen Isothiocyanat, z.B. einem Niederalkyl- oder gegebenenfalls substituierten Phenylisothiocyanat, umsetzen, wobei unter Konfigurationserhaltung überwiegend ebenfalls das entsprechende N-substituierte Imin (II, $X_2$ + $X_3$ = substituiertes Imino) entsteht.

Zwischenprodukte V werden vorzugsweise hergestellt, indem man eine Verbindung der Formel

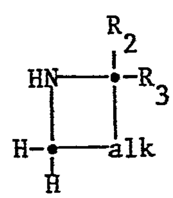

(VII)

mit Natriumnitrit und Salzsäure N-nitrosiert bzw. durch Umsetzung mit Ameisensäureäthylester und anschliessend mit Dimethylsulfat und Cyclohexylamin in das N-Formamidinderivat überführt und das Primärprodukt in Tetrahydrofuran bei höchstens -60°C in Gegenwart von Lithium-N,N-diisopropylamid mit einem Aldehyd der Formel $R_1$-CH=O (VIII) umsetzt. Gemäss einer anderen Methode cyclisiert man eine Verbindung der Formel

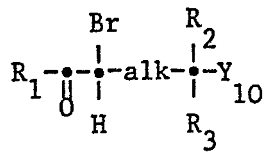

(IX),

worin $Y_{10}$ z.B. Brom bedeutet, durch Behandeln mit Ammoniak oder Benzylamin bzw. setzt man eine Säure der Formel

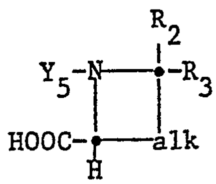

(X),

worin $Y_5$ Wasserstoff oder vorzugsweise Benzyl ist, oder vorzugsweise deren Chlorid, in Gegenwart von Aluminiumtrichlorid mit einer Verbindung $R_1$-H (XI) um, reduziert jeweils in dem Primärprodukt der Formel

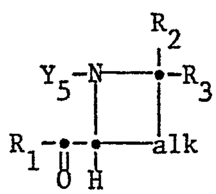

(XII)

die Carbonylgruppe mittels Natriumborhydrid oder Natrium-diäthylaluminiumdihydrid zur Carbinolgruppe und hydriert gegebenenfalls Benzyl $Y_5$ ab. In analoger Weise kann man eine Säure der Formel

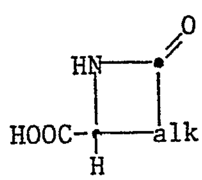

(XIII)

oder vorzugsweise deren Chlorid mit der Verbindung XI umsetzen und in dem Primärprodukt der Formel

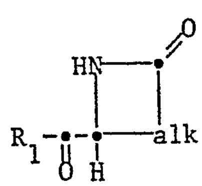

(XIV)

mit Lithiumaluminiumhydrid die exocyclische Carbonyl- zur Carbinol- und die endocyclische Carbonyl- zur Methylengruppe reduzieren.

Man kann aber auch eine Carbonsäure der Formel

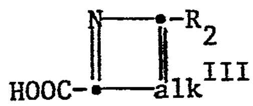

(XV),

worin $alk^{III}$ einen von alk abgeleiteten Niederalkenylylidenrest, dessen freie Valenzen von zueinander 1,3-ständigen C-Atomen ausgehen, z.B. Prop-2-en-1-yl-3-yliden, bedeuten oder vorzugsweise deren Chlorid in Gegenwart von Aluminiumtrichlorid mit der Verbindung XI umsetzen und in dem Reaktionsprodukt der Formel

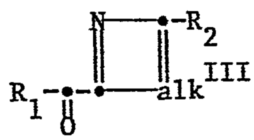

(XVI)

durch Hydrierung in Gegenwart von Adam-Platinoxid gleichzeitig die Carbonylgruppe zur Carbinolgruppe und die Doppelbindungen des Ringes zu Einfachbindungen reduzieren.

Die beschriebenen und weitere Herstellungsverfahren für Zwischenprodukte V verlaufen überwiegend stereoselektiv. So werden bei der von Ausgangsstoffen VII ausgehenden Verfahrensvariante sowie bei der Hydrierung von Ausgangsstoffen XVI überwiegend die erythro-Diastereomeren erhalten. Gewünschtenfalls kann man Verbindungen V in üblicher Weise, z.B. durch Behandeln mit Thionylchlorid und anschliessend mit Wasser oder besser Acetylieren mit Acetanhydrid in N,N-Dimethylformamid, Umsetzung mit Thionylchlorid und Behandlung mit Natronlauge, epimerisieren, d.h. das erythro- in das threo-Diastereomere überführen, und gewünschtenfalls das erhaltene Diastereomere in die Antipoden aufspalten, beispielsweise durch Bildung diastereomerer Salze, z.B. mittels Kampfersulfonsäure, Trennung der diastereomeren Salze und Freisetzung der enantiomeren Basen aus diesen.

Die intramolekulare Cyclisierung von Verbindung III gemäss Verfahrensvariante b) erfolgt spontan oder in Gegenwart eines geeigneten Kondensationsmittel, erforderlichenfalls unter Kühlen oder Erwärmen, vorteilhaft in einem inerten Lösungsmittel. Geeignete Kondensationsmittel sind beispielsweise basische Kondensationsmittel, wie Alkoholate, Hydroxide oder Carbonate von Alkali- oder Erdalkalimetallen, z.B. Natriummethanolat, Kaliumtertiärbutanolat, Kaliumhydroxid oder Kaliumcarbonat, oder tertiäre organische Stickstoffbasen, wie Triniederalkylamine, z.B. Trimethyl- oder Triäthylamin, oder heteroaromatische Stickstoffbasen, z.B. Pyridin, ferner Halogenide von Sauerstoffsäuren des Schwefels oder Phosphors, z.B. Thionylchlorid, Phosphor tribromid oder Phosphoroxychlorid. So erfolgt die Cyclisierung von Verbindungen III, worin $X_1$ Halogen ist, in allgemeinen spontan, während angehend von Verbindungen III, worin $X_1$ veräthertes Hydroxy ist, basische Kondensationsmittel, beispielsweise Alkalimetallalkoholate, wie Natriumniederalkanolat, bzw. ausgehend von Verbindungen III, worin $X_1$ Amino ist, Halogenide oder Sauerstoffsäure des Schwefels, z.B. Thionylchlorid, in Gegenwart tertiärer organischer Stickstoffbasen, z.B. von Triäthylamin oder Pyridin, jeweils reaktionsbeschleunigend wirken.

Zur Herstellung der Zwischenprodukte III geht man vorzugsweise von Verbindungen der Formel

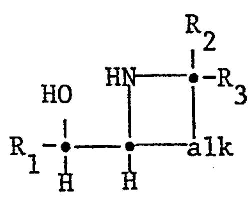

(V)

aus, und setzt diese mit einem geeigneten difunktionellen Derivat der Kohlensäure um. Geeignete difunktionelle Derivate der Kohlensäure sind beispielsweise solche, in denen mindestens eine Hydroxygruppe veräthert oder in reaktionsfähiges verestertes Hydroxy überführt ist, d.h. Verbindungen der Formel $X_1-C(X_2)(X_3)-Y_1$ (VI), worin $X_2$ und $X_3$ gemeinsam für Oxo stehen und $X_1$ und $Y_1$ unabhängig voneinander veräthertes oder reaktionsfähiges verestertes Hydroxy bedeuten und beispielsweise eine der für solche Gruppen $X_1$ angegebenen Bedeutungen hat. Geeignete Kohlensäurederivate VI sind insbesonder Phosgen, Halogenameisensäureniederalkylester oder Diniederalkyl- bzw. gegebenenfalls substituierte Diphenylcarbonate.

Die Reaktionskomponente VI wird dabei vorteilhaft so gewählt, dass die Zwischenprodukte III in situ hergestellt und ohne Isolierung weiterumgesetzt werden können.

Die Bildung und Cyclisierung der Zwischenprodukte III kann stereoselektiv durchgeführt werden. So erfolgt die Bildung der Zwischenprodukte III im allgemeinen unter Konfigurationserhaltung, während die nachfolgende Cyclisierung so gelenkt werden kann, dass Endstoffe gleicher oder umgekehrter Konfiguration gebildet werden. So erhält man durch Umsetzung von Verbindungen III mit Halogenameisensäureniederalkylester (VI; $X_1$ = Niederalkoxy, $X_2 + X_3$ = Oxo, $Y_1$ = Halogen, z.B. Chlor oder Brom) unter Bildung der entsprechenden Zwischenprodukte III überwiegend Endstoffe gleicher Konfiguration. Auch die Umsetzung von Verbindungen V mit Phosgen (VI; $X_1 = Y_1$ = Chlor, $X_2 + X_3$ = Oxo) in Endstoffen I unter intermediärer Bildung von Zwischenprodukten III verläuft unter Konfigurationserhaltung.

In einer besonders bevorzugten Ausführungsform dieses Verfahrens setzt man beispielsweise einen Ausgangsstoff V der gewünschten Konfiguration in Toluol oder einem ähnlichen inerten Lösungsmittel bei etwa 10 bis etwa 35°C, z.B. etwa 30°C mit Phosgen um. Dabei entsteht aus jeder der enantiomeren erythro-Formen V bzw. deren Racemat das jeweilige cis-Oxazolidinon I gleicher absoluter Konfiguration bzw. dessen Racemat bzw. aus jeder der enantiomeren threo-Formen V oder deren Racemat das entsprechende trans-Oxazolidinon I bzw. dessen Racemat.

Die Absättigung der Doppelbindung(en) des Oxazolinringes und gegebenenfalls des Restes $alk^I$ gemäss der Verfahrensvariante c) erfolgt vorzugsweise durch katalytische Hydrierung, d.h. Behandlung mit Wasserstoff in Gegenwart eines Hydrierungskatalysators. Geeignete Hydrierungskatalysatoren sind dabei beispielsweise Palladium-, Platin-und Nickelkatalysatoren, wie Platinoxyd, z.B. Adams-Platinoxid, Palladium, z.B. Palladiumkohle, oder Raney-Nickel. Erforderlichenfalls arbeitet man unter Erwärmen und/oder erhöhtem Druck.

Zwischenprodukte IV können beispielsweise hergestellt werden, indem man eine entsprechende Verbindung der Formel

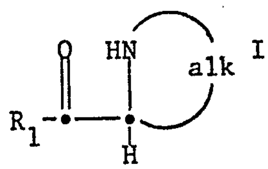

(XVII)

mit einer Verbindung der Formel $X_1-C(X_2)(X_3)-Y_1$ (VI), worin $X_1$, $X_2$, $X_3$ und $Y_1$ die für die Verfahrensvariante b) angegebenen Bedeutungen haben, umsetzt, vorzugsweise unter stark basischen Bedingungen, z.B. in Gegenwart von Natriumhydrid, Natriumamid oder Lithium-N,N-diisopropylamid.

Zur erwähnten Auftrennung eines erfindungsgemäss erhältlichen Isomerengemisches bzw. zur Isolierung der gewünschten Komponente aus einem solchen ist insbesondere folgendes zu bemerken:

Diastereomerengemische, z.B. Gemische von cis- und trans-Isomeren, können auf Grund ihrer unterschiedlichen physikalischen Eigenschaften nach den üblichen chemischen und physikalisch-chemischen Trennverfahren, z.B. durch fraktionierte Kristallisation, Destillation, Chromatographie und andere Phasenverteilungsverfahren, aufgetrennt werden.

Enantiomerengemische, wie spaltbare Gemische der beiden und gegebenenfalls weitere Enantiomeren gleichsinniger gegenseitiger Orientierung der Wasserstoffatome in 4- und 5-Stellung des Oxazolidinringes, können nach üblichen Racematspaltungsverfahren, z.B. Kristallisation aus einem optisch aktiven Lösungsmittel, Chromatographie an einer optisch aktiven stationären oder mobilen Phase, oder intermediäre Bildung einer diastereomeren Hilfsverbindung, z.B. eines diastereomeren Säureadditionssalzes, mit einer optisch aktiven Carbon- oder Sulfonsäure, z.B. mit der D- oder L-Form von Weinsäure, Di-o-toluylweinsäure, Aepfelsäure, Mandelsäure, Chinasäure oder einer Camphersulfonsäure, Trennung der Diastereomeren und Freisetzung der enantiomeren Verbindung I aufgetrennt werden. Vorzugsweise isoliert man jeweils wirksamere Enantiomere.

Als Umwandlungsreaktionen erfindungsgemäss erhältlicher Verbindungen in andere Verbindungen der Formel I sind insbesondere Einführungs-, Umwandlungs- und Abspaltungsreaktionen am Rest $R_1$ zu erwähnen.

Beispielsweise kann man in den Rest $R_1$ durch Behandlung mit Chlor bzw. Brom oder N-Bromsuccinimid Halogen einführen, oder Nitro zu Amino reduzieren und/oder Amino durch Halogen, Cyano, Niederalkoxy oder Trifluormethyl ersetzen.

Die gegenseitige Ueberführung freier Verbindungen und ihrer Salze ineinander erfolgt beispielsweise, indem man eine freie Verbindung mit einer Säure bzw. ein Salz mit einer Base oder einem Salz einer anderen Säure umsetzt. Salze der neuen Verbindungen können auch zur Reinigung derselben sowie, wie erwähnt, zur Enantiomerentrennung dienen, indem man eine verfahrensgemäss erhältliche Verbindung in ein Salz überführt, dieses reinigt bzw. in die Diastereomeren auftrennt und aus dem Salz dann die freie Verbindung wieder freisetzt und gewünschtenfalls in ein anderes Salz überführt.

So können erhaltene freie Verbindungen in an sich bekannter Weise in Säureadditionssalze überführt werden, z.B. durch Umsetzen einer Lösung der freien Verbindung in einem geeigneten Lösungsmittel oder

Lösungsmittelgemisch mit einer der vorgenannten Säuren oder mit einer Lösung davon, oder mit einem geeigneten Kationenaustauscher.

Erhaltene Säureadditionssalze können in an sich bekannter Weise in die freien Verbindungen umgewandelt werden, z.B. durch Behandeln mit einer Base, wie einem Alkalimetallhydroxid, einem Metallcarbonat oder -hydrogencarbonat, oder Ammoniak, oder mit einem geeigneten Anionenaustauscher.

Erhaltene Säureadditionssalze können in an sich bekannter Weise in andere Säureadditionssalze überführt werden, z.B. durch Behandlung eines Salzes einer organischen Säure mit einem geeigneten Metallsalz, wie einem Natrium-, Barium- oder Silbersalz, einer Säure in einem geeigneten Lösungsmittel, in welchem ein sich bildendes anorganisches Salz unlöslich ist und damit aus dem Reaktionsgemisch ausscheidet.

Die neuen Verbindungen, einschliesslich ihrer Salze können auch in Form der Hydrate erhalten werden oder das zur Kristallisation verwendete Lösungsmittel einschliessen.

Infolge der engen Beziehung zwischen den neuen Verbindungen in freier Form und in Form ihrer Salze sind im vorausgegangenen und nachfolgend unter den freien Verbindungen oder ihren Salzen sinn- und zweckmässig gegebenenfalls auch die entsprechenden Salze bzw. freien Verbindungen zu verstehen.

Die Erfindung betrifft auch diejenigen Ausführungsformen des Verfahrens, bei denen man von einer auf beliebiger Verfahrensstufe als Zwischenprodukt erhältlichen Verbindung ausgeht und die fehlenden Verfahrensschritte ausführt oder einen Ausgangsstoff unter den Reaktionsbedingungen bildet oder in Form eines Derivats davon, gegebenenfalls eines Salzes, verwendet.

Beim Verfahren der vorliegenden Erfindung werden vorzugsweise solche Ausgangsstoffe verwendet, welche zu den eingangs als besonders wertvoll geschilderten Verbindungen führen. Neue Ausgangsstoffe sowie Verfahren zu ihrer Herstellung bilden ebenfalls einen Gegenstand der vorliegenden Erfindung.

Die als Zwischenprodukte genannten Verbindungen der Formel V und ihre pharmazeutisch verwendbaren Salze weisen ähnliche pharmakologische Eigenschaften auf wie die Verbindung der Formel I und können dementsprechend ebenfalls als Antidepressiva, wenn auch mit abweichendem, eher auf einer Dopaminfreisetzung beruhendem pharmakologischen Profil, Verwendung finden. So stimulieren sie beispielsweise im Dosisbereich von etwa 3 bis 30 mg/kg p.o. die lokomotorische Aktivität der Maus, gemessen mittels des "Electronic Motility Meter". Ebenso hemmen sie im Dosisbereich von etwa 50 bis 100 mg/kg p.o. die Noradrenalin-Aufnahme im Rattenhirn in der Versuchsanordnung von Maître, Stähelin und Bein, Pharmacol. 20, 2169-2186 (1971).

Die Erfindung betrifft dementsprechend ebenso Verbindungen der Formel

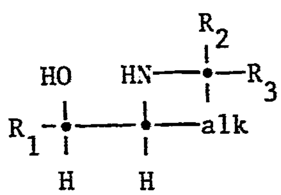

(V),

worin $R_1$ einen Aryl- oder Heteroarylrest bedeutet, $R_2$ und $R_3$ unabhängig voneinander Niederalkyl oder gemeinsam Niederalkylen darstellen und alk für Niederalkylen steht, dessen Valenzen von benachbarten oder zueinander 1,3-ständigen Kohlenstoffatomen ausgehen, und ihre Salze, Verfahren zu ihrer Herstellung, diese enthaltende pharmazeutische Präparate und ihre Verwendung als Arzneimittelwirkstoffe sowie als Zwischenprodukte zur Herstellung von Verbindungen der Formel I.

Dabei haben Aryl- bzw. Heteroarylreste $R_1$, Niederalkyl- bzw. Niederalkylenreste $R_2$ und $R_3$ sowie Niederalkylenreste alk beispielsweise die eingangs unter der Formel I angegebenen Bedeutungen.

Ebenso sind Salze von Verbindungen der Formel V vorzugsweise pharmazeutisch verwendbare Säureadditionssalze, beispielsweise mit den für Salze von Verbindungen der Formel I genannten Säuren.

Die Verbindungen der Formel V weisen ebenfalls mindestens 2 asymmetrische C-Atome auf und können dementsprechend in mindestens 4 zueinander stereoisomeren Formen, in (+)-threo-, (-)-threo-, (+)-erythro- und (-)-erythro-Form auftreten. Sind zusätzlich die Variablen $R_2$ und $R_3$ verschieden oder weisen die durch $R_1$, $R_2$, $R_3$ und/oder alk dargestellten Teilstrukturen mindestens ein weiteres Asymmetriezentrum auf, sind noch weitere stereoisomere Formen möglich. Diese entsprechen sämtlich der Formel V und sind dementsprechend sämtlich Gegenstand der Erfindung. Bevorzugt sind jedoch jeweils das threo-Diastereomere und davon abgeleitete Diastereomerengemische mit mindestens einem weiteren Asymmetriezentrum, sowohl in Form von Enantiomerengemischen als auch in Form individueller Enantiomerer.

Die Erfindung betrifft in erster Linie Verbindungen der Formel V, worin $R_1$ einen als Substituenten gegebenenfalls Niederalkyl, Niederalkoxy, Halogen, Cyano und/oder Trifluormethyl aufweisenden 6-gliedrigen monocyclischen oder mindestens einen 6-gliedrigen Ring aufweisenden bicyclischen Arylrest oder monocyclischen, als Heteroatom(e) Stickstoff, Sauerstoff, Schwefel, Sauerstoff sowie Stickstoff oder Schwefel sowie Stickstoff aufweisenden 5-gliedrigen oder als Heteroatom(e) 1 oder 2 Stickstoffatome aufweisenden 6-gliedrigen Heteroarylrest bedeutet, $R_2$ und $R_3$ unabhängig voneinander Niederalkyl oder gemeinsam Niederalkylen darstellen und alk Niederalkylen bedeutet, dessen freie Valenzen von benachbarten oder zueinander 1,3-ständigen C-Atomen ausgehen, vor allem in Form ihrer threo-Diastereomeren, und ihre Salze, insbesondere pharmazeutisch verwendbaren Salze.

Die Erfindung betrifft vor allem Verbindungen der Formel V, worin $R_1$ einen unsubstituierten oder durch Niederalkyl mit bis und mit

4 C-Atomen, wie Methyl, Niederalkoxy mit bis und mit 4 C-Atomen, wie Methoxy, Halogen der Atomnummer bis und mit 35, wie Chlor, und/oder Trifluormethyl substituierten Phenyl-, Naphthyl-, wie 1- oder 2-Naphthyl-, Furyl-, wie 2-Furyl-, Thienyl-, wie 2-Thienyl-, Pyrrolyl, wie 1-Pyrrolyl, Thiazolyl-, wie 2-Thiazolyl-, Oxazolyl-,wie 2-Oxazolyl-, Pyridyl-, wie 3- oder 4-Pyridyl-, Pyrimidinyl-, wie 2- oder 4-Pyrimidinyl- oder Pyrazinyl-, wie 2-Pyrazinylrest bedeutet, $R_2$ und $R_3$ unabhängig voneinander Niederalkyl mit bis und mit 4 C-Atomen, wie Methyl, oder gemeinsam Niederalkylen mit bis und mit 7 C-Atomen, dessen freie Valenzen von benachbarten oder zueinander 1,3-, 1,4- oder 1,5-ständigen C-Atomen ausgeht, wie Aethylen, 1,3-Propylen, 1,4-Butylen, 1,5-Pentylen bzw. 1,5-(3,3-Dimethyl)-pentylen, darstellen und alk Niederalkylen mit bis und mit 7 C-Atomen, dessen freie Valenzen von benachbarten oder zueinander 1,3-ständigen C-Atomen ausgehen, wie Aethylen, 1,3-Propylen, 1,2-(2-Methyl)-propylen, 1,3-(3-Methyl)-butylen oder 1,3-(2,2-Dimethyl)-propylen, bedeutet, vor allem in Form der threo-Diastereomeren und ihre Salze, insbesondere pharmazeutisch verwendbaren Salze.

Die Erfindung betrifft insbesondere Verbindungen der Formel V, worin $R_1$ unsubstituiertes oder durch Niederalkyl mit bis und mit 4 C-Atomen, wie Methyl, Niederalkoxy mit bis und mit 4 C-Atomen, wie Methoxy, Halogen der Atomnummer bis und mit 35, wie Chlor, und/oder Trifluormethyl substituiertes Phenyl oder unsubstituiertes Furyl, z.B. 2-Furyl, Thienyl z.B. 2-Thienyl, oder Pyridyl, z.B. 3- oder 4-Pyridyl, bedeutet, $R_2$ und $R_3$ jeweils gleiche Niederalkylreste mit bis und mit 4 C-Atomen, z.B. Methyl, oder gemeinsam Niederalkylen mit bis und mit 7 C-Atomen, dessen freie Valenzen von benachbarten oder zueinander 1,4- oder 1,5-ständigen C-Atomen ausgehen, wie 1,4-Butylen oder 1,5-Pentylen, darstellen und alk für Aethylen steht, vor allem in Form der threo-Diastereomeren, und ihre, insbesondere pharmazeutisch verwendbaren Salze.

Die Erfindung betrifft namentlich die in den Beispielen genannten Verbindungen der Formel V und ihre Salze.

Die Verbindungen der Formel V können nach an sich bekannten Methoden hergestellt werden, beispielsweise indem man

d) aus einer Verbindung der Formel

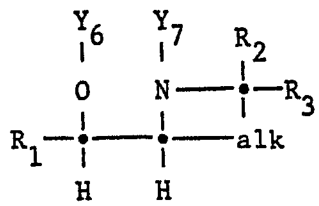

(XVIII),

worin einer der Reste $Y_6$ und $Y_7$ einen abspaltbaren Rest und der andere Wasserstoff bedeutet, oder einem Salz davon den abspaltbaren Rest unter Einführung eines Wasserstoffatoms abspaltet oder

e) eine Verbindung der Formel

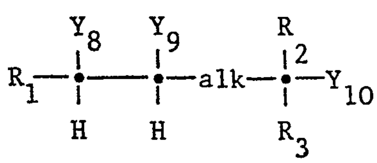

(XIX),

worin $Y_8$ Hydroxy ist und einer der Reste $Y_9$ und $Y_{10}$ reaktionsfähiges verestertes Hydroxy bedeutet oder $Y_8$ und $Y_9$ gemeinsam für Epoxy stehen, wobei der verbleibende Rest $Y_9$ bzw. $Y_{10}$ gegebenenfalls in intermediär geschützter Form vorliegendes und/oder durch einen abspaltbaren Rest substituiertes Amino darstellt, oder ein Salz davon cyclisiert oder

f) in einer Verbindung der Formel

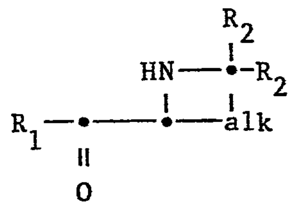

(XX),

in der das Stickstoffatom intermediär geschützt sein kann, oder in einem Salz davon die Carbonyl- zur Carbinolgruppe reduziert, erforderlichenfalls die Schutzgruppe abspaltet und gewünschtenfalls die gemäss einer der Verfahrensvarianten d) bis f) erhältliche Verbindung epimerisiert, ein verfahrensgemäss erhältliches Isomerengemisch in die Komponenten auftrennt bzw. aus einem solchen das oder die gewünschte(n) Isomere(n) abtrennt, eine verfahrensgemäss erhältliche Verbindung in eine andere Verbindung der Formel V umwandelt und/oder eine verfahrensgemäss erhältliche freie Verbindung in ein Salz bzw. ein verfahrensgemäss erhältliches Salz in die freie Verbindung oder in ein anderes Salz überführt.

Abspaltbare Reste $Y_6$ bzw. $Y_7$ sind beispielsweise Acylgruppen, Silylgruppen oder gegebenenfalls substituierte α-Aralkylgruppen, Reste $Y_7$ ebenso α-Carbanionen stabilisierende Gruppen. Als Acylgruppen kommen beispielsweise von organischen Carbonsäuren abgeleitete Acylgruppen, wie Niederalkanoyl oder gegebenenfalls substituiertes Aroyl bzw. Heteroaroyl, beispielsweise der Formel $R_1$-C(=O)-, als Silylgruppen beispielsweise Triniederalkylsilylgruppen, wie Trimethylsilyl, und als gegebenenfalls substituierte α-Aralkylgruppen beispielsweise gegebenenfalls durch Niederalkyl, Niederalkoxy, Halogen und/oder Nitro substituierte α-Phenyl-, α,α-Diphenyl- oder α,α,α-Triphenylniederalkylgruppen, z.B. Benzyl, ferner Di- oder Triphenylmethyl, in Betracht. α-Carbanionen stabilisierende Gruppen $Y_7$ sind beispielsweise Nitroso- oder N-substituierte Iminomethylgruppen sowie sterisch gehinderte, von Carbon- oder Thiocarbonsäuren abgeleitete

Acylgruppen. Als Substituenten von N-substituierten Iminomethyl kommen beispielsweise aliphatische und cycloaliphatische Reste, wie Niederalkyl, z.B. Butyl oder Tertiärbutyl, oder Cycloalkyl mit 5 bis 8 Ringgliedern, z.B. Cyclopentyl oder Cyclohexyl, in Betracht. Acylgruppen der angegebenen Art sind beispielsweise 2,4,6-Triisopropyl- bzw. 2,4,6-Tritertiärbutylbenzoylgruppen und Tertiärbutylthiocarbonylgruppen. Abspaltbare Reste als Substituenten der gegebenenfalls durch α-Aralkyl geschützten Aminogruppe von Verbindungen der Formel XIX sind beispielsweise α-halogenierte Niederalkanoylgruppen, wie Trifluoracetyl.

Als Schutzgruppen von Amino in Verbindungen der Formel XIX sowie als Schutzgruppen für das Stickstoffatom von Verbindungen XX kommen die üblichen Stickstoff-Schutzgruppen, beispielsweise die als $Y_6$ bzw. $Y_7$ genannten, vorzugsweise α-Aralkyl, wie Benzyl, in Betracht.

Salze von Zwischenprodukten XVII, XIX bzw. XX sind beispielsweise deren Säureadditionssalze, wie Hydrohalogenide.

Die Durchführung der verfahrensgemässen Reaktionen sowie die Herstellung neuer Ausgangsstoffe bzw. Zwischenprodukte erfolgt in Analogie zur Reaktions- und Bildungsweise bekannter Ausgangsstoffe bzw. Zwischenprodukte ähnlicher Struktur. Dabei werden, auch wenn nachstehend nicht ausdrücklich erwähnt, die jeweils üblichen Hilfsmittel, wie Katalysatoren, Kondensations- sowie Solvolysemittel und/oder Lösungsmittel, und Reaktions-, wie Temperatur- und Druckbedingungen, sowie gegebenenfalls Schutzgase verwendet.

Die Abspaltung von Resten $Y_6$ bzw. $Y_7$ gemäss der Verfahrensvariante d) erfolgt beispielsweise durch Solvolyse oder Reduktion. So kann man Acylgruppen, ausser den genannten trisubstituierten Benzoylgruppen $Y_7$, die reduktiv abgespalten werden, und Silylgruppen sowie N-substituierte Iminomethyl- und Tertiärbutylthiocarbonylgruppen $Y_7$ hydro-

lytisch, Acylgruppen $Y_6$ ferner alkoholytisch und Nitrosogruppen $Y_7$ ferner acidolytisch abspalten. α-Aralkylgruppen sowie die genannten trisubstituierten Benzoylgruppen $Y_7$ werden insbesondere reduktiv abgespalten.

Die Hydrolyse, Alkoholyse bzw. Acidolyse erfolgt durch Behandeln mit Wasser, einem Alkohol oder einer Säure, erforderlichenfalls in Gegenwart eines Solvolysemittels und/oder eines Lösungs- oder Verdünnungsmittels. Solvolysemittel für die Hydrolyse und Alkoholyse sowie für die Acidolyse geeignete Säuren sind beispielsweise Mineralsäuren, z.B. Chlor- bzw. Bromwasserstoffsäure oder Schwefelsäure, Sulfonsäuren, z.B. p-Toluolsulfonsäure, gegebenenfalls halogenierte Niederalkansäuren, z.B. Essigsäure oder Trifluoressigsäure, oder dergleichen, Solvolysemittel für die Hydrolyse und Alkoholyse ebenso basische Mittel, wie Hydroxide von Alkali- oder Erdalkalimetallen, z.B. Natronlauge oder Kalilauge, für die Alkoholyse von Acyl $Y_6$ ferner Alkoholate, z.B. Natriummethanolat. Lösungs- oder Verdünnungsmittel sind für die Hydrolyse beispielsweise mit Wasser mischbare Lösungsmittel, für die Alkoholyse insbesondere Alkohole im Ueberschuss. In einer bevorzugten Ausführungsform dieses Solvolyseverfahrens spaltet man beispielsweise eine Verbindung XVIII, worin $Y_6$ Wasserstoff und $Y_7$ Nitroso ist, durch Behandeln mit Chlorwasserstoff in Benzol oder einem Gemisch von Toluol und Dioxan oder mit Bromwasserstoff in Essigsäure. In einer anderen bevorzugten Ausführungsform hydrolysiert man eine Verbindung XVIII, worin $Y_6$ Wasserstoff und $Y_7$ N-substituiertes Iminomethyl ist, in Gegenwart eines Alkalimetallhydroxides, z.B. von Kaliumhydroxid, unter Erwärmen, z.B. auf etwa 50-80°C, in einem wässrigen Niederalkanol, z.B. in Methanol/Wasser oder Aethanol/Wasser.

Die reduktive Abspaltung von α-Aralkylgruppen und von Nitroso $Y_7$ erfolgt beispielsweise durch katalytische Hydrierung, die reduktive Abspaltung von Acyl $Y_6$ beispielsweise durch Umsetzung mit einem Dileichtmetallhydrid. Zur katalytischen Hydrierung behandelt man

beispielsweise das Zwischenprodukt XVIII in Gegenwart eines Platin-, Palladium- oder Nickelkatalysators, z.B. von Platinoxid, Palladium auf Kohle oder Raney Nickel, mit der stöchiometrisch erforderlichen Wasserstoffmenge, vorzugsweise in einem inerten Lösungsmittel, erforderlichenfalls unter Druck und/oder Erwärmen. In einer bevorzugten Ausführungsform hydriert man beispielsweise ein Zwischenprodukt XVIII, worin $Y_6$ Wasserstoff und $Y_7$ 1) Nitroso bzw. 2) Benzyl ist, 1) in Gegenwart von Raney Nickel oder 2) bei etwa 40-60°C, vorzugsweise in methanolischer Lösung bei normalem oder höchstens geringfügig erhöhtem Druck, in Gegenwart von Palladium-Kohle. Die reduktive Abspaltung von Acyl $Y_6$ erfolgt beispielsweise durch Behandeln mit Natriumborhydrid oder Lithiumaluminiumhydrid.

Zwischenprodukte XVIII, worin $Y_6$ Hydroxy und $Y_7$ einen α-Aralkylrest bedeutet, werden beispielsweise hergestellt, indem man eine Verbindung der Formel

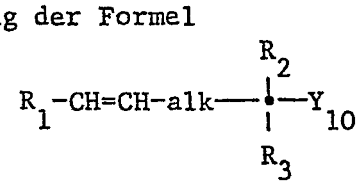

(XXI),

worin $Y_{10}$ z.B. Brom oder Chlor bedeutet, entweder mit Benzylamin umsetzt, das Reaktionsprodukt (XXI; $Y_{10}$ = Benzylamino) N-trifluoracetyliert, mit N-Bromsuccinimid zu einer Verbindung der Formel

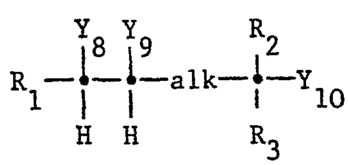

(XIX),

worin $Y_8$ Hydroxy, $Y_9$ Brom und $Y_{10}$ N-Benzyltrifluoracetylamino ist, umsetzt und dieses cyclisiert, oder die Verbindung XXI mit Wasserstoffperoxid epoxidiert und das Reaktionsprodukt (XIX; $Y_8$ + $Y_9$ = Epoxy, $Y_{10}$= Brom oder Chlor) mit Benzylamin cyclisiert.

Die Ausgangsstoffe XXI können beispielsweise durch übliche Umsetzung einer Verbindung der Formel

$R_1$—•=P$(C_6H_5)_3$ (XXII)

mit einem Aldehyd der Formel

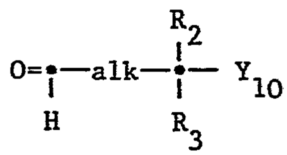

(XXIII)

erhalten werden.

Zwischenprodukte XVIII, worin $Y_6$ Hydroxy und $Y_7$ eine α-Carbanionen stabilisierende Gruppe bedeutet, werden beispielsweise erhalten, indem man eine Verbindung der Formel

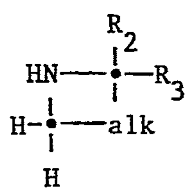

(VII)

in üblicher Weise durch einen entsprechenden Rest $Y_7$ substituiert, z.B. N-nitrosiert oder N-formamidiert, und das Reaktionsprodukt der Formel

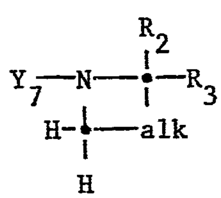

(XXVIII)

worin $Y_7$ z.B. Nitroso oder N-substituiertes Iminomethyl oder sterisch gehindertes Acyl ist, in Gegenwart einer Metallbase metalliert und anschliessend mit einem Aldehyd der Formel $R_1$-CH=O (VIII) kondensiert. Metallbasen sind beispielsweise Kohlenwasserstoffverbindungen, Amide oder Alkoholate von Alkalimetallen, wie Lithium oder Kalium, z.B. Methyllithium, Butyllithium, Sekundärbutyllithium, Tertiärbutyllithium, Phenyllithium, Lithium-N,N-diisopropylamid oder Kaliumtertiärbutanolat. So kann man das Zwischenprodukt VII beispielsweise durch Umsetzung mit einem Alkylnitrit oder mit salpetriger Säure bzw. einem Alkalimetallnitrit unter sauren Bedingungen N-nitrosieren und das Reaktionsprodukt XXVIII, beispielsweise in Gegenwart von Lithium-N,N-diisopropylamid, vorzugsweise in Tetrahydrofuran bei höchstens -60°C, oder in Gegenwart von Kaliumtertiärbutanolat bei etwa -20° bis etwa 0°C, ebenfalls vorzugsweise in Tetrahydrofuran, mit dem Aldehyd VIII zur Reaktion bringen. Dabei bildet sich vorzugsweise das erythro-Diastereomere V. Das Zwischenprodukt VII kann aber auch, z.B. durch Umsetzung mit einem Ameisensäureester, wie einem Niederalkylformiat, N-formyliert das Reaktionsprodukt mit einem Methylierungsmittel, z.B. mit Dimethylsulfat, zur entsprechenden Immoniumverbindung der Formel

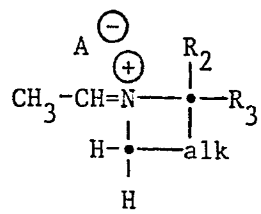

(XXIX),

worin $A^{\ominus}$ ein Anion z.B. das Methosulfat-Ion ist, umgesetzt, dieses mit einem primären Amin, z.B. mit Cyclohexylamin, und anschliessende Basebehandlung in die entsprechende N-Formamidinverbindung XXVIII ($Y_7$ z.B. $-CH=N-c-C_6H_{11}$) überführt und diese, beispielsweise in Gegenwart von Tertiärbutyllithium, vorzugsweise bei höchstens -20°C, α-metalliert und anschliessend mit dem Aldehyd VIII umgesetzt werden. Dabei erhält man die entsprechende N-Formamidinverbindung XVIII, die gewünschtenfalls, z.B. durch Behandlung mit wässrigem Methanol, zur entsprechenden N-Formylverbindung XVIII hydrolysiert werden kann.

Zwischenprodukte XVIII, worin $Y_6$ Acyl und $Y_7$ Wasserstoff ist, werden beispielsweise erhalten, indem man eine Verbindung der Formel

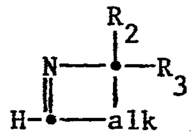

(XXXI) ,

erhältlich beispielsweise durch N-Chlorierung der entsprechenden gesättigten Verbindung

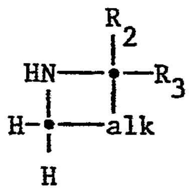

(VII),

z.B. mit Chloramin T, und anschliessender Chlorwasserstoffabspaltung, mit Cyanwasserstoffsäure umsetzt, das Reaktionsprodukt der Formel

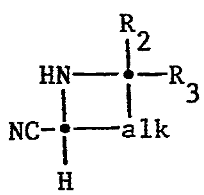

(XXXII)

N-acyliert, z.B. durch Umsetzung mit dem Chlorid einer Säure $R_1$-COOH (XXXIII), und anschliessend in üblicher Weise, beispielsweise in Gegenwart von Lithium-N,N-diisopropylamid, vorzugsweise in Tetrahydrofuran bei höchstens -60°C, mit einem Aldehyd $R_1$-CH=O (VIII) umsetzt.

Die Cyclisierung von Verbindungen XIX gemäss Verfahrensvariante e) erfolgt erforderlichenfalls in Gegenwart eines basischen Kondensationsmittels, wie eines Alkali- oder Erdalkalimetallhydroxides, z.B. von Natrium- oder Kaliumhydroxid, in An- oder Abwesenheit eines Lösungs- oder Verdünnungsmittels, vorzugsweise im Temperaturbereich

von etwa 20 bis etwa 80°C. So cyclisiert man Verbindungen XIX, worin $Y_8$ Hydroxy, $Y_9$ reaktionsfähiges verestertes Hydroxy und $Y_{10}$ gegebenenfalls in intermediär geschützter Form und/oder durch einen abspaltbaren Rest substituiertes Amino, z.B. Trifluoracetylamino oder N-Benzyltrifluoracetylamino, bedeutet, vorteilhaft in einem mit Wasser mischbaren organischen Lösungsmittel, z.B. in Dioxan, durch Behandeln mit wässriger Natron- oder Kalilauge, vorzugsweise unter Erwärmen, z.B. auf etwa 40 bis etwa 80°C. Die Cyclisierung von Verbindungen XIX, worin $Y_8$ und $Y_9$ gemeinsam für Epoxy stehen und $Y_{10}$ gegebenenfalls in intermediär geschützter Form vorliegendens Amino, z.B. Amino oder Benzylamino, oder $Y_8$ Hydroxy, $Y_9$ gegebenenfalls in intermediär geschützter Form vorliegendes Amino, z.B. Amino oder Benzylamino, und $Y_{10}$ reaktionsfähiges verestertes Hydroxy ist, erfolgt im allgemeinen spontan, z.B. beim Stehenlassen bei Raumtemperatur.

Zwischenprodukte XIX, worin $Y_8$ Hydroxy, $Y_9$ Brom und $Y_{10}$ N-Benzyltrifluoracetylamino ist, werden beispielsweise erhalten, indem man eine Verbindung der Formel

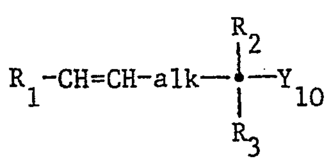

(XXI),

worin $Y_{10}$ Brom ist, zunächst mit Benzylamin zur Reaktion bringt, das Reaktionsprodukt (XXI; $Y_{10}$ = Benzylamino) in Gegenwart von Kaliumcarbonat mit Trifluoracetanhydrid N-trifluoracetyliert und anschliessend in Gegenwart von Schwefelsäure mit N-Bromsuccinimid umsetzt, vorzugsweise in Dioxan.

Zwischenprodukte XIX, worin $Y_8$ und $Y_9$ gemeinsam für Epoxy stehen und $Y_{10}$ gegebenenfalls in intermediär geschützter Form vorliegendes oder durch einen abspaltbaren Rest substituiertes Amino, z.B. Amino oder Benzylamino, ist bzw. $Y_8$ Hydroxy, $Y_9$ reaktionsfähiges

verestertes Hydroxy und $Y_{10}$ gegebenenfalls in intermediär geschützter Form vorliegendes oder durch einen abspaltbaren Rest substituiertes Amino, z.B. Amino oder Benzylamino, bedeutet, können ausgehend von Verbindungen XXI ($Y_{10}$ = reaktionsfähiges verestertes Hydroxy) durch Epoxidierung, z.B. mittels Wasserstoffperoxid, und anschliessende Umsetzung mit Ammoniak oder einem geeigneten, bei der Umsetzung eine, z.B. wie angegeben, geschützte bzw. substituierte Aminogruppe ergebenden Derivat und gegebenenfalls Ringöffnung mit einer Säure $Y_9$-H davon erhalten werden.

Die Herstellung der für diese Umsetzung zu verwendenden Ausgangsstoffe XXI ($Y_{10}$ = Brom) ist unter der Verfahrensvariante d) beschrieben.

Die Reduktion der Carbonylgruppe in Verbindungen XX gemäss Verfahrensvariante f) erfolgt beispielsweise durch Behandeln mit einem Leichtmetall- oder Dileichtmetallhydrid oder durch katalytische Hydrierung.

Geeignete Leichtmetall- bzw. Dileichtmetallhydride sind beispielsweise Borhydride, wie der Boran/Tetrahydrofurankomplex, Alkalimetallborhydride, wie Natriumborhydrid, und Alkalimetallaluminiumhydride, wie Lithiumaluminiumhydrid oder Natriumdiäthylaluminiumdihydrid. Durch geeignete Wahl des Reduktionsmittels kann die Reduktion so gelenkt werden, dass zusammen mit der Reduktion der Carbonylgruppe auch die Schutzgruppe abgespalten wird. So kann man ein Zwischenprodukt XX, worin das Stickstoffatom acyliert ist, direkt zur entsprechenden Verbindung V durchreduzieren. Ferner wird bei der Verwendung von Natriumborhydrid als Reduktionsmittel vorwiegend das eine, bei Verwendung von Natriumdiäthylaluminiumdihydrid vorwiegend das andere Diastereomere erhalten.

Die katalytische Hydrierung der Carbonylgruppe erfolgt beispielsweise durch Einwirkung von Wasserstoff in Gegenwart eines geeigneten Edelmetallkatalysators, z.B. von Adam-Platinoxid, vorzugsweise im Temperaturbereich von etwa 40 bis etwa 60°C, wobei gleichzeitig auch eine α-Aralkylgruppe reduktiv abgespalten werden kann.

Die Zwischenprodukte XX werden beispielsweise erhalten, indem man eine Verbindung der Formel

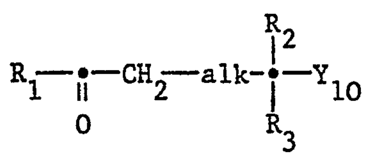 (XXIV),

worin $Y_{10}$ reaktionsfähiges verestertes Hydroxy bedeutet, in α-Stellung zur Carbonylgruppe halogeniert, z.B. durch Behandeln mit Brom, und das Reaktionsprodukt der Formel

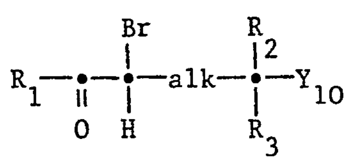 (IX)

mit Ammoniak oder einem geeigneten, bei der Umsetzung eine, z.B. wie angegeben, geschützte bzw. substituierte Aminogruppe ergebenden Derivat davon cyclisiert. Ausgangsstoffe XXIV erhält man z.B. durch Umsetzung eines Niederalkylesters einer Säure

$$R_1—Y_{11}—CH_2—alk—COOH \qquad (XXV)$$

worin $Y_{11}$ Diniederalkoxy- oder Niederalkylendioxymethylen, z.B. Diäthoxy- oder Aethylendioxymethylen, ist mit je einem Mol einer metallorganischen Verbindung der Formel $R_2$-M (XXVI) und $R_3$-M (XXVII), worin M einen Rest der Formel -Li, -Na oder Mg-Halogen bedeutet, und Ueberführung der gebildeten Hydroxygruppe in reaktionsfähiges verestertes Hydroxy und Ketalspaltung, z.B. mittels Chlor- oder Bromwasserstoffsäure.

Die Zwischenprodukte XX können ferner hergestellt werden, indem man eine Verbindung der Formel $R_1$-H (XI) in Gegenwart einer Lewissäure, z.B. von Aluminiumtrichlorid, mit einer Säure der Formel

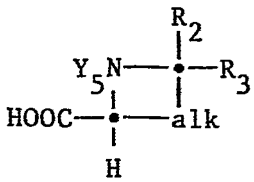

(X)

worin $Y_5$ Wasserstoff oder α-Aralkyl, z.B. Benzyl, ist, oder einem reaktiven Säurederivat, z.B. dem Chlorid, derselben umsetzt.

Die Epimerisierung verfahrensgemäss erhältlicher Verbindungen der Formel V erfolgt im Prinzip durch jeweils nukleophile Substitution und Wiedereinführung der Hydroxygruppe, wobei ein Schritt unter Konfigurationsumkehr, der andere unter Konfigurationserhaltung abläuft. So kann man eine Verbindung V mit erythro-Konfiguration durch Umsetzung mit einem Alkalimetallcyanat und Salzsäure zur entsprechenden N-Carbamylverbindung mit erythro-Konfiguration kondensieren, dieses mit Thionylchlorid zur entsprechenden trans-Verbindung der Formel

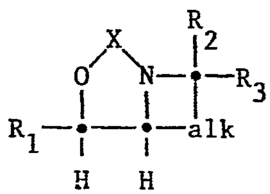

(II),

worin X Iminomethylen ist, mit threo-Konfiguration cyclisieren und dieses durch Behandeln mit einem Alkalimetallhydroxid, z.B. mit Kaliumhydroxid, in einem wässrigen Alkanol, z.B. 50%-igen Methanol, zur entsprechenden threo-Verbindung V hydrolysieren und umgekehrt. Eine Variante dieses Verfahrens besteht darin, dass man die Verbindung V zunächst durch Behandeln mit einem Carbonsäure-, z.B. Niederalkansäureanhydrid der Formel R'-C(=O)-$Y_{12}$ (XXVIII), worin R' einen Kohlenwasserstoffrest und $Y_{12}$ Chlor oder eine Gruppe -O-C(=O)-R' bedeutet, vor allem mit Acetanhydrid,acyliert, das Reaktionsprodukt mit Thionylchlorid zum entsprechenden Oxazolidiniumchlorid der Formel II, worin X eine Gruppe der Formel $>C^{\oplus}-R'Cl^{\ominus}$ bedeutet, umsetzt und anschliessend, z.B. mit Natronlauge hydrolysiert. Auch dabei erfolgt Epimerisierung unter Konfigurationsumkehr am exocyclischen C-Atom.

Die fakultative Auftrennung eines verfahrensgemäss erhältlichen Isomerengemisches bzw. die Abtrennung des gewünschten Isomeren aus einem solchen und die Aufspaltung in die Enantiomeren erfolgt in analoger Weise wie für Verbindungen der Formel I angegeben, vor allem aber durch Bildung diastereomerer Zwischenverbindungen, die ihrerseits wieder unterschiedliche physikalische Eigenschaften besitzen, Auftrennung derselben und Wiederfreisetzung der Verbindung V, in die individuellen Enantiomeren. Zur Bildung diastereomerer Zwischenverbindungen sind insbesondere optisch aktive, mit Verbindungen V Salze, Ester oder Amide bildenden Säuren, wie D- oder L-Weinsäure, Di-o-Toluylweinsäure, Aepfelsäure, Mandelsäure, Kamphersulfonsäure oder Chinasäure, geeignet.

Als fakultative Umwandlungsreaktionen verfahrensgemäss erhältlicher in andere Verbindungen der Formel V und gegenseitige Umwandlungen von verfahrensgemäss erhältlicher Basen und Salzen ineinander kommen insbesondere die für Verbindungen der Formel I und ihre Salze angegebenen Reaktionsweisen in Betracht.

Bei den erfindungsgemässen pharmazeutischen Präparaten, welche Verbindungen der Formel I und V bzw. pharmazeutisch verwendbare Salze davon enthalten, handelt es sich um solche zur enteralen wie oralen oder rektalen, und parenteralen Verabreichung an Warmblüter, welche den pharmakologischen Wirkstoff allein oder zusammen mit einem pharmazeutisch anwendbaren Trägermaterial enthalten. Die Dosierung des Wirkstoffs hängt von der Warmblüter-Spezies, dem Alter und dem individuellen Zustand, sowie von der Applikationsweise ab.

Im Normalfall ist für einen etwa 75 kg schweren Warmblüter bei oraler Applikation eine ungefähre Tagesdosis von etwa 10-100 mg, vorteilhaft in mehreren gleichen Teildosen verteilt, zu veranschlagen.

Die neuen pharmazeutischen Präparate enthalten z.B. von etwa 10% bis etwa 80%, vorzugsweise von etwa 20% bis etwa 60% des Wirkstoffs, erfindungsgemäss pharmazeutische Präparate zur enteralen bzw. parenteralen Verabreichung sind z.B. solche in Dosiseinheitsformen wie Dragées, Tabletten, Kapseln oder Suppositorien, ferner Ampullen. Diesen werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Traganth, Methylcellulose und/oder Polyvinylpyrrolidon, und/oder, wenn erwünscht, Sprengmittel, wie die obengenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne werden mit geeigneten, gegebenenfalls Magensaftresistenten Ueberzügen versehen, wobei man u.a. konzentrierte Zuckerlösungen, welche gegebenenfalls arabischen Grummi, Talk, Polyvinylpyrrolidon, Polyäthylenglykol und/oder Titandioxid enthalten, Lacklösungen in geeigneten organischen Lösungsmitteln oder Lösungsmittelgemischen oder, zur Herstellung von magensaftresistenten Ueberzügen, Lösungen von geeigneten Cellulosepräparaten, wie Acetylcellulosephthalat oder Hydroxypropylmethylcellulosephthalat verwendet. Den

Tabletten oder Dragée-Ueberzügen können Farbstoffe oder Pigmente, z.B. zur Identifizierung oder zur Kennzeichnung verschiedener Wirkstoffdosen beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyäthylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyäthylenglykole oder höhere Alkanole. Ferner können auch Gelatine-Rektalkapseln verwendet werden, die eine Kambination des Wirkstoffs mit einer Grundmasse enthalten; als Grundmassenstoffe kommen z.B. flüssige Triglyceride, Polyäthylenglykole oder Paraffinkohlenwasserstoffe in Frage.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Verhikel, wie fette Oele, z.B. Sesamöl, oder synthetische Fettsäureester, z.B. Aethyloleat oder Triglyceride, verwendet, oder wässrige Injektionssuspensionen, welche viskositätserhöhende Stoffe, z.B. Natrium-carboxymethylcellulose, Sorbit und/oder Dextran und gegebenenfalls auch Stabilisatoren enthalten.

Die vorliegende Erfindung betrifft ebenfalls die Verwendung der Verbindungen der Formeln I und V sowie der Salze solcher Verbindungen mit salzbildenden Eigenschaften, vorzugsweise zur Behandlung von Depressionen.

Die nachfolgenden Beispiele illustrieren die oben beschriebene Erfindung; sie sollen jedoch diese in ihrem Umfang in keiner Weise einschränken. Temperaturen sind in Celsiusgraden, Drucke in mbar angegeben. Die den Verbindungen der Formel I zugrundeliegenden Ringsysteme werden dabei folgendermassen bezeichnet und beziffert:

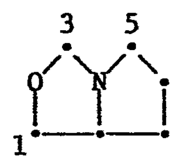

Pyrrolidino[1,5-c]oxazolidin

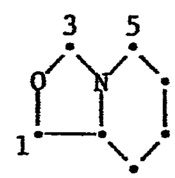

Piperidino[1,6-c]oxazolidin

Beispiel 1: erythro- und threo-1-(3-Chlorphenyl)-5,5-dimethyl-pyrrolidino[1,5-c]oxazolidin-3-on

27,6 g (0,115 Mol) erythro-(3-Chlorphenyl)-(5,5-dimethylpyrrolidin-2-yl)-methanol werden in 630 ml einer 20%igen Lösung von Phosgen in Toluol gelöst und unter Kühlung mit einem Eis-Wasser-Bad bei 20-25° Innentemperatur innerhalb von 40 Minuten tropfenweise mit einer Lösung von 58 g (80 ml, 0,57 Mol) Triäthylamin in 80 ml Toluol versetzt. Das gelbliche Reaktionsgemisch, in welchem sich allmählich eine Suspension bildet, wird dann 3 Tage bei Raumtemperatur gerührt. Unter Kühlung mit einem Eis-Wasser-Bad werden dann 750 ml Wasser zugetropft, die organische Phase wird abgetrennt, zweimal mit je 150 ml Wasser gewaschen, über Natriumsulfat getrocknet und an der Wasserstrahlpumpe zur Trockne eingedampft. Der Rückstand (bräunliches Oel) wird in 50 ml Toluol gelöst und über eine Schicht Silicagel filtriert. Man eluiert mit Toluol-Essigsäureäthylester-Gemisch (10:1) und dampft das Lösungsmittel an der Wasserstrahlpumpe ab. Man erhält

reines erythro-1-(3-Chlorphenyl)-5,5-dimethyl-pyrrolidino[1,5-c]-oxazolidin-3-on als blassgelbes Oel, das durch Digerieren mit Hexan bei 0° kristallisiert werden kann. Nach Umkristallisieren aus Hexan erhält man ein kristallines Produkt vom Smp. 84-86°.

26,4 g (0,11 Mol) threo-(3-Chlorphenyl)-(5,5-dimethylppyrolidin-2-yl)-methanol werden in 600 ml einer 20%igen Lösung von Phosgen in Toluol gelöst und unter Kühlung mit einem Eiswasser-Bad bei20-25° Innentemperatur innerhalb von 40 Minuten tropfenweise mit einer Lösung von 55 g (0,55 Mol) Triäthylamin in 75 ml Toluol versetzt. Das Reaktionsgemisch, in welchem sich allmählich eine gelbliche Suspension bildet, wird anschliessend 3 Tage bei Raumtemperatur gerührt. Unter Kühlung mit einem Eiswasser-Bad werden dann 700 ml Wasser zugetropft, die organische Phase wird abgetrennt, 2 mal mit je 150 ml Wasser gewaschen, über Natriumsulfat getrocknet und an der Wasserstrahlpumpe zur Trockne eingedampft. Das zurückbleibende, leicht rötliche Oel wird in 50 ml Aether gelöst und durch Zugabe von 50 ml Hexan zuerst bei Zimmertemperatur, dann bei 0° kristallisiert. Nach Filtration und Trocknen des Nutschgutes bei 50° im Hochvakuum erhält man weisse Kristalle von threo-1-(3-Chlorphenyl)-5,5-dimethyl-pyrrolidino[1,5-c]oxazolidin-3-on vom Smp. 83-84°.

Das jeweils als Ausgangsmaterial dienende erythro-(3-Chlorphenyl)-(5,5-dimethylpyrrolidin-2-yl)-methanol bzw. threo-(3-Chlorphenyl)-(5,5-dimethylpyrrolidin-2-yl)-methanol kann wie folgt hergestellt werden.

a) 35,4 ml (0,25 Mol) Diisopropylamin werden in 350 ml Tetrahydrofuran gelöst und unter Stickstoff mit einem Trockeneis-Aceton-Bad auf -78° gekühlt. Bei dieser Temperatur werden 180 ml (0,29 Mol) einer etwa 1,6 molaren Lösung von Butyllithium in Hexan zugetropft. Durch Entfernung des Kühlbades lässt man die Innentemperatur allmählich auf -10° steigen. Die so erhaltene Lösung von Lithiumdiisopropylamin wird wieder auf -78° gekühlt, innerhalb von 20 Minuten

tropfenweise mit 32 g (0,25 Mol) 1-Nitroso-2,2-dimethyl-pyrrolidin versetzt und dann weitere 10 Minuten gerührt. Anschliessend tropft man innerhalb von 15 Minuten 35,2 g (0,25 Mol) 3-Chlorbenzaldehyd zu und lässt weitere 3 Stunden bei -75° rühren. Die orangefarbene Lösung wird dann innerhalb von 20 Minuten tropfenweise mit einer Lösung von 40 ml (0,7 Mol) Eisessig in 80 ml Tetrahydrofuran versetzt. Die nunmehr hellgelbe Reaktionsmischung wird durch Entfernen des Kühlbades auf Raumtemperatur erwärmt und anschliessend auf ein Gemisch von je 1 Liter gesättigter Natriumchlorid-Lösung und Methylenchlorid gegossen. Nach dem Ausschütteln wird die wässrige untere Phase abgetrennt und 2 mal mit je 200 ml Methylenchlorid gewaschen. Die vereinigten organischen Phasen werden mit 200 ml Wasser gewaschen, über Natriumsulfat getrocknet und an der Wasserstrahlpumpe zur Trockne eingedampft. Der alsbald durchkristallisierende Rückstand wird heiss in einem Gemisch von 180 ml Toluol und 270 ml Hexan gelöst und zur Kristallisation gestellt. Man nutscht ab, trocknet bei 60° im Hochvakuum und erhält reines erythro-(3-Chlorphenyl)-(5,5-dimethyl-1-nitroso-pyrrolidin-2-yl)-methanol in Form weisser Kristalle vom Smp. 143-144°. Das zuerst im Wasserstrahlvakuum und anschliessend im Hochvakuum eingedampfte Filtrat liefert einen bräunlichen öligen Rückstand, in dem das threo-(3-Chlorphenyl)-(5,5-dimethyl-1-nitroso-pyrrolidin-2-yl)-methanol angereichert ist. Eine analysenreine Probe des als Oel anfallenden reinen threo-Diastereomere kann durch Filtration über eine Schicht Silicagel mit Toluol-Essigester 20:1 Gemisch als Elutionsmittel erhalten werden.

b) 32,1 g (0,12 Mol) kristallines erythro-(3-Chlorphenyl)-(5,5-dimethyl-1-nitroso-pyrrolidin-2-yl)-methanol werden in einem Gemisch von 180 ml Toluol und 120 ml Dioxan gelöst. Dann wird 1 Stunde bei Raumtemperatur trockenes Chlorwasserstoff-Gas eingeleitet. Das Reaktionsgemisch wird anschliessend 12 Stunden bei Raumtemperatur nachgerührt, wobei sich die Lösung allmählich tiefgelb färbt. Das gebildete Nitrosylchlorid wird dann durch 2-stündiges Hindurchleiten von Stickstoff aus der sich wieder hellgelb färbenden Lösung ausgetrieben und das Reaktionsgemisch

mit einem Eis-Wasserbad auf 0° gekühlt. Das auskristallisierte Produkt wird abfiltriert, mit Aether gewaschen und im Hochvakuum bei 50° getrocknet. Man erhält kristallines erythro-(3-Chlorphenyl)-(5,5-dimethylpyrrolidin-2-yl)-methanol-hydrochlorid vom Smp. 168-169°. Zur Freisetzung der Base werden 27,6 g (0,1 Mol) Hydrochlorid in 280 ml Wasser gelöst und unter Kühlung in einem Eiswasser-Bad tropfenweise mit 26 ml halbkonzentrierter Natronlauge versetzt. Die dicke weisse Suspension wird noch 30 Minuten gerührt, filtriert, der Filterrückstand mit Wasser gewaschen und anschliessend im Hochvakuum bei 60° getrocknet. Man erhält das erythro-(3-Chlorphenyl)-(5,5-dimethylpyrrolidin-2-yl)-methanol in Form weisser Kristalle, Smp. 120-121°.

c) Das wie unter a) beschrieben gewonnene Oel (28,1 g), in dem das threo-(3-Chlorphenyl)-(5,5-dimethyl-1-nitroso-pyrrolidin-2-yl)-methanol angereichert ist, wird in 120 ml Toluol gelöst und 1 Stunde lang bei Raumtemperatur von einem Strom von trockenem Chlorwasserstoff-Gas durchperlt. Das Reaktionsgemisch wird 12 Stunden bei Raumtemperatur nachgerührt und das gebildete Nitrosylchlorid durch 2-stündiges Hindurchleiten von Stickstoff ausgetrieben. Die gelb-braune Suspension wird anschliessend 2 mal mit je 100 ml Wasser ausgeschüttelt. Die vereinigten wässrigen Phasen werden 2 mal mit je 50 ml Methylenchlorid gewaschen und mit konzentrierter Natronlauge stark alkalisch gestellt. Das ausgefallene Produkt wird abfiltriert, mit Wasser gewaschen und im Hochvakuum bei 60° getrocknet. Man erhält rohes threo-(3-Chlorphenyl)-(5,5-dimethylpyrrolidin-2-yl)-methanol. Eine analysenreine Probe der Base erhält man durch Umkristallisation aus iso-Propanol; Smp. 98-99°. Das Rohprodukt wird in 70 ml Methanol gelöst und unter Kühlung mit einem Eis-Wasserbad mit 20 ml einer etwa 4n Lösung von Chlorwasserstoff in Diäthyläther versetzt. Nach Zugabe von Aether kristallisiert das weisse threo-(3-Chlorphenyl)-(5,5-dimethylpyrrolidin-2-yl)-methanol-hydrochlorid, welches abfiltriert, mit Aether gewaschen und im Hochvakuum bei 60° getrocknet wird; Smp. 209-210°.

Beispiel 2: erythro- und threo-1-(3,4-Dichlorphenyl)-5,5-dimethyl-pyrrolidino[1,5-c]oxazolidin-3-on

In analoger Weise wie in Beispiel 1 beschrieben erhält man ausgehend von 25 g (0,91 Mol) erythro-(3,4-Dichlorphenyl)-(5,5-dimethylpyrrolidin-2-yl)-methanol, 65 ml Triäthylamin und 500 ml 20%iger Phosgen-Lösung in Toluol das erythro-1-(3,4-Dichlorphenyl)-5,5-dimethylpyrrolidino[1,5-c]oxazolidin-3-on in Form weisser Kristalle vom Smp. 125-126° (aus Hexan) sowie ausgehend von 6,3 g (0,023 Mol) threo-(3,4-Dichlorphenyl)-(5,5-dimethylpyrrolidin-2-yl)-methanol, 16,3 ml Triäthylamin und 120 ml 20%iger Phosgen-Lösung in Toluol, threo-1-(3,4-Dichlorphenyl)-5,5-dimethyl-pyrrolidino[1,5-c]oxazolidin-3-on in Form weisser Kristalle vom Smp. 108-109° (aus Diäthyläther/Hexan).

Das jeweils als Ausgangsmaterial dienende erythro- bzw. threo-1-(3,4-Dichlorphenyl)-(5,5-dimethylpyrrolidin-2-yl)-methanol kann z.B. folgendermassen hergestellt werden:

a) Analog zu der im Beispiel 1 a) beschriebenen Verfahrensweise erhält man ausgehend von 43,7 g (0,25 Mol) 3,4-Dichlorbenzaldehyd und 32,0 g (0,25 Mol) 2,2-Dimethyl-1-nitroso-pyrrolidin das kristalline erythro-(3,4-Dichlorphenyl)-(5,5-dimethyl-1-nitroso-pyrrolidin-2-yl)-methanol vom Smp. 172-174° sowie ein orange-braunes Oel, in dem das diastereomere threo-1-(3,4-Dichlorphenyl)-(5,5-dimethyl-1-nitroso-pyrrolidin-2-yl)-methanol angereichert ist.

b) Analog zu der im Beispiel 1 b) beschriebenen Verfahrensweise erhält man ausgehend von 35,7 g (0,11 Mol) erythro-(3,4-Dichlorphenyl)-(5,5-dimethyl-1-nitroso-pyrrolidin-2-yl)-methanol durch Behandlung mit Chlorwasserstoff-Gas in einem Toluol-Dioxan-Gemisch kristallines erythro-(3,4-Dichlorphenyl)-(5,5-dimethylpyrrolidin-2-yl)-methanol-hydrochlorid vom Smp. 275-276°. Die aus dem Salz freigesetzte kristalline Base schmilzt bei 118-119°.

c) Analog zu der im Beispiel 1 c) beschriebenen Verfahrensweise erhält man ausgehend von dem gemäss 2 a) erhaltenen öligen Rückstand durch

Behandlung mit Chlorwasserstoff-Gas in Toluol und anschliessende Kristallisation aus Methanol/Diäthyläther reines threo-(3,4-Dichlorphenyl)-(5,5-dimethylpyrrolidin-2-yl)-methanol-hydrochlorid vom Smp. 247-249°. Die aus dem Salz freigesetzte kristalline Base schmilzt bei 76-78°.

Beispiel 3: erythro- und threo-1-(4-Chlorphenyl)-5,5-dimethyl-pyrrolidino[1,5-c]oxazolidin-3-on

In analoger Weise wie in Beispiel 1 beschrieben erhält man ausgehend von 3,3 g (0,0137 Mol) erythro-(4-Chlorphenyl)-(5,5-dimethylpyrrolidin-2-yl)-methanol, 6,9 g Triäthylamin und 75 ml einer 20%igen Phosgen-Lösung in Toluol das erythro-1-(4-Chlorphenyl)-5,5-dimethyl-pyrrolidino[1,5-c]oxazolidin-3-on in Form blass-gelblicher Kristalle vom Smp. 118-119° (aus Hexan) sowie ausgehend von 2,5 g (0,01 Mol) threo-(4-Chlorphenyl)-(5,5-dimethylpyrrolidin-2-yl)-methanol, 5,3 g Triäthylamin und 60 ml einer 20%igen Phosgen-Lösung in Toluol das threo-1-(4-Chlorphenyl)-5,5-dimethyl-pyrrolidino[1,5-c]oxazolidin-3-on in Form blass gelblicher Kristalle vom Smp.101-103° (aus Diäthyläther/Hexan).

Das jeweils als Ausgangsmaterial dienende erythro- bzw. threo-(4-Chlorphenyl)-(5,5-dimethylpyrrolidin-2-yl)-methanol kann z.B. folgendermassen hergestellt werden:

a) Analog zu der in Beispiel 1a)beschriebenen Verfahrensweise erhält man ausgehend von 35,2 g (0,25 Mol) 4-Chlorbenzaldehyd und 32,0 g (0,25 Mol) 2,2-Dimethyl-1-nitroso-pyrrolidin kristallines erythro-(4-Chlorphenyl)-(5,5-dimethyl-1-nitroso-pyrrolidin-2-yl)-methanol vom Smp. 161-162°C sowie einen bräunlichen Kristallbrei, in dem das diastereomere threo-(4-Chlorphenyl)-(5,5-dimethyl-1-nitroso-pyrrolidin-2-yl)-methanol angereichert ist. Eine analysenreine Probe des isomerenfreien threo-Diastereomeren kann durch Filtration über eine Schicht Silicagel mit Toluol/Essigester (20:1) als Elutionsmittel erhalten werden. Nach dem Abdampfen der Lösungsmittel fällt das Produkt in Form weisser Kristalle vom Smp. 113-115° an.

b) Analog zu der in Beispiel 1 b) beschriebenen Verfahrensweise erhält man ausgehend von 26,8 g (0,1 Mol) erythro-(4-Chlorphenyl)-(5,5-dimethyl-1-nitroso-pyrrolidin-2-yl)-methanol durch Behandlung mit Chlorwasserstoff-Gas in einem Toluol/Dioxan-Gemisch kristallines erythro-(4-Chlorphenyl)-(5,5-dimethylpyrrolidin-2-yl)-methanol vom Smp. 227-228°. Die aus dem Salz freigesetzte kristalline Base schmilzt bei 124-125°.

c) Analog zu der in Beispiel 1 c) beschriebenen Verfahrensweise erhält man ausgehend von 30 g des unter 3 a) erhaltenen Kristallbreis, in dem das threo-(4-Chlorphenyl)-(5,5-dimethyl-1-nitroso-pyrrolidin-2-yl)-methanol bereits angereichert ist, durch Behandlung mit Chlorwasserstoff-Gas in Toluol und anschliessende Kristallisation aus Methanol/Diäthyläther das threo-(4-Chlorphenyl)-(5,5-dimethylpyrrolidin-2-yl)-methanol-hydrochlorid vom Smp. 200 - 201°. Die aus dem Salz freigesetzte kristalline Base schmilzt bei 79-80°.

Beispiel 4: erythro- und threo-1-(3-Methylphenyl)-5,5-dimethyl-pyrrolidino[1,5-c]oxazolidin-3-on

In analoger Weise wie in Beispiel 1 beschrieben erhält man ausgehend von 4,4 g (0,02 Mol) erythro-(3-Methylphenyl)-(5,5-dimethylpyrrolidin-2-yl)-methanol, 10,1 g Triäthylamin und 110 ml einer 20%igen Phosgen-Lösung in Toluol das erythro-1-(3-Methylphenyl)-5,5-dimethyl-pyrrolidino[1,5-c]oxazolidin-3-on in Form eines amorphen weissen Pulvers vom Smp. 50-60° sowie ausgehend von 2,9 g (0,013 Mol) threo-(3-Methylphenyl)-(5,5-dimethylpyrrolidin-2-yl)-methanol, 6,7 g Triäthylamin und 75 ml einer 20%igen Phosgen-Lösung in Toluol das threo-1-(3-Methylphenyl)-5,5-dimethyl-pyrrolidino[1,5-c]oxazolidin-3-on in Form weisser Kristalle vom Smp. 65-67° (aus Hexan).

Das als Ausgangsmaterial dienende erythro- bzw. threo-(3-Methylphenyl)-(5,5-dimethylpyrrolidin-2-yl)-methanol kann z.B. folgendermassen hergestellt werden:

a) Analog zu der in Beispiel 1 a) beschriebenen Verfahrensweise erhält man ausgehend von 15,0 g (0,125 Mol) 3-Toluylaldehyd und 16 g (0,125 Mol) 2,2-Dimethyl-1-nitroso-pyrrolidin kristallines erythro-(3-Methylphenyl)-(5,5-dimethyl-1-nitroso-pyrrolidin-2-yl)-methanol-hydrochlorid vom Smp. 136-137° sowie ein bräunliches Oel, in dem das diastereomere threo-(3-Methylphenyl)-(5,5-dimethyl-1-nitroso-pyrrolidin-2-yl)-methanol angereichert ist.

b) Analog zu der in Beispiel 1 b) beschriebenen Verfahrensweise erhält man ausgehend von 14,9 g (0,06 Mol) erythro-(3-Methylphenyl)-(5,5-dimethyl-1-nitroso-pyrrolidin-2-yl)-methanol durch Behandlung mit Chlorwasserstoff-Gas in einem Toluol/Dioxan-Gemisch kristallines erythro-(3-Methylphenyl)-(5,5-dimethylpyrrolidin-2-yl)-methanol-hydrochlorid vom Smp. 200-202°.

c) Analog zu der in Beispiel 1 c) beschriebenen Verfahrensweise erhält man ausgehend von 14 g des unter 4 a) erhaltenes Oels, in dem das threo-(3-Methylphenyl)-(5,5-dimethyl-1-nitroso-pyrrolidin-2-yl)-methanol angereichert ist, durch Behandlung mit Chlorwasserstoff-Gas in Toluol und anschliessender Kristallisation aus Isopropanol/Diäthyläther reines threo-(3-Methylphenyl)-(5,5-dimethylpyrrolidin-2-yl)-methanol-hydrochlorid vom Smp. 230-232°.

Beispiel 5: erythro- und threo-1-Phenyl-5,5-dimethyl-pyrrolidino-[1,5-c]oxazolidin-3-on

In analoger Weise wie in Beispiel 1 beschrieben erhält man ausgehend von 5 g (0,024 Mol) erythro-Phenyl-(5,5-dimethylpyrrolidin-2-yl)-methanol, 17 ml Triäthylamin und 133 ml einer 20%igen Phosgen-Lösung in Toluol das erythro-1-Phenyl-5,5-dimethyl-pyrrolidino[1,5-c]oxazolidin-3-on in Form weisser Kristalle vom Smp. 135-137° (aus Hexan) sowie ausgehend von 1,3 g (0,006 Mol) threo-Phenyl-(5,5-dimethylpyrrolidin-2-yl)-methanol, 4,4 ml Triäthylamin und 35 ml einer 20%igen Phosgen-Lösung in Toluol das threo-1-Phenyl-5,5-dimethyl-pyrrolidino-[1,5-c]-oxazolidin-3-on in Form weisser Kristalle vom Smp. 70-71° (aus Aether/Hexan).

Das als Ausgangsmaterial verwendete erythro- bzw. threo-Phenyl-(5,5-dimethylpyrrolidin-2-yl)-methanol kann z.B. folgendermassen hergestellt werden:

a) Analog zu der in Beispiel 1 a) beschriebenen Verfahrensweise erhält man ausgehend von 13,3 g (0,125 Mol) Benzaldehyd und 16,0 g (0,125 Mol) 2,2-Dimethyl-1-nitroso-pyrrolidin kristallines erythro-Phenyl-(5,5-dimethyl-1-nitroso-pyrrolidin-2-yl)-methanol vom Smp. 119-121° sowie ein bräunliches Oel, in dem das diastereomere threo-Phenyl-(5,5-dimethyl-1-nitroso-pyrrolidin-2-yl)-methanol angereichert ist.

b) Analog zu der in Beispiel 1 b) beschriebenen Verfahrensweise erhält man ausgehend von 15,6 g (0,066 Mol) erythro-Phenyl-(5,5-dimethyl-1-nitroso-pyrrolidin-2-yl)-methanol durch Behandlung mit Chlorwasserstoff-Gas in einem Toluol/Dioxan-Gemisch kristallines erythro-Phenyl-(5,5-dimethylpyrrolidin-2-yl)-methanol-hydrochlorid vom Smp. 169 - 170°. Die aus dem Salz freigesetzte kristalline Base schmilzt bei 105-106°.

c) Analog zu der in Beispiel 1 c) beschriebenen Verfahrensweise erhält man ausgehend von 11 g des gemäss 5 a) erhaltenen Rückstandes durch Behandlung mit Chlorwasserstoff-Gas in Toluol und anschliessende Kristallisation aus Aethanol/Diäthyläther reines threo-Phenyl-(5,5-dimethylpyrrolidin-2-yl)-methanol-hydrochlorid vom Smp. 222-225°. Die aus dem Salz freigesetzte Base fällt als gelbliches Oel an.

Beispiel 6: erythro- und threo-1-(1-Naphthyl)-5,5-dimethyl-pyrrolidino[1,5-c]oxazolidin-3-on

In analoger Weise wie in Beispiel 1 beschrieben erhält man ausgehend von 4 g (0,013 Mol) erythro-(1-Naphthyl)-(5,5-dimethylpyrrolidin-2-yl)-methanol-hydrochlorid, 11,5 ml Triäthylamin und 75 ml einer 20%igen Phosgen-Lösung in Toluol nach zweimaliger Kristallisation aus Aether-Hexan das erythro-1-(1-Naphthyl)-5,5-dimethyl-pyrrolidino-[1,5-c]oxazolidin-3-on in Form blass-gelblicher Kristalle vom Smp. 138-139° sowie ausgehend von 1,0 g (0,003 Mol) threo-(1-Naphthyl)-

(5,5-dimethylpyrrolidin-2-yl)-methanol, 2,8 ml Triäthylamin und 18,5 ml einer 20%igen Phosgen-Lösung in Toluol das threo-1-(1-Naphthyl)-5,5-dimethyl-pyrrolidino[1,5-c]oxazolidin-3-on in Form hellbrauner Kristalle vom Smp. 132-134° (aus Diäthyläther/Hexan).

Das als Ausgangsmaterial dienende erythro- bzw- threo-(1-Naphthyl)-(5,5-dimethylpyrrolidin-2-yl)-methanol kann z.B. wie folgt hergestellt werden:

a) Analog zu der in Beispiel 1 a) beschriebenen Verfahrensweise erhält man ausgehend von 19,5 g (0,125 Mol) 1-Naphthaldehyd und 16,0 g (0,125 Mol) 2,2-Dimethyl-1-nitroso-pyrrolidin kristallines erythro-(1-Naphthyl)-(5,5-dimethyl-1-nitroso-pyrrolidin-2-yl)-methanol vom Smp. 159-160°, sowie einen bräunlichen Kristallbrei, in dem das diastereomere threo-(1-Naphthyl)-(5,5-dimethyl-1-nitroso-pyrrolidin-2-yl)-methanol angereichert ist.

b) Analog zu der in Beispiel 1 b) beschriebenen Verfahrensweise erhält man ausgehend von 9 g (0,031 Mol) erythro-(1-Naphthyl)-(5,5-dimethyl-1-nitroso-pyrrolidin-2-yl)-methanol durch Behandlung mit Chlorwasserstoff-Gas in Toluol und Umkristallisieren des Rohproduktes aus einem Methanol/Diäthyläther-Gemisch erythro-(1-Naphthyl)-(5,5-dimethyl-pyrrolidin-2-yl)-methanol-hydrochlorid in Form weisser Kristalle vom Smp. 229-230°. Die aus dem Salz freigesetzte Base schmilzt bei 152-154°.

c) Analog zu der in Beispiel 1 c) beschriebenen Verfahrensweise erhält man ausgehend von 9,2 g des gemäss 6 a) erhaltenen Kristallbreies, in dem das threo-(1-Naphthyl)-(5,5-dimethyl-1-nitroso-pyrrolidin-2-yl)-methanol angereichert ist, durch Behandlung mit Chlorwasserstoff-Gas in Toluol und anschliessender 2 maliger Kristallisation aus Methanol/Diäthyläther reines threo-(1-Naphthyl)-(5,5-dimethyl-pyrrolidin-2-yl)-methanol-hydrochlorid vom Smp. 250° (Zers.).

Beispiel 7: erythro- und threo-1-(3-Trifluormethylphenyl)-5,5-dimethyl-pyrrolidino[1,5-c]oxazolidin-3-on

In analoger Weise wie in Beispiel 1 beschrieben erhält man ausgehend von 3,0 g (0,009 Mol) erythro-(3-Trifluormethylphenyl)-(5,5-dimethyl-pyrrolidin-2-yl)-methanol-hydrochlorid, 6,7 g Triäthylamin und 52 ml einer 20%-igen Phosgenlösung in Toluol das erythro-1-(3-Trifluormethylphenyl)-5,5-dimethyl-pyrrolidino[1,5-c]oxazolidin-3-on in Form weisser Kristalle vom Smp. 71-72° (aus Diäthyläther/Hexan) sowie ausgehend von 1,9 g (0,006 Mol) threo-(3-Trifluormethylphenyl)-(5,5-dimethyl-pyrrolidin-2-yl)-methanol, 5,2 ml Triäthylamin und 34 ml einer 20%-igen Phosgen-Lösung in Toluol das threo-1-(3-Trifluormethylphenyl)-5,5-dimethyl-pyrrolidino[1,5-c]oxazolidin-3-on in Form gelblicher Kristalle vom Smp. 70-71° (aus Diäthyläther/Hexan).

Das als Ausgangsmaterial dienende erythro- bzw. threo-(3-Trifluormethylphenyl)-(5,5-dimethylpyrrolidin-2-yl)-methanol kann z.B. wie folgt hergestellt werden:

a) Analog zu der in Beispiel 1 a) beschriebenen Verfahrensweise erhält man ausgehend von 10,4 g (0,06 Mol) 3-Trifluormethylbenzaldehyd und 7,7 g (0,06 Mol) 2,2-Dimethyl-1-nitroso-pyrrolidin nach Kristallisation aus Toluol erythro-(3-Trifluormethylphenyl)-(5,5-dimethyl-1-nitroso-pyrrolidin-2-yl)-methanol vom Smp. 172-174°, sowie ein bräunliches Oel, in dem das diastereomere threo-(3-Trifluormethylphenyl)-(5,5-dimethyl-1-nitroso-pyrrolidin-2-yl)-methanol angereichert ist.

b) Analog zu der in Beispiel 1 b) beschriebenen Verfahrensweise erhält man ausgehend von 9,06 g (0,03 Mol) erythro-(3-Trifluormethylphenyl)-(5,5-dimethyl-1-nitroso-pyrrolidin-2-yl)-methanol durch Behandlung mit Chlorwasserstoff-Gas in einem Toluol/Dioxan-Gemisch und Umkristallisation des Rohproduktes aus Methanol/Diäthyläther erythro-(3-Trifluormethylphenyl)-(5,5-dimethylpyrrolidin-2-yl)-methanol-hydrochlorid in Form weisser Kristalle vom Smp. 205-206°. Die aus dem Salz freigesetzte kristalline Base schmilzt bei 104-105°C.

c) Analog zu der in Beispiel 1 c) beschriebenen Verfahrensweise erhält man ausgehend von 7,9 g des gemäss 7 a) erhaltenen öligen Rückstandes, in dem das threo-(3-Trifluormethylphenyl)-(5,5-dimethyl-1-nitroso-pyrrolidin-2-yl)-methanol angereichert ist, durch Behandlung mit Chlorwasserstoff-Gas in Toluol und anschliessende Kristallisation aus Methanol/Diäthyläther reines threo-(3-Trifluormethylphenyl)-(5,5-dimethylpyrrolidin-2-yl)-methanol-hydrochlorid vom Smp. 224-227°. Die aus dem Salz freigesetzte kristalline Base schmilzt bei 65-67°.

Beispiel 8: erythro- und threo-1-(3-Chlorphenyl)-5,5-dimethyl-piperidino[1,6-c]oxazolidin-3-on

In analoger Weise wie in Beispiel 1 beschrieben erhält man ausgehend von 5,8 g (0,023 Mol) erythro-(3-Chlorphenyl)-(6,6-dimethylpiperidin-2-yl)-methanol, 11,6 g Triäthylamin und 125 ml einer 20%igen Phosgen-Lösung in Toluol das erythro-1-(3-Chlorphenyl)-5,5-dimethyl-piperidino-[1,6-c]oxazolidin-3-on in Form weisser Kristalle vom Smp. 69-70° (aus Aether/Hexan) sowie ausgehend von 1,0 g (0,004 Mol) threo-(3-Chlorphenyl)-(6,6-dimethylpiperidin-2-yl)-methanol, 2,9 ml Triäthylamin und 25 ml einer 20%igen Phosgen-Lösung in Toluol das threo-1-(3-Chlorphenyl)-5,5-dimethyl-piperidino[1,6-c]oxazolidin-3-on in Form weisser Kristalle vom Smp. 69-71° (aus Hexan).

Das als Ausgangsmaterial dienende erythro- bzw. threo-(3-Chlorphenyl)-(6,6-dimethylpiperidin-2-yl)-methanol kann z.B. wie folgt hergestellt werden:

a) Analog zu der in Beispiel 1 a) beschriebenen Verfahrensweise erhält man ausgehend von 14,1 g (0,1 Mol) 3-Chlorbenzaldehyd und 14,2 g (0,1 Mol) 2,2-Dimethyl-1-nitroso-piperidin - siehe unter d) - kristallines erythro-(3-Chlorphenyl)-(6,6-dimethyl-1-nitroso-piperidin-2-yl)-methanol vom Smp. 125 - 127° sowie ein orange-braunes Oel, in dem das diastereomere threo-(3-Chlorphenyl)-(6,6-dimethyl-1-nitroso-piperidin-2-yl)-methanol angereichert ist. Eine analysenreine Probe

des isomerenfreien threo-Diastereomeren kann durch Filtration über eine Schicht Silicagel mit Toluol/Essigsäureäthylester (20:1) als Elutionsmittel erhalten werden. Nach dem Abdampfen der Lösungsmittel fällt das Produkt in Form blass-gelber Kristalle vom Smp. 108-110° an.

b) Analog zu der in Beispiel 1 b) beschriebenen Verfahrensweise erhält man ausgehend von 14,0 g (0,05 Mol) erythro-(3-Chlorphenyl)-(6,6-dimethyl-1-nitroso-piperidin-2-yl)-methanol durch Behandlung mit Chlorwasserstoff-Gas in einem Toluol/Dioxan-Gemisch kristallines erythro-(3-Chlorphenyl)-(6,6-dimethylpiperidin-2-yl)-methanol-hydrochlorid vom Smp. 225-227°. Die aus dem Salz freigesetzte kristalline Base schmilzt bei 138-139°.

c) In Analogie zur vorstehend unter b) beschriebenen Verfahrensweise erhält man ausgehend von 3,6 g (0,012 Mol) des reinen threo-(3-Chlorphenyl)-(6,6-dimethyl-1-nitroso-piperidin-2-yl)-methanols durch Behandlung mit Chlorwasserstoff-Gas in einem Toluol/Dioxan-Gemisch kristallines threo-(3-Chlorphenyl)-(6,6-dimethylpiperidin-2-yl)-methanol-hydrochlorid vom Smp. 240-241°. Die aus dem Salz freigesetzte kristalline Base schmilzt bei 155-156°.

d) 19,4 g (0,1 Mol) 2,2-Dimethylpiperidin-hydrobromid werden in einem Gemisch von 30 ml Wasser und 15 ml Eisessig gelöst und bei Raumtemperatur innerhalb 1 Stunde tropfenweise mit 41,4 ml (0,3 Mol) einer 50%igen wässrigen Natriumnitrit-Lösung versetzt. Das Reaktionsgemisch wird weitere 2 Stunden bei Raumtemperatur gerührt, dann mit einem Eiswasser-Bad auf 0° gekühlt und bei dieser Temperatur tropfenweise mit 25 ml konzentrierter Natronlauge versetzt. Anschliessend wird die gelbe Lösung erschöpfend mit je 100 ml Diäthyläther extrahiert. Die vereinigten organischen Phasen werden mit 100 ml Wasser gewaschen, über Natriumsulfat getrocknet und bei 30° Badtemperatur im Wasserstrahlvakuum eingedampft. Der gelbe,ölige Rückstand wird anschliessend im Wasserstrahlvakuum destilliert, wobei das 2,2-Dimethyl-1-nitrosopiperidin als gelbe Flüssigkeit vom $Kp_{13}$=101-102° übergeht.

Beispiel 9: erythro- und threo-1-(3,4-Dichlorphenyl)-5,5-dimethyl-piperidino[1,6-c]oxazolidin-3-on

In analoger Weise wie in Beispiel 1 beschrieben erhält man ausgehend von 9,0 g (0,031 Mol) erythro-(3,4-Dichlorphenyl)-(6,6-dimethylpiperidin-2-yl)-methanol, 15,8 g Triäthylamin und 170 ml einer 20%igen Phosgen-Lösung in Toluol das erythro-1-(3,4-Dichlorphenyl)-5,5-dimethyl-piperidino[1,6-c]oxazolidin-3-on in Form weisser Kristalle vom Smp. 127-129° (aus Aether-Hexan) sowie ausgehend von 6,7 g (0,023 Mol) threo-(3,4-Dichlorphenyl)-(6,6-dimethylpiperidin-2-yl)-methanol, 11,7 g Triäthylamin und 130 ml einer 20%igen Phosgen-Lösung in Toluol das threo-1-(3,4-Dichlorphenyl)-5,5-dimethyl-piperidino[1,6-c]oxazolidin-3-on in Form blass-gelblicher Kristalle vom Smp. 120-121° (aus Diäthyläther/Hexan).

Das als Ausgangsmaterial dienende erythro- bzw. threo-(3,4-Dichlorphenyl)-(6,6-dimethylpiperidin-2-yl)-methanol kann z.B. wie folgt hergestellt werden:

a) Analog zu der in Beispiel 1 a) beschriebenen Verfahrensweise erhält man ausgehend von 17,6 g (0,1 Mol) 3,4-Dichlorbenzaldehyd und 14,2 g (0,1 Mol) 2,2-Dimethyl-1-nitroso-piperidin kristallines erythro-(3,4-Dichlorphenyl)-(6,6-dimethyl-1-nitroso-piperidin-2-yl)-methanol vom Smp. 140-141° sowie einen gelb-braunen Kristallbrei, in dem das diastereomere threo-(3,4-Dichlorphenyl)-(6,6-dimethyl-1-nitroso-piperidin-2-yl)-methanol angereichert ist. Eine analysenreine Probe des isomerenfreien threo-Diastereomeren kann durch Filtration über eine Schicht Silicagel mit einem Toluol/Essigestergemisch (10:1) als Elutionsmittel erhalten werden. Nach dem Abdampfen der Lösungsmittel fällt das Produkt in Form weisser Kristalle vom Smp. 173-174° an.

b) Analog zu der in Beispiel 1 b) beschriebenen Verfahrensweise erhält man ausgehend von 5,5 g (0,017 Mol) erythro-(3,4-Dichlorphenyl)-(6,6-dimethyl-1-nitroso-piperidin-2-yl)-methanol durch Behandlung mit Chlorwasserstoff-Gas in einem Toluol/Dioxan-Gemisch das erythro-(3,4-Dichlorphenyl)-(6,6-dimethylpiperidin-2-yl)-methanol-hydrochlorid vom Smp. 288° (Zers.). Die aus dem Salz freigesetzte Base schmilzt bei 132-133°.

c) In Analogie zur vorstehend unter b) beschriebenen Umsetzung erhält man ausgehend von 3,0 g (0,009 Mol) threo-(3,4-Dichlorphenyl)-(6,6-dimethyl-1-nitroso-piperidin-2-yl)-methanol durch Behandlung mit Chlorwasserstoff-Gas in einem Toluol/Dioxan-Gemisch kristallines threo-(3,4-Dichlorphenyl)-(6,6-dimethyl-piperidin-2-yl)-methanol-hydrochlorid vom Smp. 257-258°. Die aus dem Salz freigesetzte Base schmilzt bei 153-154°.

Beispiel 10: erythro- und threo-1-(2,6-Dichlorphenyl)-5,5-dimethyl-pyrrolidino[1,5-c]oxazolidin-3-on

In analoger Weise wie in Beispiel 1 beschrieben erhält man ausgehend von 4 g (0,003 Mol) erythro-(2,6-Dichlorphenyl)-5,5-dimethyl-pyrrolidin-2-yl)-methanol-hydrochlorid, 9 ml Triäthylamin und 70 ml einer 20%-igen Phosgenlösung in Toluol das erythro-1-(2,6-Dichlorphenyl)-5,5-dimethyl--pyrrolidino[1,5-c]oxazolidin-3-on in Form weisser Kristalle vom Smp. 92-94° (aus Diäthyläther/Hexan sowie ausgehend von 2,0 g (0,006 Mol) threo-(2,6-Dichlorphenyl)-(5,5-dimethyl-pyrrolidin-2-yl)-methanol-hydrochlorid, 4,5 ml Triäthylamin und 35 ml einer 20%-igen Phosgen-Lösung in Toluol, 0,5 g (26,3% d. Th.), threo-1-(2,6-Dichlorphenyl)-5,5-dimethyl-pyrrolidino[1,5-c]oxazolidin-3-on in Form weisser Kristalle aus Aether/Hexan, vom Smp. 143-144° (aus Diäthyläther/Hexan).

Das als Ausgangsmaterial dienende erythro- bzw. threo-(2,6-Dichlorphenyl)-(5,5-dimethylpyrrolidin-2-yl)-methanol bzw. dessen Hydrochlorid kann z.B. folgendermassen hergestellt werden:

a) Analog zu der im Beispiel 1a) beschreibenen Verfahrensweise erhält man ausgehend von 21,9 g (0,125 Mol) 2,6-Dichlorbenzaldehyd und 16 g (0,125 Mol) 2,2-Dimethyl-1-nitroso-pyrrolidin kristallines erythro-(2,6-Dichlorphenyl)-(5,5-dimethyl-1-nitroso-pyrrolidin-2-yl)-methanol, Smp. 178-180°, sowie 18,5 g eines gelblichen Kristallbreis in dem das diastereomere threo-(2,6-Dichlorphenyl)-(5,5-dimethyl-1-nitroso-pyrrolidin-2-yl)-methanol angereichert ist.

b) Analog zu der in Beispiel 1 b) beschriebenen Verfahrensweise erhält man ausgehend von 19,0 g (0,063 Mol) erythro-(2,6-Dichlorphenyl)-(5,5-dimethyl-1-nitroso-pyrrolidin-2-yl)-methanol durch Behandlung mit Chlorwasserstoff-Gas in Toluol, kristallines eryhtro-(2,6-Dichlorphenyl)-(5,5-dimethyl-pyrrolidin-2-yl)-methanol-hydrochlorid, Smp. 243-244°. Die aus dem Salz freigesetzte, kristalline Base schmilzt bei 112-114°.

c) Analog zu der in Beispiel 1 c) beschriebenen Verfahrensweise erhält man ausgehend von 18,5 g des gemäss vorstehend a) erhaltenen Kristallbreis, in dem das Diastereomere angereichert ist, durch Behandlung mit Chlorwasserstoff-Gas in Toluol und anschliessende Kristallisation aus Aethanol/Diäthyläther reines threo-(2,6-Dichlorphenyl)-(5,5-dimethyl-pyrrolidin-2-yl)methanol-hydrochlorid, Smp. 224-225°. Die aus dem Salz freigesetzte, kristalline Base schmilzt bei 137-138°C.

Beispiel 11: erythro- und threo-(3-Chlorphenyl)-(5,5-dimethyl-pyrrolidin-2-yl)-methanol

3,5 g rohes (3-Chlorphenyl)-[1-(cyclohexyliminomethyl)-5,5-dimethyl--pyrrolidin-2-yl]-methanol werden in 50 ml Methanol gelöst, mit 50 ml 2n-Natronlauge versetzt und 4 Stunden zum Rückfluss erhitzt. Man dampft zur Trockne ein, nimmt in Aether auf, wäscht mit gesättigter Kochsalzlösung, trocknet über Natriumsulfat und dampft erneut zur Trockne ein. Man erhält ein Gemisch von erythro- und threo-(3-Chlorphenyl)-(5,5-dimethyl-pyrrolidin-2-yl)-methanol, das durch fraktionierte

Kristallisation aus Isopropanol und Chromatographie an Silicagel in die erythro-Form vom Smp. 120,5° und die threo-Form vom Smp. 96° aufgetrennt werden kann.

Das als Ausgangsmaterial verwendete (3-Chlorphenyl)-[1-(cyclohexyl-iminomethyl)-5,5-dimethyl-pyrrolidin-2-yl]-methanol kann folgendermasse hergestellt werden:

a) 9,9 g 2,2-Dimethylpyrrolidin werden in 25 ml Methanol gelöst, mit 7,4 g Ameisensäureäthylester versetzt und 3 Stunden zum Rückfluss erhitzt. Man engt auf die Hälfte ein, fügt 100 ml Toluol hinzu, trennt die Toluolphase ab, wäscht zweimal mit Wasser und dann mit gesättigter Kochsalzlösung und dampft unter vermindertem Druck zur Trockne ein. Man erhält rohes 1-Formyl-2,2-dimethyl-pyrrolidin, das ohne weitere Reinigung verwendet werden kann.

b) Das gemäss a) erhaltene Rohprodukt wird mit 9,4 g Dimethylsulfat verrührt und unter Stickstoff 3 Stunden auf 70° erhitzt. Man lässt abkühlen, nimmt in 100 ml Aether auf, wäscht zweimal mit n-Natriumhydrogencarbonat und dann mit gesättigter Kochsalzlösung und dampft zur Trockne ein. Man erhält rohes 1-Methoxymethylen-2,2-dimethyl-pyrrolidinium-methosulfat, das umgehend weiterverarbeitet wird.

c) Das gemäss b) erhaltene Produkt wird mit 20 g Cyclohexylamin verrührt und 15 Stunden bei Raumtemperatur gerührt. Man nimmt in Methylenchlorid auf, wäscht mit 10%-iger Kaliumhydroxid-Lösung, trocknet über Natriumsulfat und dampft zur Trockne ein. Man erhält 15 g 1-Cyclohexyl-iminomethyl-2,2-dimethyl-pyrrolidin, das sofort weiterverarbeitet wird.

d) Das gemäss c) erhaltene Produkt wird in 50 ml Tetrahydrofuran gelöst, unter Argon auf -78° gekühlt, mit 6,5 g Tertiärbutyllithium versetzt, eine Stunde lang auf -25° erwärmt und erneut auf -78° gekühlt. Dann werden unter heftigen Rühren bei -78° 10,5 g 3-Chlorbenzaldehyd

zugetropft. Man rührt 15 Minuten bei -70° nach und lässt dann langsam auf Raumtemperatur erwärmen, fügt 10 ml Methanol hinzu, neutralisiert mit verdünnter Essigsäure und dampft unter vermindertem Druck zur Trockne ein. Man erhält rohes (3-Chlorphenyl)-[1-(cyclohexylimino-methyl)-5,5-dimethyl-pyrrolidin-2-yl]-methanol, das ohne weitere Reinigung verwendet werden kann.

In analoger Weise kann man auch die Verbindungen der Formel V gemäss den Beispielen 2-10 herstellen.

Beispiel 12: erythro- und threo-(3-Chlorphenyl)-(5,5-dimethyl-pyrrolidin-2-yl)-methanol

5 g rohes (3-Chlorphenyl)-(1-benzyl-5,5-dimethyl)-methanol werden in 50 ml Methanol gelöst,mit 0,8 g Palladiumkohle (5%-ig) versetzt und unter einem Ueberdruck von 0,5 bar bei 50° bis zur Beendigung der Wasserstoffaufnahme hydriert (etwa 40 Minuten). Man filtriert vom Katalysator ab und dampft zur Trockne ein. Man erhält ein Gemisch erythro- und threo-(3-Chlorphenyl)-(5,5-dimethyl-pyrrolidin-2-yl)-methanol, das durch fraktionierte Kristallisation zunächst aus Aether/Hexan und dann aus Isopropanol in die erythro-Form von Smp. 120° und die threo-Form von Smp. 97° aufgetrennt werden kann.

Das als Ausgangsmaterial verwendete (3-Chlorphenyl)-(1-benzyl-5,5-dimethyl)-methanol kann z.B. folgendermasse hergestellt werden:

a) 2,5 g Natriumhydrid (50 ml einer 5%-igen Suspension in Mineralöl, mit Hexan entölt) werden unter Stickstoff in einem Gemisch von 100 ml Tetrahydrofuran und 100 ml Dimethylsulfoxid suspendiert und unter Rühren bei 5° tropfenweise mit einer Lösung von 46,7 g 3-Chlorbenzyltriphenylphosphoniumbromid und 13,0 g 4-Oxobuttersäureäthylester in 150 ml Tetrahydrofuran versetzt. Man lässt 30 Stunden bei Raumtemperatur nachrühren, dampft unter vermindertem Druck zur Trockne ein ,

nimmt in 1000 ml Aether auf, schüttelt mit 5 g Aktivkohle und 50 g wasserfreiem Natriumsulfat; filtriert und dampft zur Trockne ein. Man erhält rohen 5-(3-Chlorphenyl)-pent-4-en-carbonsäureäthylester in Form gelblicher Kristalle.

b) Das gemäss a) erhaltene Rohprodukt wird in 100 ml Tetrahydrofuran gelöst und zu einer Lösung von Methylmagnesiumbromid (bereitet aus 5 g Magnesiumspänen und 30 g Methylbromid) in 100 ml Tetrahydrofuran zugetropft. Man lässt 1 Stunde bei Raumtemperatur und 4 Stunden bei 60° nachrühren, dampft zur Trockne ein, nimmt in 500 ml Toluol auf, schüttelt mit Aktivkohle und wasserfreiem Natriumsulfat, filtriert und dampft unter vermindertem Druck zur Trockne ein. Man erhält rohes 6-(3-Chlorphenyl)-2-methyl-hex-5-enol, das umgehend weiterverarbeitet wird.

c) Das gemäss b) erhaltene Rohprodukt wird in 150 ml Toluol gelöst, auf 5° gekühlt, mit 100 ml 20%iger Salzsäure und 2,5 g Tetrabutylammoniumchlorid versetzt und 1 Stunde bei 5-10° sowie 2 Stunden bei Raumtemperatur heftig gerührt. Die organische Phase wird abgetrennt, mit 5 g Aktivkohle und 10 g wasserfreiem Magnesiumsulfat ausgeschüttelt und filtriert. Man erhält eine toluolische Lösung von 2-Chlor-6-(3-chlorphenyl)-2-methyl-hex-5-en.

d) Zu der gemäss c) erhaltenen toluolischen Produktlösung fügt man 15 g Benzylamin hinzu, lässt 1 Stunde bei Raumtemperatur rühren, erhitzt dann langsam zum Sieden, lässt 2 Stunden am Rückfluss sieden und dampft dann, zuletzt unter vermindertem Druck, zur Trockne ein. Man erhält ein viskoses Oel, das in 100 ml Aether aufgenommen wird. Man leitet Chlorwasserstoffgas ein, bis kein Niederschlag mehr erfolgt, saugt ab und trocknet an der Luft. Man erhält N-[6-(3-Chlorphenyl)-2-methyl-hex-5-enyl)-benzylammoniumchlorid, das ohne weitere Reinigung verwendet werden kann.

e) Das gemäss d) erhaltene Rohprodukt wird in 100 ml Methylenchlorid gelöst, mit 50 ml Trifluoressigsäureanhydrid und 10 g Natriumcarbonat versetzt und 2 Stunden bei Raumtemperatur gerührt. Man wäscht zweimal mit je 25 ml einer 10%-igen wässrigen Natriumhydrogencarbonatlösung, trocknet über Natriumsulfat und dampft zur Trockne ein. Man erhält 20,5 g rohes N-[6-(3-Chlorphenyl)-2-methyl-hex-5-enyl)-N-benzyl-trifluoracetamid.

f) Das gemäss e) erhaltene Rohprodukt wird in 75 ml Dioxan gelöst, bei 10° bis 15° und mit 10 ml Schwefelsäure (Dichte = 1,83) versetzt. Dann wird unter Rühren bei 10° eine Lösung von 8,2 g N-Bromsuccinimid in 50 ml Dioxan zugetropft. Man lässt 30 Minuten bei 10° bis 15° und 15 Stunden bei Raumtemperatur nachrühren, wäscht mit Wasser und gesättigter Kochsalzlösung und trocknet über Natriumsulfat. Man erhält eine Lösung von N-[5-Brom-6-(3-chlorphenyl)-6-hydroxy-2-methyl-hexyl]-N-benzyltrifluoracetamid in Dioxan.

g) Die gemäss f) erhaltene Produktlösung wird mit 80 ml n-Natronlauge versetzt und 1 Stunde unter Rühren zum Rückfluss erhitzt. Man engt stark ein, nimmt in Aether auf, wäscht mit Wasser und gesättigter Kochsalzlösung, trocknet über Natriumsulfat und dampft zur Trockne ein. Man erhält roher (3-Chlorphenyl)-(1-benzyl-5,5-dimethyl)-methanol, das ohne weitere Reinigung verwendet werden kann.

In analoger Weise kann man auch die Verbindungen der Formel V gemäss den Beispielen 2-10 herstellen.

Beispiel 13: threo-1-(4-Chlorphenyl)-5,5-dimethyl-pyrrolidino-[1,5-c]oxazolidin-3-on

6 g threo-1-(4-Chlorphenyl)-3-imino-5,5-dimethyl-pyrrolidino[1,5-c]-oxazolidin werden in 150 ml Wasser und 50 ml Phosphatpuffer ($P_H$ = 6,5) suspendiert und 22 Stunden unter Rühren zum Rückfluss erhitzt. Man lässt abkühlen, säuert mit verdünnter Salzsäure auf $P_H = 4$ an und schüttelt zweimal mit je 100 ml Essigester aus. Die Auszüge werden vereinigt, mit gesättigter Kochsalzlösung gewaschen, über Natriumsulfat getrocknet und unter vermindertem Druck zur Trockne eingedampft. Der kristalline Rückstand wird aus Essigester/Hexan umkristallisiert. Man erhält das threo-1-(4-Chlorphenyl)-5,5-dimethyl-pyrrolidino[1,5-c]oxazolidin-3-on, Smp. 100-102°.

Das Ausgangsmaterial kann z.B. nach einer der folgenden Methoden hergestellt werden:

a) 23,3 g threo-(4-Chlorphenyl)-(5,5-dimethyl-pyrrolidin-2-yl)-methanol werden in 100 ml Aethanol gelöst und unter Rühren tropfenweise mit einer Lösung von 6,5 g Chlorcyan in 25 ml Aethanol versetzt. Man erhitzt kurz zum Rückfluss, destilliert das Lösungsmittel ab und kristallisiert den Rückstand zweimal aus Isopropanol um. Man erhält threo-1-(4-Chlorphenyl)-3-imino-5,5-dimethyl-pyrrolidino[1,5-c]-oxazolidin, das ohne weitere Reinigung eingesetzt werden kann.

b) 23,3 g erythro-(4-Chlorphenyl)-(5,5-dimethyl-pyrrolidin-2-yl)-methanol werden in 150 ml n-Salzsäure gelöst, mit 8,1 g Kaliumcyanat versetzt und 12 Stunden bei Raumtemperatur gerührt. Man engt bis zur beginnenden Kristallisation ein, kühlt auf 5° ab, saugt ab und lässt an der Luft trocknen. Man erhält das erythro-(4-Chlorphenyl)-(1-carbamyl-5,5-dimethyl-pyrrolidin-2-yl)-methanol. Dieses wird in 200 ml Tetrahydrofuran gelöst und unter Rühren tropfenweise 12,0 g Thionylchlorid versetzt. Man erhitzt kurz zum Rückfluss, dampft unter vermindertem Druck zur Trockne ein und kristallisiert aus Aethanol um. Man erhält threo-1-(4-Chlorphenyl)-3-imino-5,5-dimethyl-pyrrolidino-[1,5-c]oxazolidin, welches nach Ausweis des IR-Spektrum mit dem gemäss a) erhaltenem Produkt identisch ist.

In analoger Weise kann man auch die Verbindungen der Formeln I und V gemäss den Beispielen 1, 2 und 4-10 herstellen.

<u>Beispiel 14:</u> <u>1-(4-Chlorphenyl)-5,5-dimethyl-pyrrolidino[1,5-c]oxazolidin-3-on, Diastereomerengemisch</u>

1,5 g erythro-(4-Chlorphenyl)-(1-carbamyl-5,5-dimethyl-pyrrolidin-2-yl)-methanol werden in 25 ml Pyridin gelöst, mit 1 g Thionylchlorid versetzt und 4 Stunden zum Sieden erhitzt. Man dampft unter vermindertem

Druck zur Trockne ein, löst in Aether, wäscht zweimal mit Wasser und anschliessend mit gesättigter Kochsalzlösung, trocknet über Natriumsulfat, dampft zur Trockne ein und kristallisiert mehrmals aus Hexan/Aether um. Man erhält ein Gemisch von erythro- und threo-1-(4-Chlorphenyl)-5,5-dimethyl-pyrrolidino[1,5-c]oxazolidin-3-on vom Smp. 82-87°.

Das Ausgangsmaterial kann z.B. folgendermassen hergestellt werden:

3,0 g erythro-(4-Chlorphenyl)-(5,5-dimethyl-pyrrolidin-2-yl)-methanol werden in 25 ml n-Salzsäuer suspendiert, mit 1,5 g Kaliumcyanat versetzt und 12 Stunden bei Raumtemperatur gerührt. Man engt bis zur beginnenden Kristallisation ein, kühlt auf 5°, saugt ab und kristallisiert aus Aethanol/Wasser um. Man erhält erythro-(4-Chlorphenyl)-(1-carbamyl-5,5-dimethyl-pyrrolidin-2-yl)-methanol, das ohne weitere Reinigung verwendet werden kann.

In analoger Weise kann man auch die Verbindungen der Formel I gemäss den Beispielen 1, 2 und 4-10 herstellen.

Beispiel 15: erythro- und threo-1-(3-Chlorphenyl)-5,5-dimethyl-pyrrolidino[1,5-c]oxazolidin-3-on

30,5 g erythro-(3-Chlorphenyl)-(1-äthoxycarbonyl-5,5-dimethyl-pyrrolidin-2-yl)-methanol werden in 100 ml Aethanol gelöst mit 0,2 g Natrium versetzt und 4 Stunden zum Rückfluss erhitzt. Man dampft zur Trockne ein und kristallisiert mehrmals aus Hexan um. Man erhält das erythro-1-(3-Chlorphenyl)-5,5-dimethyl-pyrrolidino[1,5-c]oxazolidin-3-on vom Smp. 83-84°.

30,5 g erythro-(3-Chlorphenyl)-(1-äthoxycarbonyl-5,5-dimethyl-pyrrolidin-2-yl)-methanol werden in 100 ml Aether gelöst, tropfenweise mit 11,5 g Thionylchlorid versetzt und 6 Stunden zum Rückfluss erhitzt. Man dampft zur Trockne ein, nimmt in Aether auf, wäscht mit Wasser sowie mit gesättigter Kochsalzlösung, trocknet über Natriumsulfat, dampft erneut zur Trockne ein und kristallisiert aus Hexan um. Man erhält das threo-1-(3-Chlorphenyl)-5,5-dimethyl-pyrrolidino[1,5-c]oxazolidin-3-on vom Smp. 81°.

Das Ausgangsmaterial kann z.B. folgendermassen hergestellt werden:

47,6 g erythro-(3-Chlorphenyl)-(5,5-dimethyl-pyrrolidin-2-yl)-methanol werden in 1000 ml Benzol gelöst, mit 20 g wasserfreien Kaliumcarbonat versetzt, auf +5° gekühlt und unter heftigen Rühren tropfenweise mit 21,8 g Chlorameisensäureäthylester versetzt. Man lässt 1 Stunde bei 5° bis 10° und 1 Stunde bei Raumtemperatur nachrühren, filtriert ab und dampft zur Trockne ein. Man erhält erythro-(3-Chlorphenyl)-(1-äthoxycarbonyl-5,5-dimethyl-pyrrolidin-2-yl)-methanol, das ohne weitere Reinigung verwendet werden kann.

In analoger Weise kann man auch die Verbindungen der Formel I gemäss den Beispielen 2-10 herstellen.

Beispiel 16: Gemisch von erythro- und threo-(3-Chlorphenyl)-(6,6-dimethyl-piperidin-2-yl)-methanol

26,3 g 1-(3-Chlorbenzoyl)-6,6-dimethyl-piperidin-2-carbonitril werden in 50 ml Tetrahydrofuran gelöst und zu einer auf -78° gekühlten Lösung von 12 g Lithium-N,N-diisopropylamid in 150 ml Tetrahydrofuran zugetropft. Man lässt 15 Minuten bei -78° nachrühren, fügt 15 g 3-Chlorbenzaldehyd, gelöst in wenig Tetrahydrofuran hinzu und lässt allmählich auf Raumtemperatur erwärmen. Man lässt 1 Stunde nachrühren, fügt 5 g Natriumborhydrid in 50 ml Methanol hinzu, lässt weitere 6 Stunden rühren, versetzt mit 25 ml Aceton, erwärmt 2 Stunden zum Rückfluss, dampft zur Trockne ein und kristallisiert aus Isopropanol um. Man erhält ein Gemisch von erythro- und threo-(3-Chlorphenyl)-(6,6-dimethyl-piperidin-2-yl)-methanol vom Smp. 126-131°.

Das als Ausgangsmaterial verwendete 1-(3-Chlorbenzoyl)-6,6-dimethyl-piperidin-2-carbonitril kann z.B. durch Umsetzung von 6,6-Dimethylpiperidin-2-carbonitril, erhältlich in analoger Weise wie in Beispiel 18a) beschrieben, mit 3-Chlorbenzoylchlorid/Kaliumcarbonat in Methylenchlorid hergestellt werden.

In analoger Weise kann man auch die Verbindungen der Formel V gemäss den Beispielen 1-7, 9 und 10 herstellen.

Beispiel 17: Isomerisierung von erythro- zu threo-(3-Chlorphenyl)-(5,5-dimethyl-pyrrolidin-2-yl)-methanol

5 g erythro-(3-Chlorphenyl)-(5,5-dimethyl-pyrrolidin-2-yl)-methanol werden in 50 ml Chloroform gelöst, auf 0° gekühlt und tropfenweise mit 10 ml Thionylchlorid versetzt. Man lässt 1 Stunde bei Raumtemperatur rühren, giesst auf 200 g Eis, lässt 30 Minuten bei Raumtemperatur und 2 Stunden unter Rückfluss rühren, kühlt erneut auf 0°, stellt mit Natronlauge auf PH = 11 und schüttelt viermal mit je 50 ml Chloroform aus. Die Auszüge werden vereinigt, mit Wasser gewaschen, über Natriumsulfat getrocknet und zur Trockne eingedampft. Man nimmt in Essigester auf, gibt 10 ml 5n-Salzsäure hinzu, lässt 15 Minuten bei 0° rühren und saugt ab. Man erhält das threo-(3-Chlorphenyl)-(5,5-dimethyl-pyrrolidin-2-yl)-methanol-hydrochlorid vom Smp. 209-210°.

Dieses kann durch Schütteln mit 100 ml Methylenchlorid und 10 ml 2n-Natronlauge und Abtrennen sowie Eindampfen der Methylenchloridphase in die freie Base vom Smp. 68° überführt werden.

In analoger Weise kann man auch das threo-Isomere zum erythro-Isomeren sowie die erythro-(threo)-Formen der Verbindungen der Formel V gemäss den Beispielen 2-10 zu den threo-(erythro)-Isomeren isomerisieren.

Beispiel 18: erythro-(4-Chlorphenyl)-(5,5-dimethyl-pyrrolidin-2-yl)-methanol

12,5 g 2-(4-Chlorbenzoyl)-5,5-dimethyl-pyrrolidin, gelöst in 50 ml Tetrahydrofuran, werden tropfenweise zu einer Suspension von 1,6 g Lithiumaluminiumhydrid in 250 ml Tetrahydrofuran zugegeben. Man lässt 2 Stunden unter Rückfluss sieden, kühlt im Eisbad ab, tropft vorsichtig 5 ml Wasser hinzu, lässt 30 Minuten Rühren, dampft unter vermindertem Druck zur Trockne, nimmt in Benzol auf, wäscht zweimal mit Wasser und dann mit gesättigter Kochsalzlösung, trocknet über Natriumsulfat, dampft erneut unter vermindertem Druck ein und kristallisiert aus Benzol/Dioxan um. Man erhält das erythro-(4-Chlorphenyl)-(5,5-dimethyl-pyrrolidin-2-yl)-methanol vom Smp. 123-124°.

Das Ausgangsmaterial kann z.B. folgendermassen hergestellt werden:

a) 20,8 g 1-Cyclohexyliminomethyl-2,2-dimethyl-pyrrolidin (vgl. Beispiel 11c) werden in 100 ml Tetrahydrofuran gelöst, unter Argon auf -78° gekühlt, mit 6,4 g Tertiärbutyllithium versetzt und 30 Minuten bei -75 bis -70° gerührt. Die erhaltene Lösung wird in einen auf -78° gekühlten Tropftrichter überführt und langsam zu einer heftig gerührten, auf -75° gekühlten Lösung von 6,2 g Chlorcyan in 50 ml Tetrahydrofuran zugetropft. Man lässt 30 Minuten bei -78° nachrühren, erwärmt dann allmählich auf 20°, gibt 50 ml Wasser und 10 ml Methanol hinzu, lässt 4 Stunden bei 20°-30° rühren, dampft zur Trockne ein, nimmt in Aether auf, trocknet über Natriumsulfat und dampft erneut ein. Man erhält das 5,5-Dimethyl-pyrrolidin-2-carbonitril, welches ohne weitere Reinigung verwendet werden kann.

b) Das gemäss a) erhaltene Rohprodukt sowie 10 g 4-Chlorbenzol werden in 150 ml Nitrobenzol gelöst, portionsweise mit 30 g Aluminiumtrichlorid versetzt und 2 Stunden bei 80° gerührt. Man lässt abkühlen, giesst in ein Gemisch aus 500 g Eis und 500 ml 5n-Salzsäure und lässt 12 Stunden heftig rühren. Der gebildete Niederschlag wird abgesaugt, an der Luft getrocknet und aus Essigester umkristallisiert. Man erhält rohes 2-(4-Chlorbenzoyl)-5,5-dimethyl-pyrrolidin, das ohne weitere Reinigung verwendet wird.

Beispiel 19: Isomerisierung von erythro- zu threo-(3-Chlorphenyl)-(5,5-dimethyl-pyrrolidin-2-yl)-methanol

Zu einer Lösung von 3,6 g (0,015 Mol) erythro-(3-Chlorphenyl)-(5,5-dimethyl-pyrrolidin-2-yl)-methanol in 20 ml Dimethylformamid werden bei 0-5° 1,53 g (0,015 Mol) Acetanhydrid zugetropft. Die Lösung wird anschliessend bei Raumtemperatur 1 Stunde gerührt, dann auf 100 ml Wasser gegossen und zweimal mit je 40 ml Diäthyläther extrahiert. Die vereinigten organischen Phasen werden mit 30 ml einer gesättigten

wässrigen Natriumhydrogencarbonat-Lösung gewaschen, über Magnesiumsulfat getrocknet und in Wasserstrahlvakuum zur Trockne eingedampft. Man erhält erythro-(3-Chlorphenyl)-(5,5-dimethyl-1-acetylpyrrolidin-2-yl)-methanol als farbloses Oel.

Dieses wird in 15 ml Methylenchlorid gelöst und bei 5-10° innerhalb von 5 Minuten zu einer Lösung von 2,6 g Thionylchlorid in 10 ml Methylenchlorid getropft. Die Lösung wird anschliessend bei Raumtemperatur über Nacht gerührt, dann im Wasserstrahlvakuum eingedampft. Man erhält rohes threo-1-(3-Chlorphenyl)-3-methyl-5,5-dimethyl-pyrrolidino[1,5-c]oxazoliniumchlorid als dickes, bald kristallisierendes Oel. Die hygroskopische Kristalle lassen sich aus Diäthyläther umkristallisieren, Smp. 114-117°.

2,1 g (0,007 Mol) des kristallinen Oxazoliniumchlorid werden in 10 ml Aethanol und 3,5 ml konzentrierter Natronlauge 1 Stunde zum Rückfluss erhitzt. Anschliessend wird das Gemisch auf 60 ml Eiswasser gegossen und dreimal mit je 20 ml Aethylenchlorid extrahiert. Die vereinigten organischen Phasen werden über Magnesiumsulfat getrocknet und am Wasserstrahlvakuum zur Trockne eingedampft. Der so erhaltene Rückstand wird in 5 ml Methanol aufgenommen und mit 2 ml einer Chlorwasserstoff-Lösung (4n in Diäthyläther) versetzt. Nach Zugabe von wenig Diäthyläther werden die ausgefallenen Kristalle abgenutscht und getrocknet. Man erhält threo-(3-Chlorphenyl)-(5,5-dimethyl-pyrrolidin-2-yl)-methanol-hydrochlorid von Smp. 208-209°.

Beispiel 20: erythro-1-(3-Cyanophenyl)-5,5-dimethyl-pyrrolidino[1,5-c]-oxazolidin-3-on

In analoger Weise wie im Beispiel 1 beschrieben, erhält man ausgehend von 2,2 g (0,008 Mol) erythro-(3-Cyanophenyl)-(5,5-dimethyl-pyrrolidin-2-yl)-methanol-hydrochlorid in 50 ml Toluol suspendiert, 24 ml einer 12,5%igen wässrigen Kaliumhydroxid-Lösung und 13,1 ml einer 20%-igen Phosgen-Lösung in Toluol durch Aufarbeitung nach 1 Stunde kristallines

erythro-1-(3-Cyanophenyl)-5,5-dimethyl-pyrrolidino[1,5-c]oxazolidin-3-on, Smp. 113-115°C.

Das Ausgangsmaterial kan z.B. folgendermassen hergestellt werden:

a) Analog zu der im Beispiel 1 a) beschriebenen Verfahrensweise erhält man ausgehend von 16,4 g (0,125 Mol) 3-Cyanobenzaldehyd und 16 g (0,125 Mol) 2,2-Dimethyl-1-nitroso-pyrrolidin, kristallines erythro-(3-Cyanophenyl)-(5,5-dimethyl-1-nitroso-pyrrolidin-2-yl)-methanol, Smp. 133-134°.

b) Analog zu der im Beispiel 1 b) beschriebenen Verfahrensweise erhält man ausgehend von 12,0 g (0,046 Mol) erythro-(3-Cyanophenyl)-(5,5-dimethyl-1-nitroso-pyrrolidin-2-yl)-methanol durch Behandlung mit Chlorwasserstoff-Gas in einem Toluol/Dioxan Gemisch, kristallines erythro-(3-Cyanophenyl)-(5,5-dimethyl-pyrrolidin-2-yl)-methanol-hydrochlorid, Smp. 203-205°. Die aus dem Salz freigesetzte, kristalline Base schmilzt bei 111-113°.

Beispiel 21: erythro- und threo-1-(2,4-Dichlorphenyl)-5,5-dimethyl-pyrrolidino[1,5-c]oxazolidin-3-on

In analoger Weise wie im Beispiel 1 beschrieben erhält man ausgehend von 4,1 g (0,015 Mol) erythro-(2,4-Dichlorphenyl)-(5,5-dimethyl-pyrrolidin-2-yl)-methanol, gelöst in 50 ml Toluol, 39 ml einer 25%igen wässrigen Kaliumhydroxid-Lösung und 24,5 ml einer 20%igen Phosgen-Lösung in Toluol durch Aufarbeitung nach 1 Stunde kristallines erythro-1-(2,4-Dichlorphenyl)-5,5-dimethyl-pyrrolidino[1,5-c]oxazolidin-3-on, Smp. 153-155° sowie ausgehend von 3,9 g (0,012 Mol) threo-(2,4-Dichlorphenyl)-(5,5-dimethyl-pyrrolidin-2-yl)-methanol in 50 ml Toluol, 38,5 ml einer 12,5%igen Kaliumhydroxid-Lösung und 20,6 ml einer 20%igen Phosgen-Lösung in Toluol durch Aufarbeitung nach 1 Stunde threo-1-(2,4-Dichlorphenyl)-5,5-dimethyl-pyrrolidino[1,5-c]-oxazolidin-3-on in Form einer blassgelben Harzes.

Das als Ausgangsmaterial dienende erythro- bzw. threo-(2,4-Dichlorphenyl)-(5,5-dimethyl-pyrrolidin-2-yl)-methanol kan z.B. folgendermassen hergestellt werden:

a) Analog zu der im Beispiel 1 a) beschriebenen Verfahrensweise erhält man ausgehend von 52,5 g (0,3 Mol) 2,4-Dichlorbenzaldehyd und 38,4 g (0,3 Mol) 2,2-Dimethyl-1-nitroso-pyrrolidin, das kristalline erythro-(2,4-Dichlorphenyl)-(5,5-dimethyl-1-nitroso-pyrrolidin-2-yl)-methanol, Smp. 156-158°, sowie 41,0 g eines gelblichen Oels in dem das diastereomere threo-(2,4-Dichlorphenyl)-(5,5-dimethyl-1-nitroso-pyrrolidin-2-yl)-methanol angereichert ist.

b) Analog zu der in Beispiel 1 b) beschriebenen Verfahrensweise erhält man ausgehend von 49,9 g (0,164 Mol) erytrho-(2,4-Dichlorphenyl)-(5,5-dimethyl-1-nitroso-pyrrolidin-2-yl)-methanol durch Behandlung mit Chlorwasserstoff-Gas in einem Toluol/Dioxan-Gemisch, kristallines erythro-(2,4-Dichlorphenyl)-(5,5-dimethyl-pyrrolidin-2-yl)-methanol-hydrochlorid von Smp. 249-251°. Die aus dem Salz freigesetzte kristalline Base schmilzt bei 113-115°.

c) Analog zu der im Beispiel 1 c) beschriebenen Verfahrensweise erhält man ausgehend von 41,0 g des unter a) erhaltenen Oeles, in dem das threo-(2,4-Dichlorphenyl)-(5,5-dimethyl-1-nitroso-pyrrolidin-2-yl)-methanol bereits angereichert ist, durch Behandlung mit Chlorwasserstoff-Gas in Toluol und anschliessend zweimalige Kristallisation aus Dichlormethan/Diäthyläther reines threo-(2,4-Dichlorphenyl)-(5,5-dimethyl-pyrrolidin-2-yl)-methanol-hydrochlorid von Smp. 221-222°. Die aus dem Salz freigesetzte Base schmilzt bei 50-51°.

Beispiel 22: erythro- und threo-1-(4-Pyridyl)-5,5-dimethyl-pyrrolidino[1,5-c]oxazolidin-3-on

In analoger Weise wie im Beispiel 1 beschrieben erhält man ausgehend von 5,0 g (0,018 Mol) erythro-(4-Pyridyl)-(5,5-dimethyl-pyrrolidin-2-yl)-methanol-dihydrochlorid in 70 ml Toluol suspendiert, 77 ml einer

12%igen wässrigen Kaliumhydroxid-Lösung und 28 ml einer 20%igen Phosgen-Lösung in Toluol durch Aufarbeitung nach 1 Stunde kristallines erythro-1-(4-Pyridyl)-5,5-dimethyl-pyrrolidino[1,5-c]oxazolidin-3-on, Smp. 96-98°, wobei sich durch Behandlung mit einem Ueberschuss einer Chlorwasserstofflösung (4n in Diäthyläther) ein kristallines Hydrochlorid von Smp. 175° (Z.) herstellen lässt, sowie ausgehend von 2,7 g (0,013 Mol) threo-(4-Pyridyl)-(5,5-dimethyl-pyrrolidin-2-yl)-methanol in 50 ml Toluol, 41 ml einer 12,5%igen wässrigen Kaliumhydroxid-Lösung und 22 ml einer 20%igen Phosgen-Lösung in Toluol durch Aufarbeitung nach einer Stunde reines threo-1-(4-Pyridyl)-5,5-dimethyl-pyrrolidino[1,5-c]oxazolidin-3-on in Form weisser Kristalle, Smp. 120-121°. Durch Behandlung mit einem Ueberschuss an einer etwa 4n Lösung von Chlorwasserstoff in Diäthyläther lässt sich ein kristallines Hydrochlorid herstellen, Smp. 205° (Z.).

Das als Ausgangsmaterial dienende erythro- bzw. threo-(4-Pyridyl)-(5,5-dimethyl-pyrrolidin-2-yl)-methanol kann z.B. folgendermassen hergestellt werden:

a) Analog zu der in Beispiel 1 a) beschriebenen Verfahrensweise erhält man ausgehend von 10,7 g (0,1 Mol) Pyridin-4-aldehyd und 12,8 g (0,1 Mol) 2,2-Dimethyl-1-nitroso-pyrrolidin, 12,8 g kristallines erythro-(4-Pyridyl)-(5,5-dimethyl-1-nitroso-pyrrolidin-2-yl)-methanol, Smp. 181-183°, sowie 10,5 g eines rötliche Oeles in dem das diastereomere threo-(4-Pyridyl)-(5,5-dimethyl-1-nitroso-pyrrolidin-2-yl)-methanol angereichert ist.

b) Analog zu der im Beispiel 1 b) beschriebenen Verfahrensweise erhält man ausgehend von 12,8 g (0,054 Mol) erythro-(4-Pyridyl)-(5,5-dimethyl-1-nitroso-pyrrolidin-2-yl)-methanol durch Behandlung mit Chlorwasserstoff-Gas in einem Toluol/Dioxan Gemisch kristallines erythro-(4-Pyridyl)-(5,5-dimethyl-pyrrolidin-2-yl)-methanol-dihydrochlorid, Smp. 225-227°. Die aus dem Salz freigesetzte kristalline Base schmilzt bei 122-124°.

c) Analog zu der im Beispiel 1 c) beschriebenen Verfahrensweise erhält man ausgehend von 10,5 g des unter a) erhaltene Oeles in dem das threo-(4-Pyridyl)-(5,5-dimethyl-1-nitroso-pyrrolidin-2-yl)-methanol angereichert ist, durch Behandlung mit Chlorwasserstoff-Gas in Toluol und anschliessende 2-malige Kristallisation aus Methanol/Aether reines threo-(4-Pyridyl)-(5,5-dimethyl-pyrrolidin-2-yl)-methanol-hydrochlorid, Smp. 178-179°. Die aus dem Salz freigesetzte Base schmilzt bei 122-124°.

Beispiel 23: erythro- und threo-1-(4-Chlorphenyl)-5,5-dimethyl-pyrrolidino[1,5-c]oxazolidin-3-on

In analoger Weise wie im Beispiel 1 beschrieben erhält man ausgehend von 3,2 g (0,015 Mol) erythro-(4-Chlorphenyl)-(pyrrolidin-2-yl)-methanol, 7,6 g Triäthylamin und 85 ml einer 20%igen Phosgen-Lösung in Toluol kristallines erythro-1-(4-Chlorphenyl)-pyrrolidino[1,5-c]-oxazolidin-3-on, Smp. 114-115°, sowie ausgehend von 2,7 g (0,011 Mol) threo-(4-Chlorphenyl)-(pyrrolidin-2-yl)-methanol-hydrochlorid, in 50 ml Toluol suspendiert, 35 ml einer 12,5%igen wässrigen Kaliumhydroxid-Lösung und 19 ml einer 20%igen Phosgen-Lösung in Toluol durch Aufarbeitung nach 1 Stunde, threo-1-(4-Chlorphenyl)-pyrrolidino[1,5-c]oxazolidin-3-on, Smp. 82-84°.

Beispiel 24: erythro-1-Phenyl-pyrrolidino[1,5-c]oxazolidin-3-on

In analoger Weise wie im Beispiel 1 beschrieben erhält man ausgehend von 3,5 g (0,02 Mol) erythro-Phenyl-(pyrrolidin-2-yl)-methanol, 10,1 g Triäthylamin und 110 ml einer 20%igen Phosgen-Lösung in Toluol kristallines erythro-1-Phenyl-pyrrolidino[1,5-c]oxazolidin-3-on, Smp. 67-69°, sowie ausgehend von 5,0 g (0,028 Mol) threo-Phenyl-(pyrrolidin-2-yl)-methanol, 14,1 g Triäthylamin und 154 ml einer 20%igen Phosgen-Lösung in Toluol, kristallines threo-1-Phenyl-pyrrolidino[1,5-c]oxazolidin-3-on, Smp. 66-67°.

Beispiel 25: erythro- und threo-1-(3,4-Dichlorphenyl)-5,5-tetramethylen-piperidino[1,6-c]oxazolidin-3-on

In analoger Weise wie im Beispiel 1 beschrieben erhält man ausgehend von 1,9 g (0,006 Mol) erythro-(3,4-Dichlorphenyl)-(6,6-tetramethylen-piperidin-2-yl)-methanol in 70 ml Toluol gelöst, 15,6 ml einer 12,5%igen wässrigen Kaliumhydroxid-Lösung und 9,8 ml einer 20%igen Phosgen-Lösung in Toluol durch Aufarbeitung nach 1 Stunde, kristallines erythro-1-(3,4-Dichlorphenyl)-5,5-tetramethylen-piperidino[1,6-c]oxazolidin-3-on, Smp. 152-153°, sowie ausgehend von 1,4 g (0,0045 Mol) threo-(3,4-Dichlorphenyl)-(6,6-tetramethylen-piperidin-2-yl)-methanol in 60 ml Toluol gelöst, 11,8 ml einer 12,5%igen wässrigen Kaliumhydroxid-Lösung und 7,4 ml einer 20%igen Phosgen-Lösung in Toluol, durch Aufarbeitung nach 1 Stunde, kristallines threo-1-(3,4-Dichlorphenyl)-5,5-tetramethylen-piperidino[1,6-c]oxazolidin-3-on, Smp. 101-103°.

Das als Ausgangsmaterial dienende erythro- bzw. threo-(3,4-Dichlorphenyl)-(6,6-tetramethylen-piperidin-2-yl)-methanol kann z.B. folgendermassen hergestellt werden:

a) Aus 72,4 g (0,4 Mol) 1-Brom-4-äthoxybutan, 33,7 g (0,4 Mol) Cyclopentanon und 10 g Magnesium in 150 ml Diäthyläther erhält man 1-(4-Aethoxybutyl)-cyclopentanol, Sdp.$_{0,004}$ = 73-75°.

b) Aus 32,8 g (0,176 Mol) 1-(4-Aethoxybutyl)-cyclopentanol, 12 g Kaliumcyanid in 23 ml Eisessig und 44 g konzentrierter Schwefelsäure in 23 ml Eisessig erhält man nach alkalischer Aufarbeitung 1-(4-Aethoxybutyl)-cyclopentylamin, Sdp.$_{16}$ = 130-132°.

c) Aus 16 g (0,086 Mol) 1-(4-Aethoxybutyl)-cyclopentylamin und 90 ml 48%iger wässrigen Bromwasserstoff erhält man 1-(4-Brombutyl)-cyclopentylamin-hydrobromid, Smp. 205-206°.

d) Aus 24,2 g (0,08 Mol) 1-(4-Brombutyl)-cyclopentylamin-hydrobromid in 300 ml Wasser und 80 ml n-Natronlauge erhält man 6-Azaspiro[4,5]decan, Sdp.$_{18}$ = 67-69°. Das entsprechende Hydrobromid schmilzt bei 161-163°.

e) In Analogie zu der in Beispiel 8 d) beschriebenen Verfahrensweise erhält man ausgehend von 15,6 g (0,071 Mol) 6-Azaspiro[4,5]decan, gelöst in 30 ml Wasser und 15 ml Eisessig durch Behandlung mit 29,4 ml einer 50%igen wässrigen Natriumnitrit-Lösung 6-Nitroso-6-Azaspiro[4,5]decan als gelbe Flüssigkeit, Sdp.$_{0,001}$ = 174-177°.

f) Analog zu der in Beispiel 1 a) beschriebenen Verfährensweise erhält man ausgehend von 10,9 g (0,065 Mol) 6-Nitroso-6-Azaspiro[4,5]decan und 11,4 g (0,065 Mol) 3,4-Dichlorbenzaldehyd kristallines erythro-(3,4-Dichlorphenyl)-(6,6-tetramethylen-1-nitroso-piperidin-2-yl)-methanol, Smp. 148-149°, sowie einen gelblichen Kristallbrei in dem das diastereomere threo-(3,4-Dichlorphenyl)-(6,6-tetramethylen-1-nitroso-piperidin-2-yl)-methanol angereichert ist. Eine analysenreine Probe des isomerenfreien threo-Diastereomeren kann durch Filtration über eine Schicht Silicagel mit einem Toluol/Essigsäureäthylester (10:1) Gemisch als Elutionsmittel erhalten werden. Nach dem Abdampfen der Lösungsmittel fällt das Produkt in Form weisser Kristalle vom Smp. 151-152° an.

g) Analog zu der in Beispiel 1 b) beschriebenen Verfahrensweise erhält man aus 10,3 g (0,03 Mol) erythro-(3,4-Dichlorphenyl)-(6,6-tetramethylen-1-nitroso-piperidin-2-yl)-methanol durch Behandlung mit Chlorwasserstoff-Gas in einem Toluol/Dioxan Gemisch kristallines erythro-(3,4-Dichlorphenyl)-(6,6-tetramethylen-piperidin-2-yl)-methanol-hydrochlorid, Smp. 281-282°. Die aus dem Salz freigesetzte Base schmilzt bei 116-118°.

h) In Analogie zur vorstehend unter g) beschriebenen Umsetzung erhält man ausgehend von 3,3 g (0,009 Mol) threo-(3,4-Dichlorphenyl)-(6,6-tetramethylen-1-nitroso-piperidin-2-yl)-methanol durch Behandlung mit Chlorwasserstoff-Gas in Toluol, 3,2 g kristallines threo-(3,4-Dichlorphenyl)-(6,6-tetramethylen-piperidin-2-yl)-methanol-hydrochlorid, Smp. 261-262°. Die aus dem Salz freigesetzte Base schmilzt bei 172-173°.

Beispiel 26: In analoger Weise wie in den Beispielen 1-25 beschrieben kann man ferner folgende Verbindungen herstellen:

threo-(3-Cyanophenyl)-(5,5-dimethyl-pyrrolidin-2-yl)-methanol,
threo-(3-Cyanophenyl)- 5,5-dimethyl-pyrrolidino[1,5-c]oxazolidin-3-on,
erythro- sowie threo-1-(3-Chlorphenyl)-5,5-diäthyl-piperidino[1,6-c]oxazolidin-3-on,
erythro- sowie threo-1-(3-Chlorphenyl)-5,5-di-n-pentyl-piperidino-[1,6-c]oxazolidin-3-on,
erythro- sowie threo-1-(3-Chlorphenyl)-5,5-tetramethylen-piperidino-[1,6-c]oxazolidin-3-on und
erythro- sowie threo-1-(3-Chlorphenyl)-5,5-pentamethylen-piperidino-[1,6-c]oxazolidin-3-on.

Beispiel 27: Tabletten enthaltend 25 mg Wirkstoff, z.B. threo-(3,4-Dichlorphenyl)-5,5-dimethyl-piperidino[1,6-c]oxazolidin-3-on, können folgendermassen hergestellt werden:

| Bestandteile (für 1000 Tabletten) | |
|---|---|
| Wirkstoff | 25,0 g |
| Lactose | 100,7 g |
| Weizenstärke | 7,5 g |
| Polyäthylenglykol 6000 | 5,0 g |
| Talkum | 5,0 g |
| Magnesiumstearat | 1,8 g |
| entmineralisiertes Wasser | q.s. |

Herstellung: Sämtliche festen Ingredienzien werden zunächst durch ein Sieb von 0,6 mm Maschenweite getrieben. Dann wird der Wirkstoff, die Lactose, das Talkum, das Magnesiumstearat und die Hälfte der Stärke vermischt. Die andere Hälfte der Stärke wird in 40 ml Wasser suspendiert und diese Suspension zu einer siedenden Lösung des Polyäthylenglykols in 100 ml Wasser hinzugegeben und das Gemisch, wenn nötig unter Hinzufügen von Wasser, granuliert. Das Granulat wird über Nacht bei 35° getrocknet, durch ein Sieb mit 1,2 mm Maschenweite getrieben und zu beidseitig konkaven Tabletten von etwa 6 mm Durchmesser verpresst.

Beispiel 28: Tabletten enthaltend 10 mg Wirkstoff, z.B. threo-1-(3,4-Dichlorphenyl)-5,5-dimethylpiperidino[1,6-c]oxazolidin-3-on, können folgendermassen hergestellt werden:

| Bestandteile (für 1000 Tabletten) | |
|---|---|
| Wirkstoff | 10,0 g |
| Lactose | 100,7 g |
| Weizenstärke | 7,5 g |
| Polyäthylenglykol 6000 | 5,0 g |
| Talkum | 5,0 g |
| Magnesiumstearat | 1,8 g |
| entmineralisiertes Wasser | q.s. |

Herstellung: Sämtliche festen Ingredienzien werden zunächst durch ein Sieb von 0,6 mm Maschenweite getrieben. Dann wird der Wirkstoff, die Lactose, das Talkum, das Magnesiumstearat und die Hälfte der Stärke vermischt. Die andere Hälfte der Stärke wird in 40 ml Wasser suspendiert und diese Suspension zu einer siedenden Lösung des Polyäthylenglykols in 100 ml Wasser hinzugegeben und das Gemisch, wenn nötig unter Hinzufügen von Wasser, granuliert. Das Granulat wird über Nacht bei 35° getrocknet, durch ein Sieb mit 1,2 mm Maschenweite getrieben und zu beidseitig konkaven Tabletten von etwa 6 mm Durchmesser verpresst.

Beispiel 29: Tabletten enthaltend 75 mg Wirkstoff, z.B. threo-1-(3,4-Dichlorphenyl)-5,5-dimethyl-piperidino[1,6-c]oxazolidin-3-on, können folgendermassen hergestellt werden:

| Bestandteile (für 1000 Tabletten) | |
|---|---|
| Wirkstoff | 75,0 g |
| Lactose | 100,7 g |
| Weizenstärke | 7,5 g |
| Polyäthylenglykol 6000 | 5,0 g |
| Talkum | 5,0 g |
| Magnesiumstearat | 1,8 g |
| entmineralisiertes Wasser | q.s. |

Herstellung: Sämtliche festen Ingredienzien werden zunächst durch ein Sieb von 0,6 mm Maschenweite getrieben. Dann wird der Wirkstoff, die Lactose, das Talkum, das Magnesiumstearat und die Hälfte der Stärke vermischt. Die andere Hälfte der Stärke wird in 40 ml Wasser suspendiert und diese Suspension zu einer siedenden Lösung des Polyäthylenglykols in 100 ml Wasser hinzugegeben und das Gemisch, wenn nötig unter Hinzufügen von Wasser, granuliert. Das Granulat wird über Nacht bei 35° getrocknet, durch ein Sieb mit 1,2 mm Maschenweite getrieben und zu beidseitig konkaven Tabletten von etwa 6 mm Durchmesser verpresst.

Beispiel 30: In analoger Weise wie in den Beispielen 27-29 beschrieben können auch pharmazeutische Präparate, enthaltend eine andere Verbindung der Formel I bzw. eine Verbindung der Formel V oder ein Salz davon, jeweils gemäss den Beispielen 1-26 oder threo-1-Phenyl-piperidino[1,6-c]oxazolidin-3-on bzw. ein Salz davon hergestellt werden.

Patentansprüche für die Vertragsstaaten: BE, DE, FR, IT, LU, NL, SE, CH, LI, GB

1. Ein trisubstituiertes Oxazolidinon der Formel

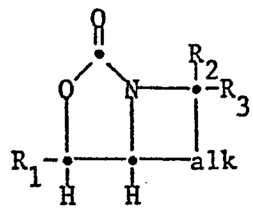

(I),

worin $R_1$ einen Aryl- oder Heteroarylrest bedeutet, $R_2$ und $R_3$ unabhängig voneinander Wasserstoff oder Niederalkyl oder gemeinsam Niederalkylen darstellen und alk für Niederalkylen steht, dessen Valenzen von benachbarten oder zueinander 1,3-ständigen Kohlenstoffatomen ausgehen, mit der Massgabe, dass in Verbindungen mit (-)-trans-Anordnung der Wasserstoffatome in 4- und 5-Stellung des Oxazolidinringes $R_1$ von unsubstituiertem Phenyl verschieden ist, wenn $R_2$ und $R_3$ für Wasserstoff stehen und alk 1,3-Propylen bedeutet, oder ein Salz davon.

2. Eine Verbindung gemäss Anspruch 1, worin $R_1$ einen als Substituenten gegebenenfalls Niederalkyl, Niederalkoxy, Halogen, Cyano und/oder Trifluormethyl aufweisenden 6-gliedrigen monocyclischen oder mindestens einen 6-gliedrigen Ring aufweisenden bicyclischen Arylrest oder monocyclischen als Heteroatom(e) Stickstoff, Sauerstoff, Schwefel, Sauerstoff sowie Stickstoff bzw. Schwefel sowie Stickstoff aufweisenden 5-gliedrigen bzw. als Heteroatom(e) 1 oder 2 Stickstoffatome aufweisenden 6-gliedrigen Heteroarylrest bedeutet, $R_2$ und $R_3$ unabhängig voneinander Wasserstoff, Niederalkyl oder gemeinsam Niederalkylen darstellen und alk Niederalkylen bedeutet, dessen Valenzen von benachbarten oder zueinander 1,3-ständigen C-Atomen ausgehen, mit der Massgabe, dass in Verbindungen mit (-)-trans-Anordnung der Wasserstoffatome in 4- und 5-Stellung des Oxazolidinringes $R_1$ von unsubstituiertem Phenyl verschieden ist, wenn $R_2$ und $R_3$ für Wasserstoff stehen und alk 1,3-Propylen bedeutet.

3. Eine Verbindung gemäss Anspruch 1, worin $R_1$ einen unsubstituierten oder durch Niederalkyl mit bis und mit 4 C-Atomen, Niederalkoxy mit bis und mit 4 C-Atomen, Halogen der Atomnummer bis und mit 35 und/oder Trifluormethyl substituierten Phenyl-, Naphthyl-, Furyl-, Thienyl-, Pyrrolyl-, Thiazolyl-, Oxazolyl-, Pyridyl-, Pyrimidinyl- oder Pyrazinylrest bedeutet, und worin entweder $R_2$ und $R_3$ unabhängig voneinander Wasserstoff oder Niederalkyl mit bis und mit 4 C-Atomen oder gemeinsam Niederalkylen mit bis und mit 7 C-Atomen, dessen freie Valenzen von benachbarten oder zueinander 1,3-, 1,4- oder 1,5-ständigen C-Atomen ausgehen, darstellen und alk Niederalkylen mit bis und mit 7 C-Atomen, dessen Valenzen von benachbarten C-Atomen ausgehen, bedeutet oder $R_2$ und $R_3$ unabhängig voneinander Niederalkyl mit bis und mit 4 C-Atomen, dessen freie Valenzen von benachbarten oder zueinander 1,3-, 1,4- oder 1,5-ständigen C-Atomen ausgehen, darstellen und Alk Niederalkylen mit bis und mit 7 C-Atomen, dessen Valenzen von zueinander 1,3-ständigen C-Atomen ausgehen, bedeutet.

4. Eine Verbindung gemäss Anspruch 1, worin $R_1$ unsubstituiertes oder durch Niederalkyl mit bis und mit 4 C-Atomen, Niederalkoxy mit bis und mit 4 C-Atomen, Halogen der Atomnummer bis und mit 35 und/oder Trifluormethyl substituiertes Phenyl oder unsubstituiertes Furyl, Thienyl oder Pyridyl bedeutet, $R_2$ und $R_3$ jeweils Niederalkylreste mit bis und mit 4 C-Atomen oder gemeinsam Niederalkylen mit bis und mit 7 C-Atomen, dessen freie Valenzen von benachbarten oder zueinander 1,4- oder 1,5-ständigen C-Atomen ausgehen, darstellen und alk für Aethylen oder 1,3-Propylen steht.

5. Eine Verbindung gemäss Anspruch 1, worin $R_1$ durch Halogen der Atomnummer bis und mit 35 substituiertes Phenyl bedeutet, $R_2$ und $R_3$ gleiche Niederalkylreste mit bis und mit 4 C-Atomen darstellen und alk für Aethylen steht.

6. Das threo-Diastereomere einer Verbindung gemäss einem der Ansprüche 1-5, worin die Wasserstoffatome in 4- und 5-Stellung des Oxazolidinringes zueinander trans-ständig sind.

7. Das erythro-1-(3-Chlorphenyl)-5,5-dimethyl-pyrrolidino[1,5-c]-oxazolidin-3-on, threo-1-(3-Chlorphenyl)-5,5-dimethyl-pyrrolidino-[1,5-c]-oxazolidin-3-on, erythro-1-(3,4-Dichlorphenyl)-5,5-dimethyl-pyrrolidino[1,5-c]oxazolidin-3-on, threo-1-(3,4-Dichlorphenyl)-5,5-dimethyl-pyrrolidino[1,5-c]oxazolidin-3-on, erythro-1-(4-Chlorphenyl)-5,5-dimethyl-pyrrolidino[1,5-c]oxazolidin-3-on, threo-1-(4-Chlorphenyl)-5,5-dimethyl-pyrrolidino[1,5-c]oxazolidin-3-on, erythro-1-(3-Methylphenyl)-5,5-dimethyl-pyrrolidino[1,5-c]oxazolidin-3-on, threo-1-(3-Methylphenyl)-5,5-dimethyl-pyrrolidino[1,5-c]oxazolidin-3-on, erythro-1-Phenyl-5,5-dimethyl-pyrrolidino[1,5-c]oxazolidin-3-on, threo-1-Phenyl-5,5-dimethyl-pyrrolidino[1,5-c]oxazolidin-3-on, erythro-1-(1-Naphthyl)-5,5-dimethyl-pyrrolidino[1,5-c]oxazolidin-3-on, threo-1-(1-Naphthyl)-5,5-dimethyl-pyrrolidino[1,5-c]oxazolidin-3-on, erythro-1-(3-Trifluormethylphenyl)-5,5-dimethyl-pyrrolidino-[1,5-c]oxazolidin-3-on, threo-1-(3-Trifluormethylphenyl)-5,5-dimethyl-pyrrolidino[1,5-c]oxazolidin-3-on, erythro-1-(3-Chlorphenyl)-5,5-dimethyl-piperidino[1,6-c]oxazolidin-3-on, threo-1-(3-Chlorphenyl)-5,5-dimethyl-piperidino[1,6-c]oxazolidin-3-on, erythro-1-(3,4-Dichlorphenyl)-5,5-dimethyl-piperidino[1,6-c]oxazolidin-3-on, threo-1-(3,4-Dichlorphenyl)-5,5-dimethyl-piperidino[1,6-c]oxazolidin-3-on, erythro-1-(2,6-Dichlorphenyl)-5,5-dimethyl-pyrrolidino[1,5-c]-oxazolidin-3-on, threo-1-(2,6-Dichlorphenyl)-5,5-dimethyl-pyrrolidino[1,5-c]oxazolidin-3-on, erythro-1-(3-Cyanophenyl)-5,5-dimethyl-pyrrolidino[1,5-c]oxazolidin-3-on, threo-1-(3-Cyanophenyl)-5,5-dimethyl-pyrrolidino[1,5-c]oxazolidin-3-on, erythro-1-(2,4-Dichlorphenyl)-5,5-dimethyl-pyrrolidino[1,5-c]oxazolidin-3-on, threo-1-(2,4-Dichlorphenyl)-5,5-dimethyl-pyrrolidino[1,5-c]oxazolidin-3-on, erythro-1-(4-Pyridyl)-5,5-dimethyl-pyrrolidino[1,5-c]oxazolidin-3-on, threo-1-(4-Pyridyl)-5,5-dimethyl-pyrrolidino[1,5-c]oxazolidin-3-on, erythro-1-(3-Chlorphenyl)-5,5-diäthyl-piperidino[1,6-c]oxazolidin-3-on, threo-1-(3-Chlorphenyl)-5,5-diäthyl-piperidino[1,6-c]oxazolidin-3-on, erythro-1-(3-Chlorphenyl)-5,5-di-n-pentyl-piperidino[1,6-c]-oxazolidin-3-on, threo-1-(3-Chlorphenyl)-5,5-di-n-pentyl-piperidino-[1,6-c]oxazolidin-3-on, erythro-1-(3-Chlorphenyl)-5,5,tetramethylen-piperidino[1,6-c]oxazolidin-3-on, threo-1-(3-Chlorphenyl)-5,5-tetramethylen-piperidino[1,6-c]oxazolidin-3-on, erythro-1-(3-Chlorphenyl)-5,5-pentamethylen-piperidino[1,6-c]-oxazolidin-3-on oder threo-1-(3-Chlorphenyl)-5,5-pentamethylen-piperidino[1,6-c]oxazolidin-3-on gemäss Anspruch 1.

8. Das erythro-1-Phenylpyrrolidino[1,5-c]oxazolidin-3-on, erythro-1-(3,4-Dichlorphenyl)-5,5-tetramethylen-piperidino[1,6-c]oxazolidin-3-on, threo-1-(3,4-Dichlorphenyl)-5,5-tetramethylen-piperidino-[1,6-c]oxazolidin-3-on, (+)-threo-1-(3,4-Dichlorphenyl)-5,5-tetramethylen-piperidino[1,6-c]oxazolidin-3-on oder (-)-threo-1-(3,4-Dichlorphenyl)-5,5-tetramethylen-piperidino[1,6-c]oxazolidin-3-on gemäss Anspruch 1.

9. Das (+)-threo-Enantiomere einer Verbindung gemäss einem der Ansprüche 1-7 mit threo-Konfiguration.

10. Das (-)-threo-Enantiomere einer Verbindung gemäss einem der Ansprüche 1-7 mit threo-Konfiguration.

11. Verfahren zur Herstellung von trisubstituierten Oxazolidinonen der Formel

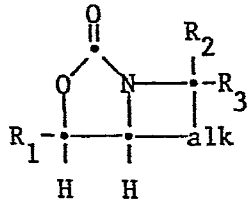

(I) ,

worin $R_1$ einen Aryl- oder Heteroarylrest bedeutet, $R_2$ und $R_3$ unabhängig voneinander Wasserstoff oder Niederalkyl oder gemeinsam

Niederalkylen darstellen und alk für Niederalkylen steht, dessen Valenzen von benachbarten oder zueinander 1,3-ständigen Kohlenstoffatomen ausgehen, mit der Massgabe, dass in Verbindungen mit (-)-trans-Anordnung der Wasserstoffatome in 4- und 5-Stellung des Oxazolidinringes $R_1$ von unsubstituiertem Phenyl verschieden ist, wenn $R_2$ und $R_3$ für Wasserstoff stehen und alk 1,3-Propylen bedeutet, und ihrer Salze, dadurch gekennzeichnet, dass man

a) in einer Verbindung der Formel

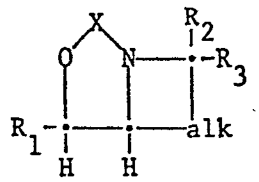

(II) ,

worin X einen in die Carbonylgruppe überführbaren Rest bedeutet, oder in einem Salz davon X in die Carbonylgruppe überführt oder

b) eine Verbindung der Formel

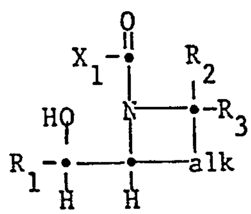

(III),

worin $X_1$ eine nukleofuge Abgangsgruppe darstellt, oder ein Salz davon intramolekular cyclisiert oder

c) in einer Verbindung der Formel

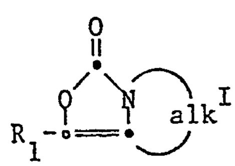

(IV),

worin $alk^{I}$ eine Gruppe der Formel $-alk-C(R_2)(R_3)-$, $-alk^{II}-C(R_2)(R_3)-$ oder $-alk^{III}=C(R_2)-$ bedeutet, die mit dem Stickstoffatom der Oxazolinringes über die $C(R_2)(R_3)$- bzw. $=C(R_2)$-Gruppe verbunden ist, wobei $alk^{II}$ einen Niederalkenylenrest und $alk^{III}$ einen Niederalkanylyliden- bzw. Niederalkenylylidenrest, dessen freie Valenzen von benachbarten oder zueinander 1,3-ständigen Kohlenstoffatomen ausgehen, darstellt, die Doppelbindung(en) des Oxazolinringes und gegebenenfalls des Restes $alk^{I}$ absättigt und gewünschtenfalls ein verfahrensgemäss erhältliches Isomerengemisch in die Komponenten auftrennt bzw. aus einem solchen das oder die gewünschte(n) Isomere(n) isoliert, eine verfahrensgemäss erhältliche Verbindung in eine andere Verbindung der Formel I umwandelt und/oder eine verfahrensgemäss erhältliche freie Verbindung in ein Salz oder ein verfahrensgemäss erhältliches Salz in die freie Verbindung oder in ein anderes Salz überführt.

12. Eine Verbindung der Formel

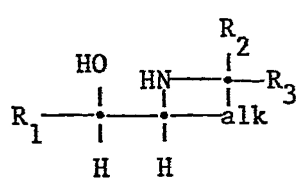

(V),

worin $R_1$ einen Aryl- oder Heteroarylrest bedeutet, $R_2$ und $R_3$ unabhängig voneinander Niederalkyl oder gemeinsam Niederalkylen darstellen und alk für Niederalkylen steht, dessen Valenzen von benachbarten oder zueinander 1,3-ständigen Kohlenstoffatomen ausgehen, oder ein Salz davon.

13. Eine Verbindung gemäss Anspruch12, worin $R_1$ einen als Substituenten gegebenenfalls Niederalkyl, Niederalkoxy, Halogen, Cyano und/oder Trifluormethyl aufweisenden 6-gliedrigen monocyclischen oder mindestens einen 6-gliedrigen Ring aufweisenden bicyclischen Arylrest oder monocyclischen, als Heteroatom(e) Stickstoff, Sauerstoff, Schwefel, Sauerstoff sowie Stickstoff oder Schwefel sowie Stickstoff aufweisenden 5-gliedrigen oder als Heteroatom(e) 1 oder 2

Stickstoffatome aufweisenden 6-gliedrigen Heteroarylrest bedeutet, $R_2$ und $R_3$ unabhängig voneinander Niederalkyl oder gemeinsam Niederalkylen darstellen und alk Niederalkylen bedeutet, dessen freie Valenzen von benachbarten oder zueinander 1,3-ständigen C-Atomen ausgehen.

14. Eine Verbindung gemäss Anspruch 12, worin $R_1$ einen unsubstituierten oder durch Niederalkyl mit bis und mit 4 C-Atomen, Niederalkoxy mit bis und mit 4 C-Atomen, Halogen der Atomnummer bis und mit 35 und/oder Trifluormethyl substituierten Phenyl-, Naphthyl-, Furyl-, Thienyl-, Pyrrolyl, Thiazolyl-, Oxazolyl-, Pyridyl-, Pyrimidinyl- oder Pyrazinylrest bedeutet, $R_2$ und $R_3$ unabhängig voneinander Niederalkyl mit bis und mit 4 C-Atomen oder gemeinsam Niederalkylen mit bis und mit 7 C-Atomen, dessen freie Valenzen von benachbarten oder zueinander 1,3-, 1,4- oder 1,5-ständigen C-Atomen ausgeht, darstellen und alk Niederalkylen mit bis und mit 7 C-Atomen, dessen freie Valenzen von benachbarten oder zueinander 1,3-ständigen C-Atomen ausgehen, bedeutet.

15. Eine Verbindung gemäss Anspruch 12, worin $R_1$ unsubstituiertes oder durch Niederalkyl mit bis und mit 4 C-Atomen, Niederalkoxy mit bis und mit 4 C-Atomen, Halogen der Atomnummer bis und mit 35 und/oder Trifluormethyl substituiertes Phenyl oder unsubstituiertes Furyl, Thienyl oder Pyridyl bedeutet, $R_2$ und $R_3$ jeweils gleiche Niederalkylreste mit bis und mit 4 C-Atomen oder gemeinsam Niederalkylen mit bis und mit 7 C-Atomen, dessen freie Valenzen von benachbarten oder zueinander 1,4- oder 1,5-ständigen C-Atomen ausgehen, darstellen und alk für Aethylen steht.

16. Das erythro-(3-Chlorphenyl)-(5,5-dimethylpyrrolidin-2-yl)-methanol, threo-(3-Chlorphenyl)-(5,5-dimethylpyrrolidin-2-yl)-methanol, erythro-(3,4-Dichlorphenyl)-(5,5-dimethylpyrrolidin-2-yl)-methanol, threo-(3,4-Dichlorphenyl)-(5,5-dimethylpyrrolidin-2-yl)-methanol, erythro-(4-Chlorphenyl)-(5,5-dimethylpyrrolidin-2-yl)methanol, threo-(4-Chlorphenyl)-(5,5-dimethylpyrrolidin-2-yl)-methanol, erythro-(3-Methylphenyl)-(5,5-dimethylpyrrolidin-2-yl)-methanol, threo-(3-Methylphenyl)-(5,5-dimethylpyrrolidin-2-yl)-methanol, erythro-Phenyl-(5,5-dimethylpyrrolidin-2-yl)-methanol, threo-Phenyl-(5,5-dimethylpyrrolidin-2-yl)-methanol, erythro-(1-Naphthyl)-(5,5-dimethylpyrrolidin-2-yl)-methanol, threo-(1-Naphthyl)-(5,5-dimethylpyrrolidin-2-yl)-methanol, erythro-(3-Trifluormethylphenyl)-(5,5-dimethylpyrrolidin-2-yl)-methanol, threo-(3-Trifluormethylphenyl)-(5,5-dimethylpyrrolidin-2-yl)-methanol, erythro-(3-Chlorphenyl)-(6,6-dimethylpiperidin-2-yl)-methanol, threo-(3-Chlorphenyl)-(6,6-dimethylpiperidin-2-yl)-methanol, erythro-(3,4-Dichlorphenyl)-(6,6-dimethylpiperidin-2-yl)-methanol, threo-(3,4-Dichlorphenyl)-(6,6-dimethylpiperidin-2-yl)-methanol, erythro-(3-Cyanophenyl)-5,5-dimethylpyrrolidin-2-yl)-methanol, threo-(3-Cyanophenyl)-(5,5-dimethylpyrrolidin-2-yl)-methanol, erythro-(2,4-Dichlorphenyl)-(5,5-dimethylpyrrolidin-2-yl)-methanol, threo-(2,4-Dichlorphenyl)-(5,5-dimethylpyrrolidin-2-yl)-methanol, erythro-(4-Pyridyl)-5,5-dimethylpyrrolidin-2-yl)-methanol oder threo-(4-Pyridyl)-(5,5-dimethylpyrrolidin-2-yl)-methanol oder jeweils ein Salz davon gemäss Anspruch 12.

17. Das erythro-(3,4-Dichlorphenyl)-(6,6-tetramethylenpiperidin-2-yl)-methanol oder threo-(3,4-Dichlorphenyl)-(6,6-tetramethylenpiperidin-2-yl)-methanol oder ein Salz davon gemäss Anspruch 12.

18. Verfahren zur Herstellung von Verbindungen der Formel

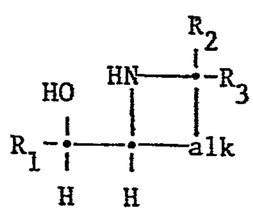 (V) ,

worin $R_1$ einen Aryl- oder Heteroarylrest bedeutet, $R_2$ und $R_3$ unabhängig voneinander Niederalkyl oder gemeinsam Niederalkylen darstellen und alk für Niederalkylen steht, dessen Valenzen von benachbarten oder zueinander 1,3-ständigen Kohlenstoffatomen ausgehen und ihrer Salze, dadurch gekennzeichnet, dass man

a) aus einer Verbindung der Formel

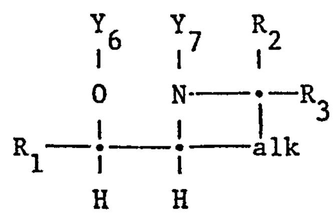

(XVIII),

worin einer der Reste $Y_6$ und $Y_7$ einen abspaltbaren Rest und der andere Wasserstoff bedeutet, oder einem Salz davon den abspaltbaren Rest unter Einführung eines Wasserstoffatoms abspaltet oder

b) eine Verbindung der Formel

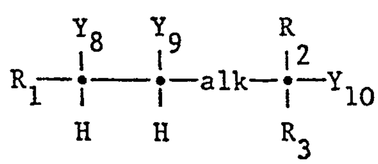

(XIX),

worin $Y_8$ Hydroxy ist und einer der Reste $Y_9$ und $Y_{10}$ reaktionsfähiges verestertes Hydroxy bedeutet oder $Y_8$ und $Y_9$ gemeinsam für Epoxy stehen, wobei der verbleibende Rest $Y_9$ bzw. $Y_{10}$ gegebenenfalls in intermediär geschützter Form vorliegendes und/oder durch einen abspaltbaren Rest substituiertes Amino darstellt, oder ein Salz davon cyclisiert oder

c) in einer Verbindung der Formel

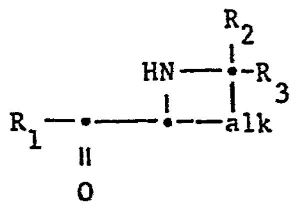

(XX),

in der das Stickstoffatom intermediär geschützt sein kann, oder in einem Salz davon die Carbonyl- zur Carbinolgruppe reduziert, erforderlichenfalls die Schutzgruppe abspaltet und gewünschtenfalls die gemäss einer der Verfahrensvarianten a) bis c) erhältliche Verbindung epimerisiert, ein verfahrensgemäss erhältliches Isomerengemischin die Komponenten auftrennt bzw. aus einem solchen das oder die gewünschte(n) Isomere(n) abtrennt, eine verfahrensgemäss erhältliche Verbindung in eine andere Verbindung der Formel V umwandelt und/oder eine verfahrensgemäss erhältliche freie Verbindung in ein Salz bzw. ein verfahrensgemäss erhältliches Salz in die freie Verbindung oder in ein anderes Salz überführt.

19. Ein trisubstituiertes Oxazolidinon der Formel

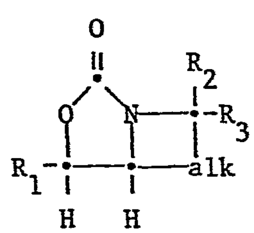

(I) ,

worin $R_1$ einen Aryl- oder Heteroarylrest bedeutet, $R_2$ und $R_3$ unabhängig voneinander Wasserstoff oder Niederalkyl oder gemeinsam Niederalkylen darstellen und alk für Niederalkylen steht, dessen Valenzen von benachbarten oder zueinander 1,3-ständigen Kohlenstoffatomen ausgehen,oder ein pharmazeutisch verwendbares Salz davon zur Anwendung in einem Verfahren zur Behandlung des menschlichen oder tierischen Körpers.

20. Eine Verbindung gemäss Anspruch 19,worin $R_1$ einen unsubstituierten oder durch Niederalkyl mit bis und mit 4 C-Atomen, Niederalkoxy mit bis und mit 4 C-Atomen, Halogen der Atomnummer bis und mit 35 und/oder Trifluormethyl substituierten Phenyl-, Naphthyl-, Furyl-, Thienyl-, Pyrrolyl-, Thiazolyl-, Oxazolyl-, Pyridyl-, Pyrimidinyl- oder Pyrazinylrest bedeutet,$R_2$ und $R_3$ unabhängig voneinander Wasserstoff oder Niederalkyl mit bis und mit 4 C-Atomen oder gemeinsam Niederalkylen mit bis und mit 7 C-Atomen, dessen freie Valenzen von benachbarten oder zueinander 1,3-, 1,4- oder 1,5-ständigen C-Atomen, ausgehen, darstellen und alk Niederalkylen mit bis und mit 7 C-Atomen, dessen freie Valenzen von benachbarten oder zueinander 1,3-ständigen C-Atomen ausgehen, bedeutet, zur Anwendung gemäss Anspruch 19.

21. Das threo-Diastereomere einer Verbindung gemäss Anspruch 19 oder 20 zur Anwendung gemäss Anspruch 19.

22. Das threo-1-Phenyl-piperidino[1,6-c]oxazolidin-3-on oder ein Salz davon zur Anwendung gemäss Anspruch 19.

23. Eine Verbindung gemäss einem der Ansprüche 1-7, 9, 10 und 12-16 zur Anwendung gemäss Anspruch 19.

24. Eine Verbindung gemäss einem der Ansprüche 8 und 17 zur Anwendung gemäss Anspruch 19.

25. Eine Verbindung gemäss einem der Ansprüche 1-10, 12-17 und 19-24 als antidepressives Arzneimittel.

26. Ein pharmazeutisches Präparat, enthaltend eine Verbindung gemäss einem der Ansprüche 1-7, 9, 10 und 12-16 neben üblichen pharmazeutischen Träger- und Hilfsstoffen.

27. Ein pharmazeutisches Präparat enthaltend eine Verbindung gemäss einem der Ansprüche 8, 17 und 22 neben üblichen pharmazeutischen Träger- und Hilfsstoffen.

FO 7.4 KVB/gs*/rz*/hc*/rn*

Patentansprüche für den Vertragsstaat AT

1. Verfahren zur Herstellung von trisubstituierten Oxazolidinonen der Formel

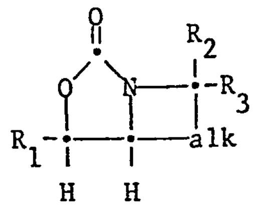

(I) ,

worin $R_1$ einen Aryl- oder Heteroarylrest bedeutet, $R_2$ und $R_3$ unabhängig voneinander Wasserstoff oder Niederalkyl oder gemeinsam Niederalkylen darstellen und alk für Niederalkylen steht, dessen Valenzen von benachbarten oder zueinander 1,3-ständigen Kohlenstoffatomen ausgehen, mit der Massgabe, dass in Verbindungen mit (-)-trans-Anordnung der Wasserstoffatome in 4- und 5-Stellung des Oxazolidinringes $R_1$ von unsubstituiertem Phenyl verschieden ist, wenn $R_2$ und $R_3$ für Wasserstoff stehen und alk 1,3-Propylen bedeutet, und ihrer Salze, dadurch gekennzeichnet, dass man

a) in einer Verbindung der Formel

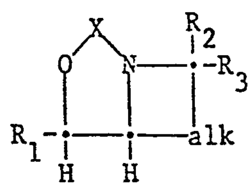

(II) ,

worin X einen in die Carbonylgruppe überführbaren Rest bedeutet, oder in einem Salz davon X in die Carbonylgruppe überführt oder

b) eine Verbindung der Formel

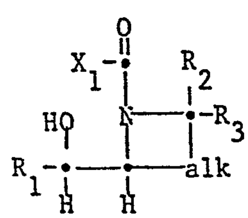

(III),

worin $X_1$ eine nukleofuge Abgangsgruppe darstellt, oder ein Salz davon intramolekular cyclisiert oder

c) in einer Verbindung der Formel

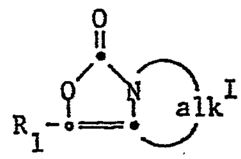

(IV),

worin $alk^I$ eine Gruppe der Formel $-alk-C(R_2)(R_3)-$, $-alk^{II}-C(R_2)(R_3)-$ oder $-alk^{III}=C(R_2)-$ bedeutet, die mit dem Stickstoffatom der Oxazolinringes über die $C(R_2)(R_3)-$ bzw. $=C(R_2)$-Gruppe verbunden ist, wobei $alk^{II}$ einen Niederalkenylenrest und $alk^{III}$ einen Niederalkanylyliden- bzw. Niederalkenylylidenrest, dessen freie Valenzen von benachbarten oder zueinander 1,3-ständigen Kohlenstoffatomen ausgehen, darstellt, die Doppelbindung(en) des Oxazolinringes und gegebenenfalls des Restes $alk^I$ absättigt und gewünschtenfalls ein verfahrensgemäss erhältliches Isomerengemisch in die Komponenten auftrennt bzw. aus einem solchen das oder die gewünschte(n) Isomere(n) isoliert, eine verfahrensgemäss erhältliche Verbindung in eine andere Verbindung der Formel I umwandelt und/oder eine verfahrensgemäss erhältliche freie Verbindung in ein Salz oder ein verfahrensgemäss erhältliches Salz in die freie Verbindung oder in ein anderes Salz überführt.

2. Verfahren gemäss Anspruch 1, dadurch gekennzeichnet, dass man eine Verbindung der Formel I, worin $R_1$ einen unsubstituierten oder durch Niederalkyl mit bis und mit 4 C-Atomen, Niederalkoxy mit bis und mit 4 C-Atomen, Halogen der Atomnummer bis und mit 35 und/oder Trifluormethyl substituierten Phenyl-, Naphthyl-, Furyl-, Thienyl-, Pyrrolyl-, Thiazolyl-, Oxazolyl-, Pyridyl-, Pyrimidinyl- oder Pyrazinylrest bedeutet, und worin entweder $R_2$ und $R_3$ unabhängig voneinander Wasserstoff oder Niederalkyl mit bis und mit 4 C-Atomen oder gemeinsam Niederalkylen mit bis und mit 7 C-Atomen, dessen freie Valenzen von benachbarten oder zueinander 1,3-, 1,4- oder 1,5-ständigen C-Atomen

ausgehen, darstellen und alk Niederalkylen mit bis und mit 7 C-Atomen, dessen Valenzen von benachbarten C-Atomen ausgehen, bedeutet, oder $R_2$ und $R_3$ unabhängig voneinander Niederalkyl mit bis und mit 4 C-Atomen oder gemeinsam Niederalkylen mit bis und mit 7 C-Atomen, dessen freie Valenzen von benachbarten oder zueinander 1,3-, 1,4- oder 1,5-ständigen C-Atomen ausgehen, darstellen und alk Niederalkylen mit bis und mit 7 C-Atomen, dessen Valenzen von zueinander 1,3-ständigen C-Atomen ausgehen, bedeutet, oder ein Salz davon herstellt.

3. Verfahren gemäss Anspruch 1, dadurch gekennzeichnet, dass man eine Verbindung der Formel I, worin $R_1$ unsubstituiertes oder durch Niederalkyl mit bis und mit 4 C-Atomen, Niederalkoxy mit bis und mit 4 C-Atomen, Halogen der Atomnummer bis und mit 35 und/oder Trifluormethyl substituiertes Phenyl oder unsubstituiertes Furyl, Thienyl oder Pyridyl bedeutet, $R_2$ und $R_3$ jeweils Niederalkylreste mit bis und mit 4 C-Atomen oder gemeinsam Niederalkylen mit bis und mit 7 C-Atomen, dessen freie Valenzen von benachbarten oder zueinander 1,4- oder 1,5-ständigen C-Atomen ausgehen, darstellen und alk für Aethylen oder 1,3-Propylen steht, oder ein Salz davon herstellt.

4. Verfahren gemäss Anspruch 1, dadurch gekennzeichnet, dass man eine Verbindung der Formel I, worin $R_1$ durch Halogen der Atomnummer bis und mit 35 substituiertes Phenyl bedeutet, $R_2$ und $R_3$ gleiche Niederalkylreste mit bis und mit 4 C-Atomen darstellen und alk für Aethylen steht, oder ein Salz davon herstellt.

5. Verfahren gemäss einem der Ansprüche 1-4, dadurch gekennzeichnet, dass man das threo-Diastereomere, worin die Wasserstoffatome in 4- und 5-Stellung des Oxazolidinringes zueinander trans-ständig sind, oder ein Salz davon herstellt.

6. Verfahren gemäss Anspruch 1, dadurch gekennzeichnet, dass man das erythro-1-(3-Chlorphenyl)-5,5-dimethyl-pyrrolidino[1,5-c]oxazolidin-3-on, threo-1-(3-Chlorphenyl)-5,5-dimethyl-pyrrolidino[1,5-c]oxazolidin-3-on, erythro-1-(3,4-Dichlorphenyl)-5,5-dimethyl-pyrrolidino-[1,5-c]oxazolidin-3-on, threo-1-(3,4-Dichlorphenyl)-5,5-dimethyl-pyrrolidino[1,5-c]oxazolidin-3-on, erythro-1-(4-Chlorphenyl)-5,5-dimethyl-pyrrolidino[1,5-c]oxazolidin-3-on, threo-1-(4-Chlorphenyl)-5,5-dimethyl-pyrrolidino[1,5-c]oxazolidin-3-on, erythro-1-(3-Methylphenyl)-5,5-dimethyl-pyrrolidino[1,5-c]oxazolidin-3-on, threo-1-(3-Methylphenyl)-5,5-dimethyl-pyrrolidino[1,5-c]oxazolidin-3-on, erythro-1-Phenyl-5,5-dimethyl-pyrrolidino[1,5-c]oxazolidin-3-on, threo-1-Phenyl-5,5-dimethyl-pyrrolidino[1,5-c]oxazolidin-3-on, erythro-1-(1-Naphthyl)-5,5-dimethyl-pyrrolidino[1,5-c]oxazolidin-3-on, threo-1-(1-Naphthyl)-5,5-dimethyl-pyrrolidino[1,5-c]oxazolidin-3-on, erythro-1-(3-Trifluormethylphenyl)-5,5-dimethyl-pyrrolidino[1,5-c]-oxazolidin-3-on, threo-1-(3-Trifluormethylphenyl)-5,5-dimethyl-pyrrolidino-[1,5-c]oxazolidin-3-on, erythro-1-(3-Chlorphenyl)-5,5-dimethyl-piperidino[1,6-c]oxazolidin-3-on, threo-1-(3-Chlorphenyl)-5,5-dimethyl-piperidino[1,6-c]oxazolidin-3-on, erythro-1-(3,4-Dichlorphenyl)-5,5-dimethyl-piperidino[1,6-c]oxazolidin-3-on, threo-1-(3,4-Dichlorphenyl)-5,5-dimethyl-piperidino[1,6-c]oxazolidin-3-on, erythro-1-(2,6-Dichlorphenyl)-5,5-dimethyl-pyrrolidino[1,5-c]oxazolidin-3-on, threo-1-(2,6-Dichlorphenyl)-5,5-dimethyl-pyrrolidino-[1,5-c]oxazolidin-3-on, erythro-1-(3-Cyanophenyl)-5,5-dimethyl-pyrrolidino[1,5-c]oxazolidin-3-on, threo-1-(3-Cyanophenyl)-5,5-dimethyl-pyrrolidino[1,5-c]oxazolidin-3-on, erythro-1-(2,4-Dichlorphenyl)-5,5-dimethyl-pyrrolidino[1,5-c]oxazolidin-3-on, threo-1-(2,4-Dichlorphenyl)-5,5-dimethyl-pyrrolidino[1,5-c]oxazolidin-3-on, erythro-1-(4-Pyridyl)-5,5-dimethyl-pyrrolidino[1,5-c]oxazolidin-3-on, threo-1-(4-Pyridyl)-5,5-dimethyl-pyrrolidino[1,5-c]oxazolidin-3-on, erythro-1-(3-Chlorphenyl)-5,5-diäthyl-piperidino[1,6-c]oxazolidin-3-on, threo-1-(3-Chlorphenyl)-5,5-diäthyl-piperidino[1,6-c]oxazolidin-3-on, erythro-1-(3-Chlorphenyl)-5,5-di-n-pentyl-piperidino[1,6-c]oxazolidin-3-on, threo-1-(3-Chlorphenyl)-5,5-di-n-pentyl-piperidino[1,6-c]oxa-

zolidin-3-on, erythro-1-(3-Chlorphenyl)-5,5-tetramethylen-piperidino-[1,6-c]oxazolidin-3-on, threo-1-(3-Chlorphenyl)-5,5-tetramethylen-piperidino[1,6-c]oxazolidin-3-on, erythro-1-(3-Chlorphenyl)-5,5-pentamethylen-piperidino[1,6-c]oxazolidin-3-on, threo-1-(3-Chlorphenyl)-5,5-pentamethylen-piperidino[1,6-c]oxazolidin-3-on, erythro-1-Phenyl-pyrrolidino[1,5-c]oxazolidin-3-on, erythro-1-(3,4-Dichlorphenyl)-5,5-tetramethylen-piperidino[1,6-c]oxazolidin-3-on oder threo-1-(3,4-Dichlorphenyl)-5,5-tetramethylen-piperidino[1,6-c]oxazolidin-3-on oder ein Salz davon herstellt.

7. Verfahren gemäss einem der Ansprüche 1-6, dadurch gekennzeichnet, dass man das (+)-threo-Enantiomere einer Verbindung der Formel I mit threo-Konfiguration oder ein Salz davon herstellt.

8. Verfahren gemäss einem der Ansprüche 1-6, dadurch gekennzeichnet, dass man (-)-threo-Enantiomere einer Verbindung der Formel I mit threo-Konfiguration herstellt.

9. Verfahren zur Herstellung von Verbindungen der Formel

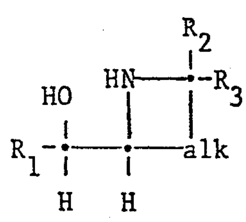

(V) ,

worin $R_1$ einen Aryl- oder Heteroarylrest bedeutet, $R_2$ und $R_3$ unabhängig voneinander Niederalkyl oder gemeinsam Niederalkylen darstellen und alk für Niederalkylen steht, dessen Valenzen von benachbarten oder zueinander 1,3-ständigen Kohlenstoffatomen ausgehen und ihrer Salze, dadurch gekennzeichnet, dass man

a) aus einer Verbindung der Formel

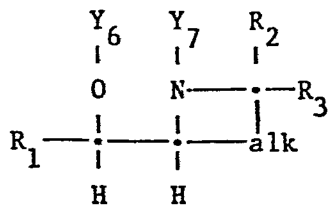 (XVIII),

worin einer der Reste $Y_6$ und $Y_7$ einen abspaltbaren Rest und der andere Wasserstoff bedeutet, oder einem Salz davon den abspaltbaren Rest unter Einführung eines Wasserstoffatoms abspaltet oder

b) eine Verbindung der Formel

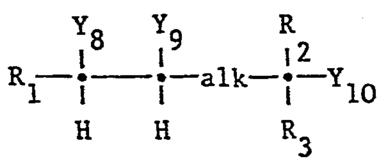 (XIX),

worin $Y_8$ Hydroxy ist und einer der Reste $Y_9$ und $Y_{10}$ reaktionsfähiges verestertes Hydroxy bedeutet oder $Y_8$ und $Y_9$ gemeinsam für Epoxy stehen, wobei der verbleibende Rest $Y_9$ bzw. $Y_{10}$ gegebenenfalls in intermediär geschützter Form vorliegendes und/oder durch einen abspaltbaren Rest substituiertes Amino darstellt, oder ein Salz davon cyclisiert oder

c) in einer Verbindung der Formel

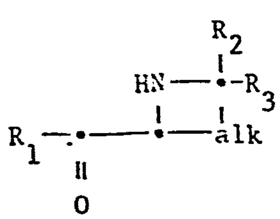 (XX),

in der das Stickstoffatom intermediär geschützt sein kann, oder in einem Salz davon die Carbonyl- zur Carbinolgruppe reduziert, erforderlichenfalls die Schutzgruppe abspaltet und gewünschtenfalls die gemäss einer der Verfahrensvarianten a) bis c) erhältliche Verbindung epimerisiert, ein verfahrensgemäss erhältliches Isomerengemisch in die Komponenten auftrennt bzw. aus einem solchen das oder die gewünschte(n) Isomere(n) abtrennt, eine verfahrensgemäss erhältliche Verbindung in eine andere Verbindung der Formel V umwandelt und/oder eine verfahrensgemäss erhältliche freie Verbindung in ein Salz bzw. ein verfahrensgemäss erhältliches Salz in die freie Verbindung oder in ein anderes Salz überführt.

10. Verfahren gemäss Anspruch 9, dadurch gekennzeichnet, dass man eine Verbindung der Vormel V, worin $R_1$ einen unsubstituierten oder durch Niederalkyl mit bis und mit 4 C-Atomen, Niederalkoxy mit bis und mit 4 C-Atomen, Halogen der Atomnummer bis und mit 35 und/oder Trifluormethyl substituierten Phenyl-, Naphthyl-, Furyl-, Thienyl-, Pyrrolyl, Thiazolyl-, Oxazolyl-, Pyridyl-, Pyrimidinyl- oder Pyrazinylrest bedeutet, $R_2$ und $R_3$ unabhängig voneinander Niederalkyl mit bis und mit 4 C-Atomen oder gemeinsam Niederalkylen mit bis und mit 7 C-Atomen, dessen freie Valenzen von benachbarten oder zueinander 1,3-, 1,4- oder 1,5-ständigen C-Atomen ausgeht, darstellen und alk Niederalkylen mit bis und mit 7 C-Atomen, dessen freie Valenzen von benachbarten oder zueinander 1,3-ständigen C-Atomen ausgehen, bedeutet, oder ein Salz davon herstellt.

11. Verfahren gemäss Anspruch 9, dadurch gekennzeichnet, dass man eine Verbindung der Formel V, worin $R_1$ unsubstituiertes

oder durch Niederalkyl mit bis und mit 4 C-Atomen, Niederalkoxy mit bis und mit 4 C-Atomen, Halogen der Atomnummer bis und mit 35 und/oder Trifluormethyl substituiertes Phenyl oder unsubstituiertes Furyl, Thienyl oder Pyridyl bedeutet, $R_2$ und $R_3$ jeweils gleiche Niederalkylreste mit bis und mit 4 C-Atomen oder gemeinsam Niederalkylen mit bis und mit 7 C-Atomen, dessen freie Valenzen von benachbarten oder zueinander 1,4- oder 1,5-ständigen C-Atomen ausgehen, darstellen und alk für Aethylen steht, oder ein Salz davon herstellt.

12. Verfahren gemäss Anspruch 9, dadurch gekennzeichnet, dass man das erythro-(3-Chlorphenyl)-(5,5-dimethylpyrrolidin-2-yl)-methanol, threo-(3-Chlorphenyl)-(5,5-dimethylpyrrolidin-2-yl)-methanol, erythro-(3,4-Dichlorphenyl)-(5,5-dimethylpyrrolidin-2-yl)-methanol, threo-(3,4-Dichlorphenyl)-(5,5-dimethylpyrrolidin-2-yl)-methanol, erythro-(4-Chlorphenyl)-(5,5-dimethylpyrrolidin-2-yl)-methanol, threo-(4-Chlorphenyl)-(5,5-dimethylpyrrolidin-2-yl)-methanol, erythro-(3-Methylphenyl)-(5,5-dimethylpyrrolidin-2-yl)-methanol, threo-(3-Methylphenyl)-(5,5-dimethylpyrrolidin-2-yl)-methanol, erythro-Phenyl-(5,5-dimethylpyrrolidin-2-yl)-methanol, threo-Phenyl-(5,5-dimethylpyrrolidin-2-yl)-methanol, erythro-(1-Naphthyl)-(5,5-dimethylpyrrolidin-2-yl)-methanol, threo-(1-Naphthyl)-(5,5-dimethylpyrrolidin-2-yl)-methanol, erythro-(3-Trifluormethylphenyl)-(5,5-dimethylpyrrolidin-2-yl)-methanol, threo-(3-Trifluormethylphenyl)-(5,5-dimethylpyrrolidin-2-yl)-methanol, erythro-(3-Chlorphenyl)-(6,6-dimethylpiperidin-2-yl)-methanol, threo-(3-Chlorphenyl)-(6,6-dimethylpiperidin-2-yl)-methanol, erythro-(3,4-Dichlorphenyl)-(6,6-dimethylpiperidin-2-yl)-methanol, threo-(3,4-Dichlorphenyl)-(6,6-dimethylpiperidin-2-yl)-methanol, erythro-(3-Cyanophenyl)-(5,5-dimethylpyrrolidin-2-yl)-methanol, threo-(3-Cyanophenyl)-(5,5-dimethylpyrrolidin-2-yl)-methanol, erythro-(2,4-Dichlorphenyl)-(5,5-dimethylpyrrolidin-2-yl)-methanol, threo-(2,4-Dichlorphenyl)-(5,5-dimethyl-pyrrolidin-2-yl)-methanol, erythro-(4-Pyridyl)-(5,5-dimethylpyrrolidin-2-yl)-methanol, threo-(4-Pyridyl)-(5,5-dimethylpyrrolidin-2-yl)-methanol, erythro-(3,4-Dichlorphenyl)-(6,6-tetramethylenpiperidin-2-yl)-methanol oder threo-(3,4-Dichlorphenyl)-(6,6-tetramethylen-piperidin-2-yl)-methanol oder ein Salz davon herstellt.

13. Verfahren zur Herstellung pharmazeutischer Präparate, dadurch gekennzeichnet, dass man eine gemäss einem der Ansprüche 1-12 erhältliche Verbindung mit üblichen pharmazeutischen Träger- und/oder Hilfsstoffen vermischt.

14. Verfahren zur Herstellung pharmazeutischer Präparate, dadurch gekennzeichnet, dass man threo-1-Phenyl-piperidino[1,6-c]oxazolidin-3-on oder ein pharmazeutisch verwendbares Salz davon mit üblichen pharmazeutischen Träger- und/oder Hilfsstoffen vermischt.

15. Verfahren zur Herstellung pharmazeutischer Präparate, dadurch gekennzeichnet, dass man

a) in einer Verbindung der Formel

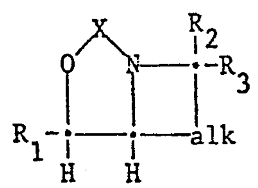

(II) ,

worin X einen in die Carbonylgruppe überführbaren Rest bedeutet, oder in einem Salz davon X in die Carbonylgruppe überführt oder

b) eine Verbindung der Formel

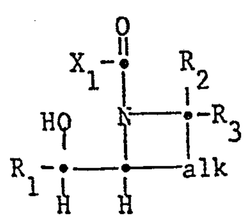

(III),

worin $X_1$ eine nukleofuge Abgangsgruppe darstellt, oder ein Salz davon intramolekular cyclisiert oder

c) in einer Verbindung der Formel

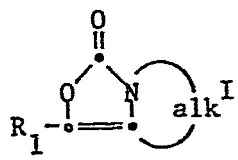

(IV),

worin $alk^I$ eine Gruppe der Formel $-alk-C(R_2)(R_3)-$, $-alk^{II}-C(R_2)(R_3)-$ oder $-alk^{III}=C(R_2)-$ bedeutet, die mit dem Stickstoffatom der Oxazolinringes über die $C(R_2)(R_3)-$ bzw. $=C(R_2)$-Gruppe verbunden ist, wobei $alk^{II}$ einen Niederalkenylenrest und $alk^{III}$ einen Niederalkanylyliden- bzw. Niederalkenylylidenrest, dessen freie Valenzen von benachbarten oder zueinander 1,3-ständigen Kohlenstoffatomen ausgehen, darstellt, die Doppelbindung(en) des Oxazolinringes und gegebenenfalls des Restes $alk^I$ absättigt gewünschtenfalls ein verfahrensgemäss erhältliches Isomerengemisch in die Komponenten auftrennt bzw. aus einem solchen das oder die gewünschte(n) Isomere(n) isoliert, eine verfahrensgemäss erhältliche Verbindung in eine andere Verbindung der Formel I umwandelt und/oder eine verfahrensgemäss erhältliche freie Verbindung in ein Salz oder ein verfahrensgemäss erhältliches Salz in die freie Verbindung oder in ein anderes Salz überführt und das erhaltene Oxazolinon der Formel

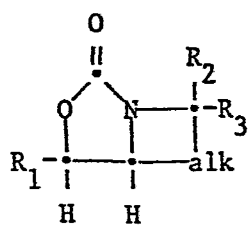

(I) ,

worin $R_1$ einen Aryl- oder Heteroarylrest bedeutet, $R_2$ und $R_3$ unabhängig voneinander Wasserstoff oder Niederalkyl oder gemeinsam Niederalkylen darstellen und alk für Niederalkylen steht, dessen Valenzen von benachbarten oder zueinander 1,3-ständigen Kohlenstoffatomen ausgehen, vorzusweise in Form des threo-Diastereomeren oder ein pharmazeutisch verwendbares Salz davon mit üblichen pharmazeutischen Träger- und/oder Hilfsstoffen vermischt.